(19) Europäisches Patentamt
European Patent Office
Office européen des brevets

(11) EP 0 815 197 B1

(12) EUROPEAN PATENT SPECIFICATION

(45) Date of publication and mention of the grant of the patent:
**18.01.2006 Bulletin 2006/03**

(21) Application number: **96911268.9**

(22) Date of filing: **08.03.1996**

(51) Int Cl.:
*C12C 3/00* (2006.01) *A23D 7/00* (2006.01)
*C12Q 1/68* (2006.01) *C12P 19/34* (2006.01)
*G01N 33/00* (2006.01) *C07K 1/00* (2006.01)
*C07K 16/00* (2006.01) *C07H 21/04* (2006.01)

(86) International application number:
**PCT/US1996/003186**

(87) International publication number:
**WO 1996/028537 (19.09.1996 Gazette 1996/42)**

(54) **LONG QT GENES AND METHOD FOR DIAGNOSING OR PREVENTING THE OCCURRENCE OF LONG QT SYNDROME**

"LONG QT" GENE UND METHODE ZUR DIAGNOSE ODER PRÄVENTION DES "LONG QT" SYNDROMS

GENES DE L'INTERVALLE QT LONG ET PROCEDE DE DIAGNOSTIC OU DE PREVENTION DE L'APPARITION DU SYNDROME A INTERVALLE QT LONG

(84) Designated Contracting States:
**AT BE CH DE DK ES FI FR GB GR IE IT LI LU MC NL PT SE**

(30) Priority: **09.03.1995 US 401512**
**20.04.1995 US 426118**

(43) Date of publication of application:
**07.01.1998 Bulletin 1998/02**

(73) Proprietor: **University of Utah Research Foundation**
**Salt Lake City,**
**Utah 84108 (US)**

(72) Inventors:
- **KEATING, Mark T.**
  **Salt Lake City, UT 84103 (US)**
- **SANGUINETTI, Michael C.**
  **Park City, UT 84060 (US)**
- **CURRAN, Mark E.**
  **Salt Lake City, UT 84102 (US)**
- **WANG, Quing**
  **Salt Lake City, UT 84108 (US)**

(74) Representative: **Dörries, Hans Ulrich et al**
**Dörries, Frank-Molnia & Pohlman,**
**Triftstrasse 13**
**80538 München (DE)**

(56) References cited:
**US-A- 4 800 159** **US-A- 5 364 790**

- **WANG Q ET AL.: "Cardiac sodium channel mutations in patients with long QT syndrome, and inherited cardiac arrhythmia" HUMAN MOLECULAR GENETICS, vol. 4, no. 9, 1995, pages 1603-1607, XP002154290**
- **SPECTOR P S ET AL.: "Class III antiarrhythmic drugs block HERG, a human cardiac delayed rectifier K+ channel" CIRCULATION RESEARCH, vol. 78, 1996, pages 499-503, XP000965264**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



(Cont. next page)

- SANGUINETTI M C ET AL.: "A mechanistic link between an inherited and an acquired cardiac arrhythmia: HERG encodes the Ikr potassium channel" CELL, vol. 81, 1995, pages 299-307, XP002154291
- ANN. REV. BIOCHEM., 1981, YELTON et al., "Monoclonal Antibodies: A Powerful New Tool in Biology and Medicine", pages 657-677.
- PROC. NATL. ACAD. SCI. U.S.A., Vol. 86, 1989, ORITA et al., "Detection of Polymorphisms of Human DNA by Gel Electrophoresis as Single-Strand Conformation Plymorphisms", pages 2766-2770.
- METHODS IN ENZYMOLOGY, Vol. 152, WAHL et al., "Molecular Hybridization of Immobilized Nucleic Acids: Theoretical Concepts and Practical Considerations", pages 399-407.
- METHODS IN ENZYMOLOGY, Vol. 154, 1987, MIYADA et al., "Oligonucleotide Hybridization Techniques", pages 94-107.
- NATURE GENETICS, Vol. 8, October 1994, JIANG et al., "Two Long QT Syndrome Loci Map to Chromosomes 3 and 7 with Evidence of Further Heterogeneity", pages 141-147.
- CIRCULATION, 01 July 1995, Vol. 92, No. 1, M.T. KEATING, "Genetic Approaches to Cardiovascular Disease Supravalvular Aortic Stenosis, Williams Syndrome and Long-QT Syndrome", pages 142-147.
- CIRCULATION, June 1992, Vol. 85, No. 6, M. KEATING, "Linkage Analysis and Long QT Syndrome", pages 1973-1986.
- CELL, Vol. 67, 29 November 1991, PTACEK et al., "Identification of a Mutation in the Gene Causing Hyperkalemic Periodic Paralysis", pages 1021-1027.
- CELL, Vol. 85, March 1995, CURRAN et al., "A Molecular Basis for Cardiac Arrhythmia: HERG Mutations Cause Long QT Syndrome", pages 795-803.
- CELL, Vol. 80, 10 March 1995, WANG et al., "SCN5A Mutations Associated with an Inherited Cardiac Arrhythmia, Long QT Syndrome", pages 805-811.

## Description

The present invention is directed to a process for the diagnosis and prevention of long QT syndrome (LQT). LQT has been associated with, specific genes including *HERG* and *SCN5A.* LQT may be hereditary and due to specific mutations in the above genes or it may be acquired, e.g., as a result of treatment with drugs given to treat cardiac arrhythmias or of treatment with other types of medications such as antihistamines or antibiotics such as erythromycin. The acquired form of LQT is the more prevalent form of the disorder. This invention shows that the *HERG* gene encodes a $K^+$ channel which is involved in the acquired form of LQT. It is shown that increasing the $K^+$ levels in patients taking drugs to prevent cardiac arrhythmias may decrease the chances of the acquired form of LQT from developing and can be used as a preventive measure. Also, this knowledge can now be used to develop drugs which may activate this $K^+$ channel and which could be given in conjunction with the drugs presently used to treat cardiac arrhythmias. Activation of the $K^+$ channel should decrease the risk of developing LQT and *torsade de pointes.*

The publications and other materials used herein to illuminate the background of the invention or provide additional details respecting the practice, are for convenience respectively grouped in the appended List of References.

Although sudden death from cardiac arrhythmias is thought to account for 11% of all natural deaths, the mechanisms underlying arrhythmias are poorly understood (Kannel, 1987; Willich et al., 1987). One form of long QT syndrome (LQT) is an inherited cardiac arrhythmia that causes abrupt loss of consciousness, syncope, seizures and sudden death from ventricular tachyarrhythmias, specifically *torsade de pointes* and ventricular fibrillation (Ward, 1964; Romano, 1965; Schwartz et al.. 1975; Moss et al., 1991). This disorder usually occurs in young, otherwise healthy individuals (Ward, 1964; Romano, 1965; Schwartz, 1975). Most LQT gene carriers manifest prolongation of the QT interval on electrocardiograms, a sign of abnonnal cardiac repolarization (Vincent et al., 1992). The clinical features of LQT result from episodic cardiac arrhythmias, specifically *torsade de pointes,* named for the characteristic undulating nature of the electrocardiogram in this arrhythmia. *Torsade de pointes* may degenerate into ventricular fibrillation, a particularly lethal arrhythmia. Although LQT is not a common diagnosis, ventricular arrhythmias are very common; more than 300,000 United States citizens die suddenly every year (Kannel et al., 1987; Willich et al., 1987) and, in many cases, the underlying mechanism may be aberrant cardiac repolarization. LQT, therefore, provides a unique opportunity to study life-threatening cardiac arrhythmias at the molecular level. A more common form of this disorder is called "acquired LQT" and it can be induced by many different factors, particularly treatment with certain medications and reduced serum $K^+$ levels (hypokalemia).

Autosomal dominant and autosomal recessive forms of the hereditary form of this disorder have been reported. Autosomal recessive LQT (also known as Jervell-Lange-Nielson syndrome) has been associated with congenital neural deafness; this form of LQT is rare (Jervell and Lange-Nielson, 1957). Autosomal dominant LQT (Romano-Ward syndrome) is more common, and is not associated with other phenotypic abnormalities. A disorder very similar to inherited LQT can also be acquired, usually as a result of pharmacologic therapy (Schwartz et al., 1975; Zipes, 1987).

In 1991, the complete linkage between autosomal dominant LQT and a polymorphism at *HRAS* was reported (Keating et al., 1991a; Keating et al., 1991b). This discovery localized *LQT1* to chromosome 11p15.5 and made presymptomatic diagnosis possible in some families. Autosomal dominant LQT was previously thought to be genetically homogeneous, and the first seven families that were studied were linked to 11p15.5 (Keating et al., 1991b). In 1993, it was found that there was locus heterogeneity for LQT (Benhorin et al., 1993; Curran et al., 1993b; Towbin et al., 1994). Two additional LQT loci were subsequently identified, *LQT2* on chromosome 7q35-36 (nine families) and *LQT3* on 3p21-24 (three families) (Jiang et al., 1994). Several families remain unlinked to the known loci, indicating additional locus heterogeneity for LQT. This degree of heterogeneity suggests that distinct LQT genes may encode proteins that interact to modulate cardiac repolarization and arrhythmia risk.

Although little is known about the physiology of LQT, the disorder is associated with prolongation of the QT interval on electrocardiograms, a sign of abnormal cardiac repolarization. This association suggests that genes encoding ion channels, or their modulators, are reasonable candidates for LQT. *HRAS,* which was localized to chromosome 11p15.5, was excluded as a candidate for *LQT1* based on direct DNA sequence analyses (unpublished observations) and by linkage analyses (Roy et al., 1994). A neuroendocrine calcium channel gene *(CACNLIA2 ;* Chin et al., 1991; Seino et al., 1992) and a gene encoding a GTP-binding protein that modulates potassium channels *(GNAI2;* Weinstein et al., 1988; Magovcevic et al., 1992) became candidates for *LQT3* based on their chromosomal location. Subsequent linkage analyses, however, have excluded these genes (Wang and Keating, unpublished data). A skeletal muscle chloride channel (*CLCN1*; Koch et al., 1992) and a cardiac muscarinic-acetylcholine receptor (*CHRM2*; Bonner et al., 1987) became candidates for *LQT2* based on their chromosome 7q35-36 location, but subsequent linkage analyses have excluded these genes (Wang et al., 1995).

In theory, mutations in a cardiac sodium channel gene could cause LQT. Voltage-gated sodium channels mediate rapid depolarization in ventricular myocytes, and also conduct a small current during the plateau phase of the action potential (Attwell et al., 1979). Subtle abnormalities of sodium channel function (e.g., delayed sodium channel inactivation or altered voltage-dependence of channel inactivation) could delay cardiac repolarization, leading to QT prolongation and arrhythmias. In 1992, Gellens and colleagues cloned and characterized a cardiac sodium channel gene, *SCN5A* (Gellens et al., 1992). The structure of this gene was similar to other, previously characterized sodium channels, encoding a large protein of 2016 amino acids. These channel proteins contain four homologous domains (DI-DIV), each of which contains six putative membrane spanning segments (S1-S6). *SCN5A* was recently mapped to chromosome 3p21, making it an excellent candidate gene for *LQT3* (George et al., 1995).

In 1994, Warmke and Ganetzky identified a novel human cDNA, human ether a-go-go related gene *(HERG,* Warmke and Ganetzky, 1994). *HERG* was localized to human chromosome 7 by PCR analysis of a somatic cell hybrid panel (Warmke and Ganetzky, 1994). The function of the protein encoded by *HERG* was not known, but it has predicted amino acid sequence homology to potassium channels. *HERG* was isolated from a hippocampal cDNA library by homology to the *Drosophila* ether a-go-go gene (*eag*), which encodes a calcium-modulated potassium channel (Bruggemann et al., 1993). *HERG* is not the human homolog of *eag*, however, sharing only ~50% amino acid sequence homology. The function of HERG was unknown, but it was strongly expressed in the heart and was hypothesized to play an important role in repolarization of cardiac action potentials (Curran et al., 1995).

Evidence is presented here indicating that *SCN5A* is *LQT3* and *HERG* is *LQT2.* Three families with mutations in *SCN5A* were identified and characterized and it was shown that in all three families there was complete linkage between *LQT3* and *SCN5A.* For the *HERG* gene, new LQT families were identified and characterized and it was shown that all were linked to markers on chromosome 7q35-36, confirming the location of *LQT2.* Second, *HERG* was mapped to chromosome 7q35-36, making *HERG* a candidate gene for *LQT2.* Third, it was demonstrated that *HERG* is strongly expressed in the heart. Finally, six *HERG* mutations which cause LQT were identified; one of these mutations arose *de novo.*

Acquired LQT usually results from therapy with medications that block cardiac $K^+$ channels (Roden, 1988). The medications most commonly associated with LQT are antiarrhythmic drugs (e.g., quinidine, sotalol) that block the cardiac rapidly-activating delayed rectifier $K^+$ current, $I_{Kr}$, as part of their spectrum of pharmacologic activity. Other drugs may also cause acquired LQT. These include antihistamines and some antibiotics such as erythromycin. $I_{Kr}$ has been characterized in isolated cardiac myocytes (Balser et al., 1990; Follmer et al., 1992; Sanguinetti and Jurkiewicz, 1990; Shibasaki, 1987; Yang et al., 1994), and is known to have an important role in initiating repolarization of action potentials. Despite extensive effort, the gene encoding this channel has not been identified.

To define the physiologic role of *HERG,* the full-length cDNA was cloned and the channel was expressed in *Xenopus* oocytes. Voltage-clamp analyses of the resulting currents revealed that *HERG* encodes a $K^+$ channel with biophysical characteristics nearly identical to $I_{Kr}$. These data suggest that *HERG* encodes the major subunit for the $I_{Kr}$ channel, and provide a mechanistic link between some forms of inherited and drug-induced LQT.

It should be mentioned here that Takahashi et al in Journal of Cardiology 23: 99-106, 1993, describe clinical characteristics and findings of patients having acquired or inherited long QT syndrome and developed in parallel torsades de pointes caused by medication. They describe also the use of potassium as a therapeutical supplement for the treatment of torsades de pointes.

The present invention demonstrates the molecular basis of long QT syndrome. More specifically, the present invention has determined that molecular variants of either one of the *SCN5A* or the *HERG* genes cause or are involved in the pathogenesis of LQT. Furthermore, the invention has determined that the acquired form of LQT is associated with the $K^+$ channel encoded by *HERG.* Genotypic analyses show that *SCNSA* is completely linked to *LQT3* in three unrelated families. The same intragenic deletion of *SCN5A* was identified in affected members of two of these families. These deletions disrupted sequences within a region of known importance for sodium channel inactivation, suggesting a likely cellular mechanism for chromosome 3-linked LQT. Genotypic analyses show that *HERG* is also linked to *LQT2* in six families. The mutations found in these families include two intragenic deletions, one splice-donor mutation and three missense mutations. Analysis of the *SCN5A* and *HERG* genes will provide an early diagnosis of subjects with hereditary LQT. The diagnostic method comprises analyzing the DNA sequence of the *SCN5A* and/or *HERG* gene of an individual to be tested and comparing it with the DNA sequence of the native, non-variant gene. In a second embodiment, the *SCN5A* and/or *HERG* gene of an individual to be tested is screened for mutations which cause LQT. The ability to predict LQT will enable physicians to prevent the disease with medical therapy such as beta blocking agents. Individuals with familial LQT or individuals being treated with drugs for cardiac arrhythmias or with other drugs which may cause acquired LQT will be monitored for $K^+$ levels and $K^+$ will be administered if necessary to raise $K^+$ levels somewhat above normal levels.

The accompanying drawings may be summarized as follows.

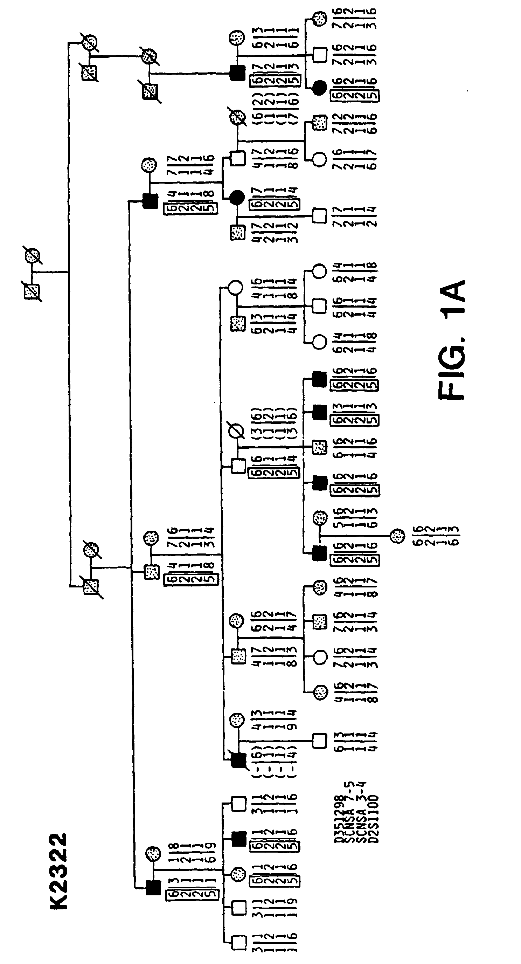

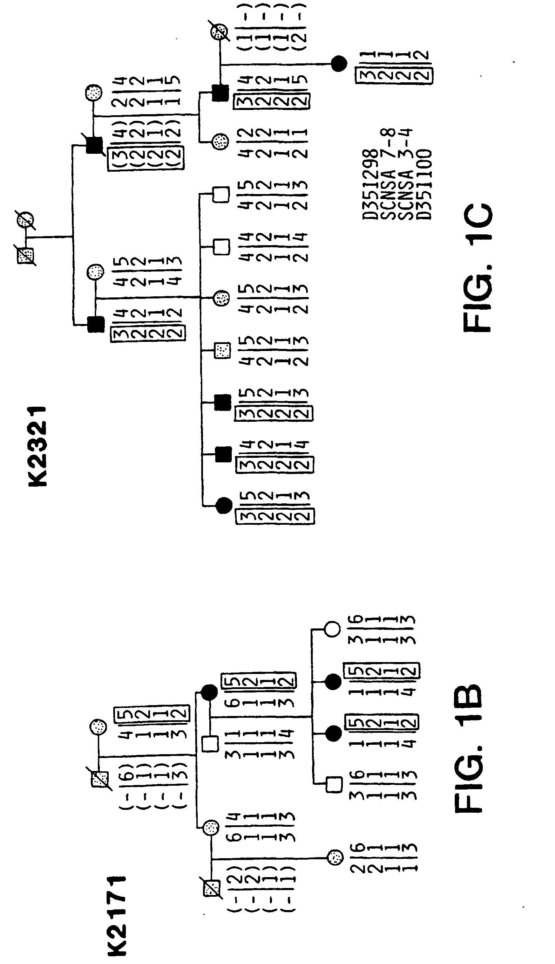

Figures 1A. 1B and 1C. Genetic linkage of *SCN5A* and *LQT3.* Pedigree structure and genotypic analyses of LQT kindreds 2322 (Figure 1A), 2171 (Figure 1B), and 2321 (Figure 1C). Individuals with the characteristic features of LQT, including prolongation of the QT interval on electrocardiogram and a history of syncope or aborted sudden death are indicated by filled circles (females) or squares (males). Unaffected individuals are indicated by empty symbols, and individuals with an equivocal phenotype are stippled. Deceased individuals are indicated by a slash. The results of genotypic analyses are shown below each symbol. Genotypes for the following *LQT3*-linked polymorphic markers are shown (telomere to centromere): *D3S1298, SCN5A 7-8, SCN5A 3-4, and D3S1100.* Haplotypes cosegregating with the disease are indicated by a box. Recombination events are indicated by a horizontal black line. Haplotype analyses indicate that *LQT3* and *SCN5A* are tightly linked in all chromosome 3-linked families.

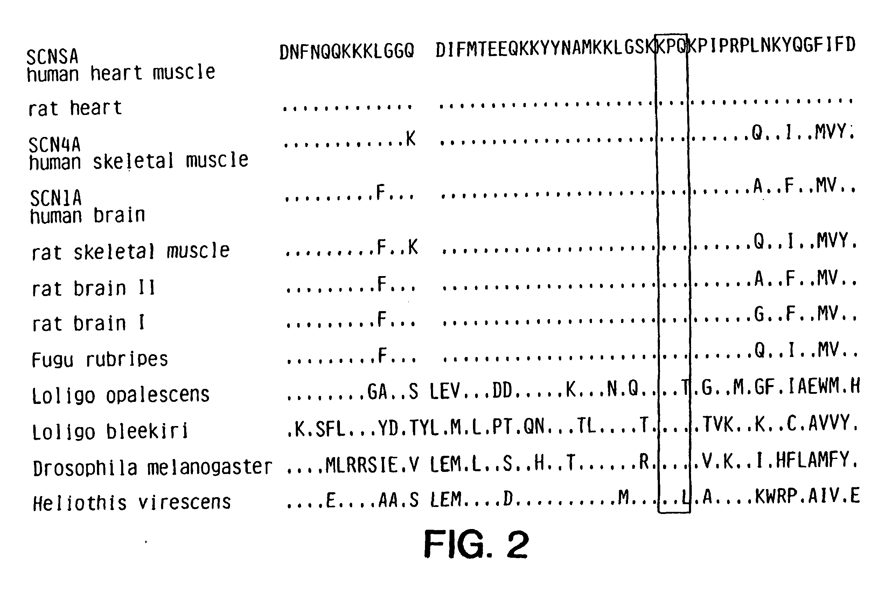

Figure 2. Amino acid sequence homology of the cytoplasmic region between DIII and DIV of sodium channels. Sequences were obtained from GDB.

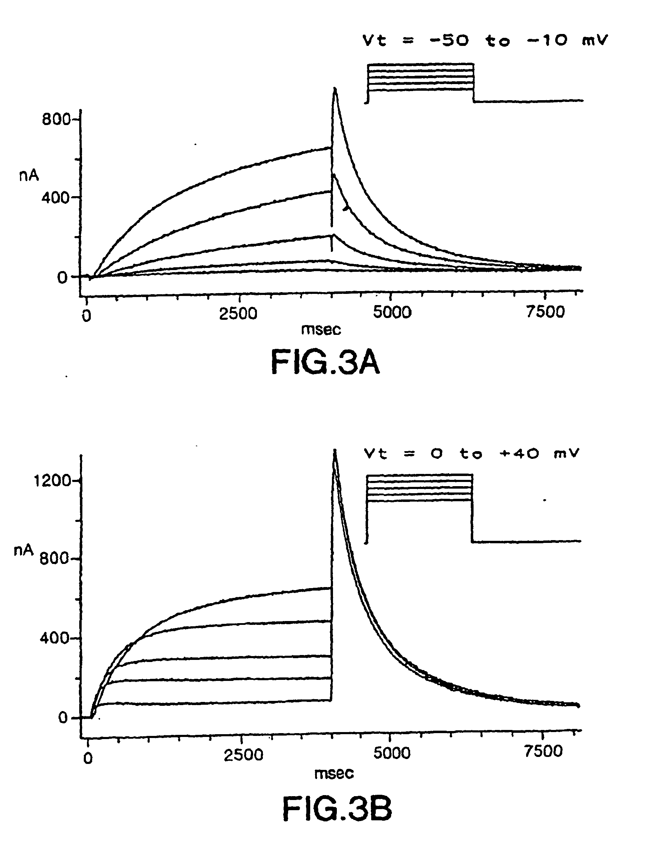

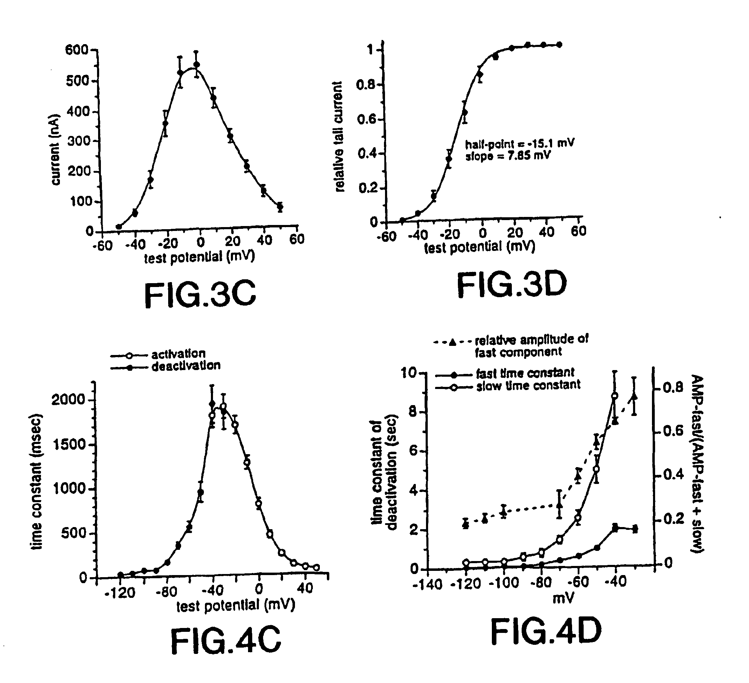

Figures 3A-D. Currents elicited by depolarizing voltage steps in *Xenopus* oocytes injected with *HERG* cRNA. Figure 3A - Currents activated by 4 sec pulses, applied in 10 mV increments from -50 to -10 mV. Current during the pulse progressively increased with voltage, as did tail current upon return to the holding potential. Holding potential was -70 mV. The inset illustrates the voltage pulse protocol. Figure 3B - Currents activated with test pulses of 0 to +40 mV, applied in 10 mV increments. Current magnitude during the pulse progressively decreased with voltage; whereas the tail current saturated at +10 mV. Note that currents do not exhibit slow inactivation. Figure 3C - Current-voltage relationship for peak HERG current recorded during 4 sec pulses (n=10). Figure 3D - Voltage-dependence of HERG channel activation. Amplitude of tail currents were measured at -70 mV following 4 sec pulses, then normalized relative to the largest current. Data was fit to a Boltzmann function: $I = 1/(1 + \exp[(V_t - V_{1/2})/k])$, where I = relative tail current, $V_t$ = test potential, $V_{1/2}$ is the voltage required for half activation of current, and k is the slope factor. ($V_{1/2}$ = -15.1±0.6 mV; k = 7.85±0.2 mV, n=10)

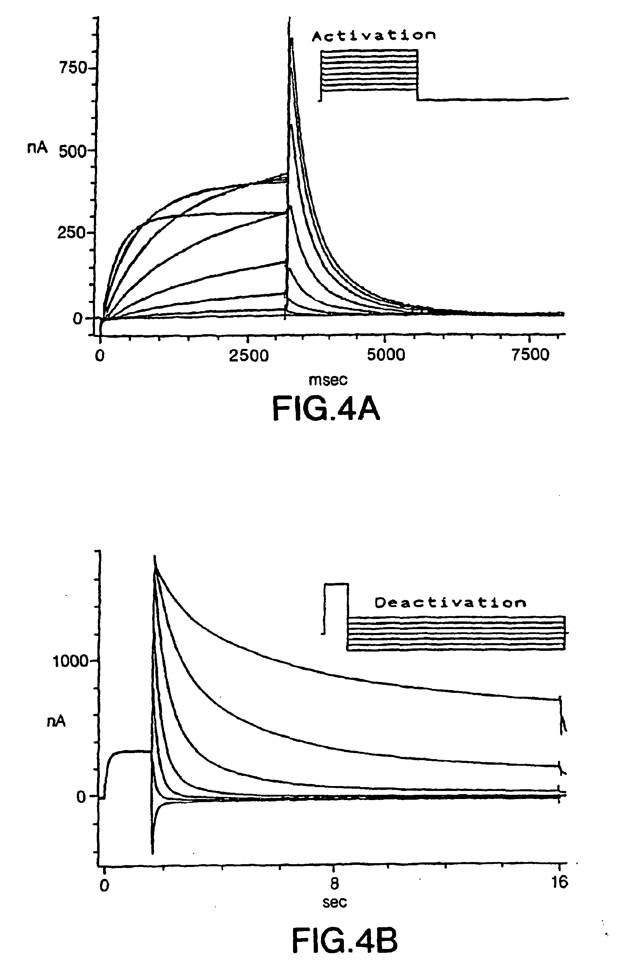

Figures 4A-D. Kinetics of HERG current activation and deactivation. Figure 4A - Activating currents were activated by 3.25 sec pulses to test potentials ranging from -50 to +20 mV (10 mV steps). Currents and corresponding single exponential fits ($I = A_0 + A_1 e^{-t/t}$) are superimposed. Figure 4B - Deactivation of HERG currents. Current was activated with 1.6 sec pulses to +20 mV, followed by return to test potentials ranging from -40 to -100 mV (10 mV steps). Deactivating currents and corresponding biexponential fits ($I_{tail} = A_0 + AMP_{fexp}{}^{-t/tf} + AMP_s exp^{-t/ts}$ ) are superimposed. Currents were not leak subtracted. Figure 4C - Voltage-dependent kinetics of activation (n = 15) and rapid deactivation (n = 11). Figure 4D - Plot of time constants ($t_f$, $t_s$) and relative amplitudes of the fast ($AMP_f$) and slow ($AMP_s$) components of HERG current deactivation as a function of test potential (n = 11). Relative amplitudes were not determined at -80 and -90 mV due to the small current magnitudes near the reversal potential.

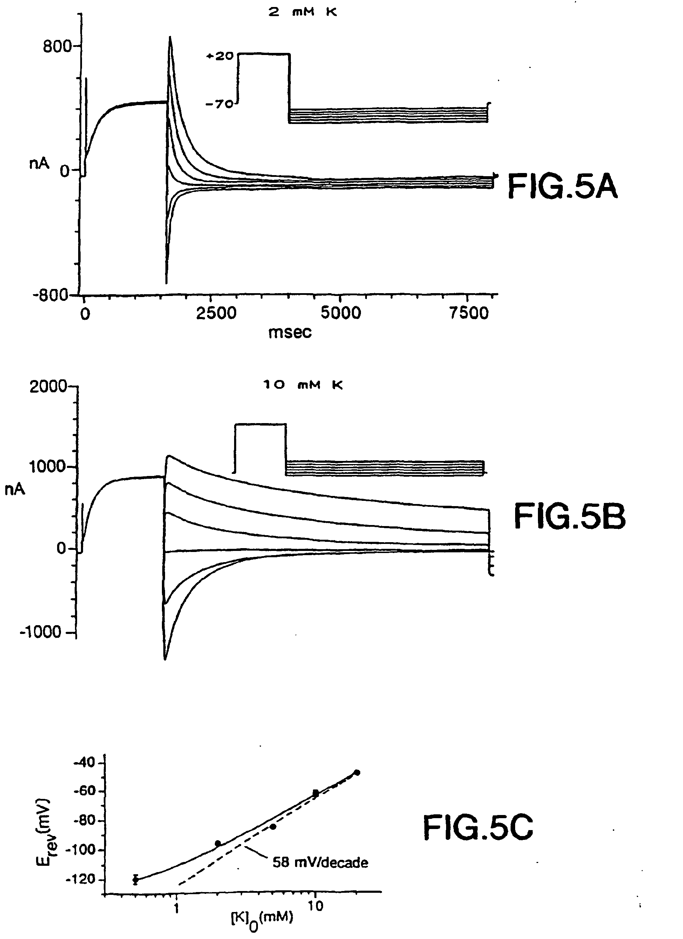

Figures 5A-C. Reversal potential of HERG current varies with $[K^+]_e$ as expected for a $K^+$-selective channel. Figure 5A - Tail currents were elicited at potentials of -105 to -80 mV (applied in 5 mV steps) in an oocyte bathed in ND96 solution ($[K^+]_e$ = 2 mM) following a pulse to +20 mV. The estimated reversal potential of tail currents was -97 mV. Currents were not leak subtracted. Figure 5B - Tail currents were elicited at potentials of -75 to -50 mV (applied in 5 mV steps) in the same oocyte bathed in modified ND96 solution ($[K^+]_e$ = 10 mM). The reversal potential of tail currents was -65 mV. Figure 5C - Reversal potential ($E_{rev}$) of HERG current varies as a function of $[K^+]_e$. $E_{rev}$ was measured for each oocyte by determining the zero-intercept from a plot of tail current amplitude as a function of test potential. Data represent the mean of 5 determinations, except for 2 mM $[K^+]_e$ (n = 15). The dotted line is the relationship predicted by the Nernst equation for a perfectly $K^+$-selective channel. The solid curve represents a fit of the data to the Goldman-Hodgkin-Katz current equation (Goldman, 1943; Hodgkin and Katz, 1949): $E_{rev} = 58 \cdot \log\{(r[Na^+]_e + [K^+]_e)/(r[Na^+]_i + [K^+]_i)\}$. The relative permeability of $Na^+$ to $K^+$ (r) determined from this fit was 0.007.

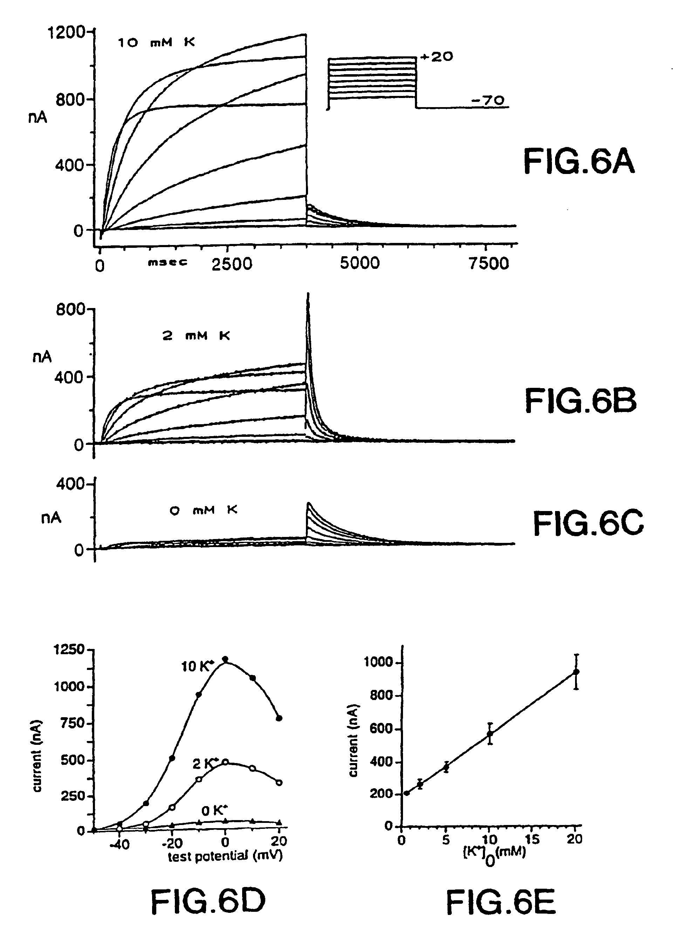

Figures 6A-E. Activation of HERG current by extracellular $K^+$. Figures 6A-C-Currents elicited by 4 sec pulses to test potentials ranging from -50 to +20 mV in an oocyte bathed in modified ND96 solution containing 10 mM KCl (A), 2 mM KCl (B), or 5 min after switching to ND96 solution with no added KCl (C). Figure 6D - Current-voltage relationship for currents shown in panels A-C. Figure 6E - HERG current amplitude varies as a function of $[K^+]_e$. Currents were measured at a test potential of +20 mV (n = 4 - 6). The solid line is a linear fit to data ($I_{HERG} = 189 + 37.5.[K^+]_e$). Note that this relationship at lower and higher $[K^+]_e$ would not be expected to be a linear function of $[K+]_e$

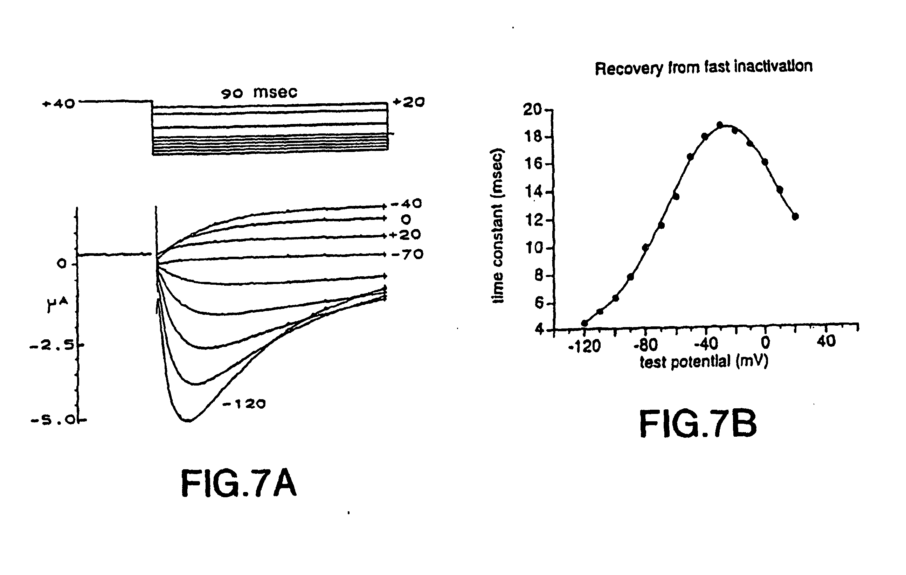

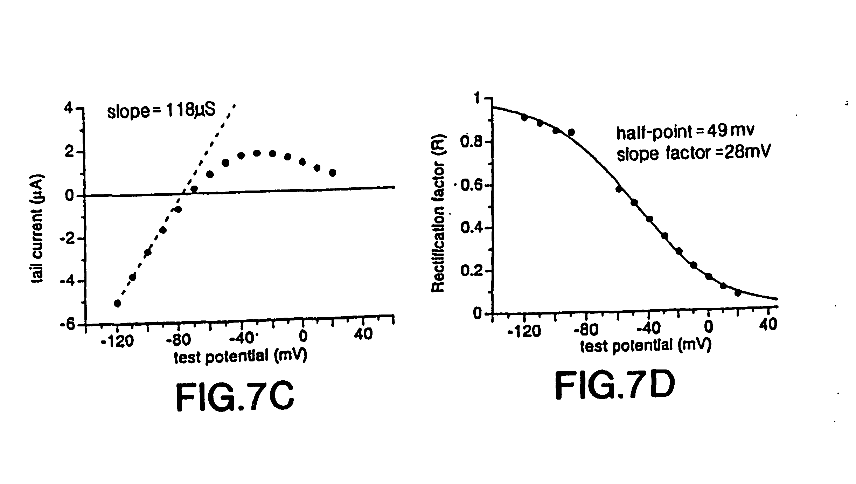

Figures 7A-D. HERG rectification results from rapid inactivation. Figure 7A - Currents recorded at test potentials of +20. 0, -40, and -70 to -120 mV (in 10 mV steps) following activation with a 260 msec pulse to +40 mV ($[K^+]_e$ = 10 mM). Currents were recorded at a sampling rate of 10 kHz. Only the final 30 msec of the activating pulse is shown, followed by the 90 msec tail current. P/3 subtraction was used to eliminate leak current; initial 2 msec of tail currents were blanked. Tail currents recorded at some potentials (+20 to -60 mV) were fit with a single exponential function, since deactivation was slow enough that it did not to contribute significantly to net kinetics of tail current. At more negative potentials (-70 to -120 mV), currents were fit with a biexponential function to account for the fast phase of deactivation that overlapped recovery from inactivation. Fits to the data are superimposed over the current traces. Figure 7B - Time constants for recovery from fast inactivation determined from fits of tail currents as described above. Figure 7C - Fully-activated HERG I-V relationship. The maximal conductance of HERG current (118 μS) was determined from the slope of a linear fit to current amplitudes at potentials between -90 and -120 mV. Figure 7D - Voltage-dependence of rapid inactivation of HERG current. The rectification factor, R, at each potential was calculated using current amplitudes plotted in panel (C):

$$R = [G \cdot n \cdot (V_t - E_{rev})]/I_{HERG}$$

where: G = maximal conductance of HERG (118 μS); n = activation variable at +40 mV (1.0); $V_t$ = test potential; $E_{rev}$ = reversal potential (-73 mV). The data were fit with a Boltzmann equation: $1/(1 + \exp[(E_{rev} - V_{1/2})/k])$. The value of $V_{1/2}$ was -49 mV and the slope factor (k) was +28 mV.

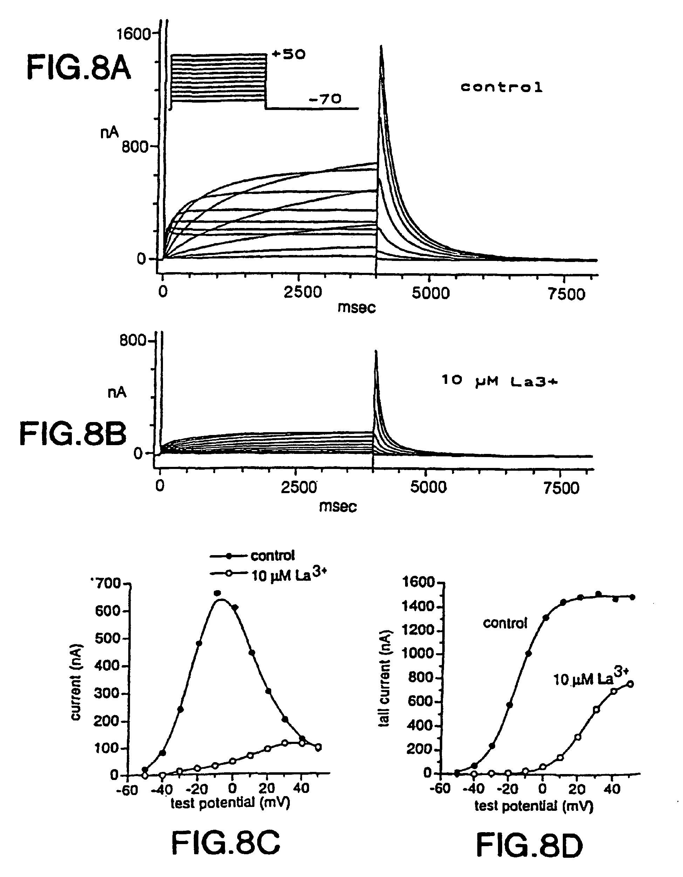

Figures 8A-D. HERG current is blocked by $La^{3+}$. Figure 8A - Control currents activated by 4 sec pulses to potentials ranging from -50 to +50 mV. Currents were not leak subtracted. Figure 8B - Currents elicited with the same pulse protocol after exposure of oocyte to 10 μM $LaCl_3$. Figure 8C - I-V relationship of HERG currents measured at the end of 4 sec test pulses. Figure 8D - Isochronal activation curves were determined from plots of tail current amplitudes as a function of test potential. Data were fitted to a Boltzmann function to obtain the smooth isochronal activation curve. $La^{3+}$ shifted the half-point of activation from -16 mV to +23 mV.

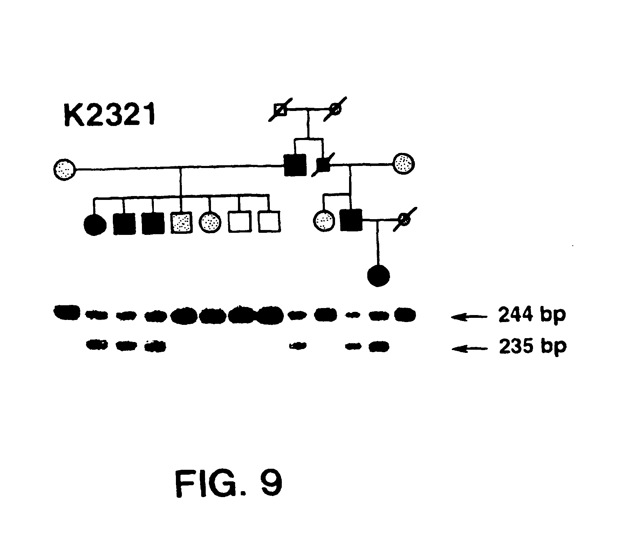

Figure 9. *SCN5A* intragenic deletion cosegregates with the disease in kindred 2321. The pedigree structure for kindred 2321 is shown. The results of PCR analyses using primer pair 3-4 and denaturing polyacrylamide gel electrophoresis are shown below each symbol. Note that the 235 bp allele cosegregates with the disease in this family, indicating the presence of a disease-associated intragenic deletion of *SCN5A*. To avoid phenotypic misclassification, the phenotypic criteria used in this study were stringent, and many individuals were classified as having an uncertain phenotype. Individuals with a QTc of 0.47 seconds or greater were classified as affected, whereas individuals with a QTc of 0.41 seconds or less were considered unaffected. All other individuals were classified as uncertain. If typical criteria were used (individuals with a QTc of 0.44 seconds or greater considered affected and individuals with a QTc less than 0.44 seconds classified as normal), all affected members of kindreds 2321 and 2322 would carry the *SCN5A* deletion and all unaffected individuals would carry only the normal allele.

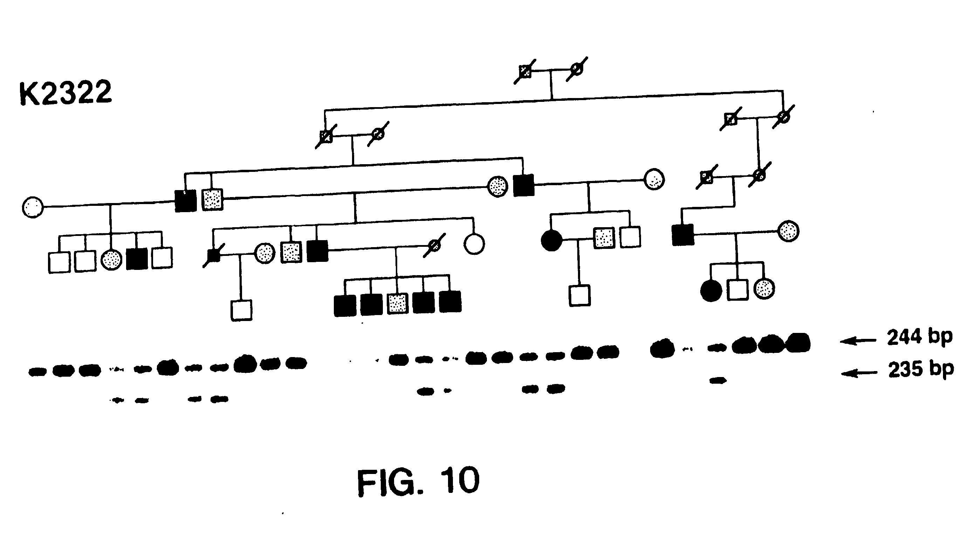

Figure 10. *SCN5A* intragenic deletion in kindred 2322. The results of PCR analyses using primer pair 3-4 and denaturing polyacrylamide gel electrophoresis are shown below each symbol. Note that the 235 bp allele cosegregates with the disease in this family, indicating the presence of a disease-associated intragenic deletion. The individuals represented in lanes 4 and 8 carried the deletion but were phenotypically classified as uncertain. These individuals had a QTc of 0.46 seconds, and with less stringent phenotypic criteria would be considered affected.

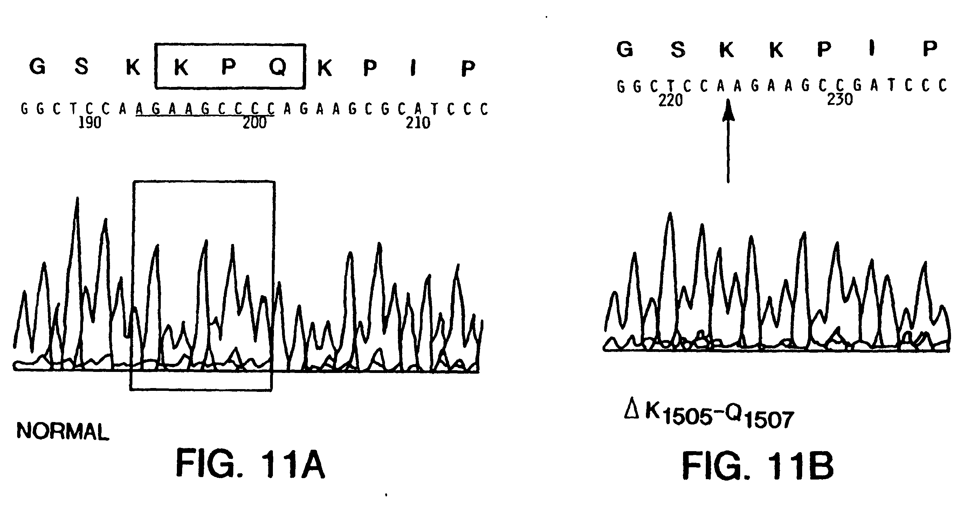

Figures 11A and 11B. DNA and amino acid sequence of the *SCN5A* intragenic deletion associated with LQT. DNA sequence analysis of the *SCN5A* intragenic deletion identified in kindred 2322. DNA sequence of normal (Figure 11A) and aberrant (Figure 11B) PCR products defines a 9 bp deletion. This mutation causes a deletion of 3 amino acids (KPQ) in the cytoplasmic region between DIII and DIV. Deleted sequences are indicated.

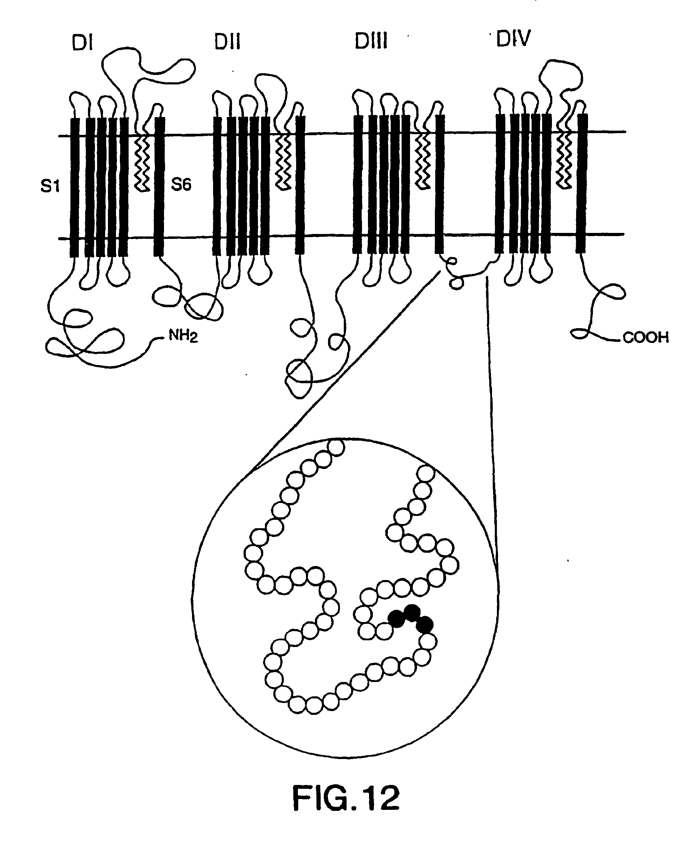

Figure 12. Schematic representation of the predicted topology of the protein encoded by *SCN5A,* and the location of the LQT-associated deletion.

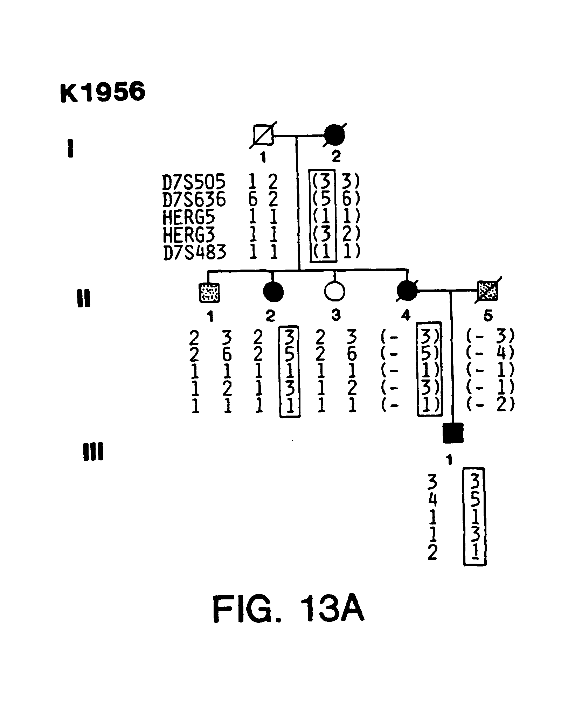

Figures 13A-13E. Pedigree structure and genotypic analyses of five new LQT families. Individuals showing the characteristic features of LQT, including prolongation of the QT interval and history of syncope, seizures or aborted sudden death, are indicated by filled circles (females) or squares (males). Unaffected individuals are indicated by empty circles or squares. Individuals with an equivocal phenotype, or for whom phenotypic data are unavailable, are stippled. Circles or squares with a slash denote deceased individuals. Haplotypes for polymorphic markers linked to *LQT2* are shown under each individual. These markers include (centromere to telomere) D7S505, D7S636. *HERG* 5-11, *HERG* 3-8, D7S483 (Gyapay et al., 1994: Wans et al., 1995). Haplotypes cosegregating with the disease phenotype are indicated by a box. Recombination events are indicated with a horizontal black line. Informed consent was obtained from all individuals, or their guardians, in accordance with local institutional review board guidelines. Haplotype analyses indicate that the LQT phenotype in these kindreds is linked to markers on chromosome 7q35-36.

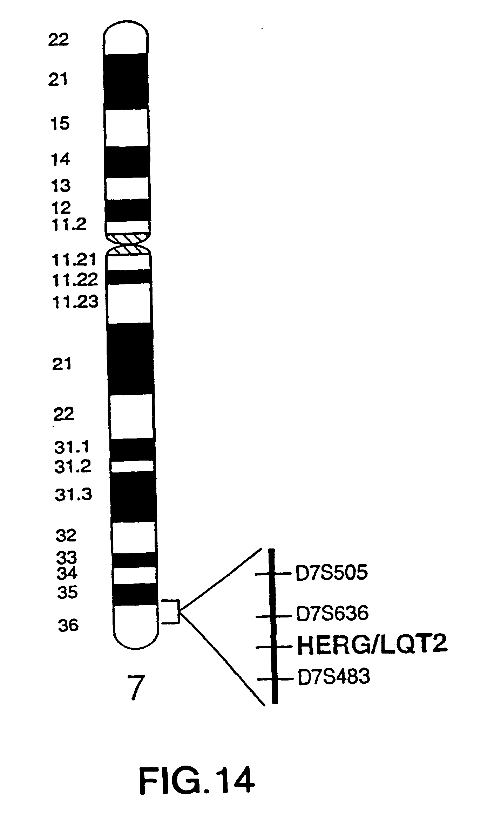

Figure 14**.** Idiogram of chromosome 7 showing location of *HERG*/*LQT2.*

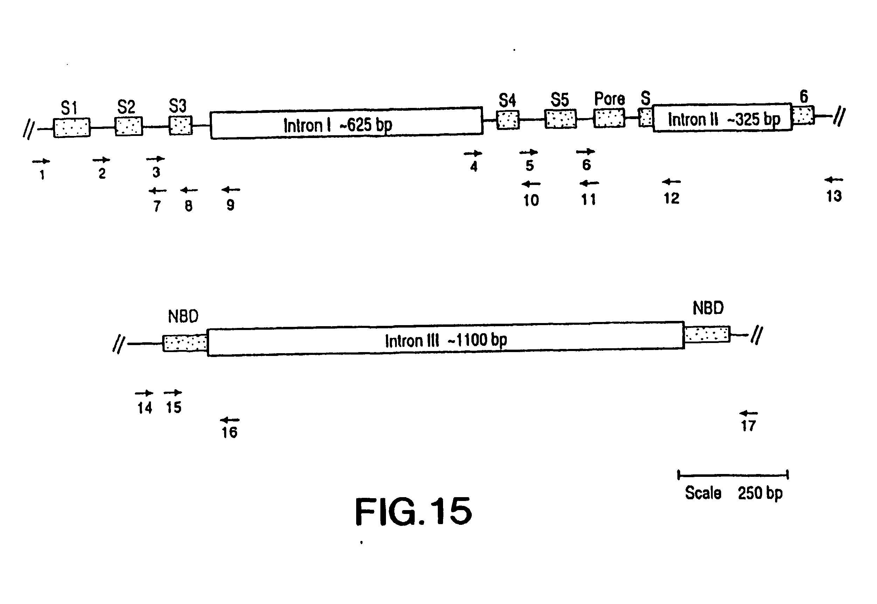

Figure 15. Partial genomic structure of *HERG* and location of PCR primers used in this study. Regions encoding predicted membrane spanning domains (S1-S6), the pore domain, and the nucleotide binding domain (NBD) are indicated. The DNA sequence for the intron-exon boundaries are: Intron I, 5'-AGGAGgtgggg(SEQ ID NO:15) ... ccccagCTGATC(SEQ ID NO:16)-3', Intron II, 5'-TGGCTgtgagt(SEQ ID NO:17)...ccccagCCCTC(SEQ ID NO:18)-3', Intron III, 5'-CCTGGgtatgg(SEQ ID NO:19)...ctccagGGAAG(SEQ ID NO:20)-3'.

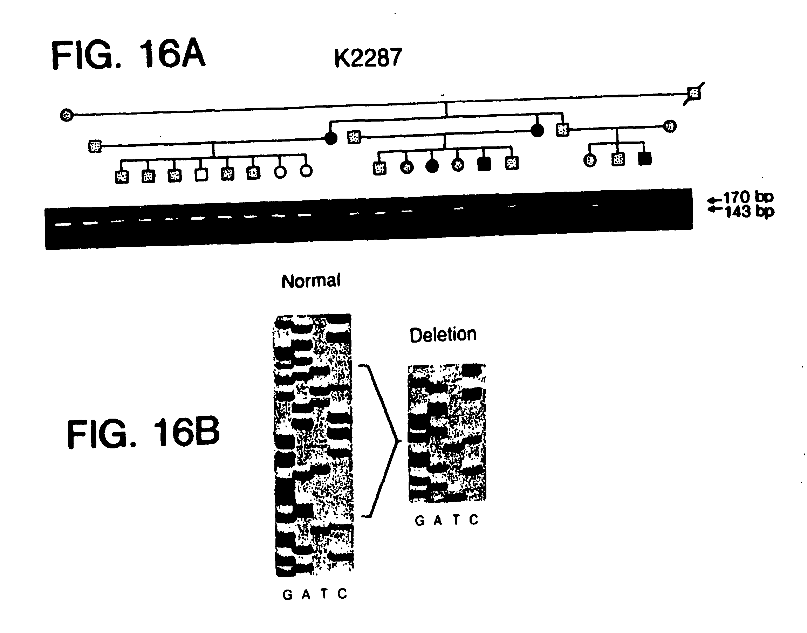

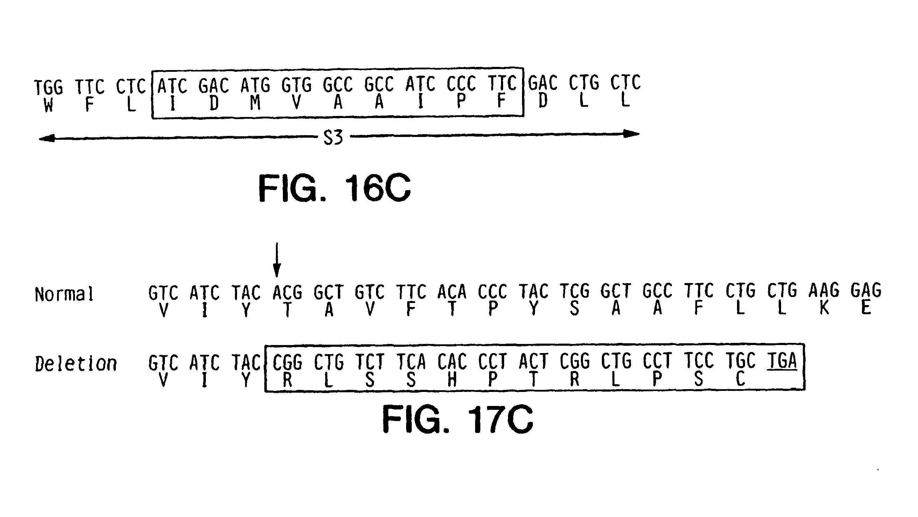

Figures 16A-C. *HERG* intragenic deletions associated with LQT in two families. Pedigree structure of K2287 (Figure 16A), results of PCR amplification using primer pair 1-9 (Figure 16A), results of DNA sequencing of normal and mutant K2287 *HERG* genes (Figure 16B), and the effect of the deletion on predicted structure of *HERG* protein (Figure 16C) are shown. Note that an aberrant fragment of 143 bp is observed in affected members of this kindred, indicating the presence of a disease-associated intragenic deletion. DNA sequence of normal and aberrant PCR products defines a 27 bp deletion (ΔI500-F508). This mutation causes an in-frame deletion of 9 amino acids in the third membrane spanning domain (S3). Deleted sequences are indicated.

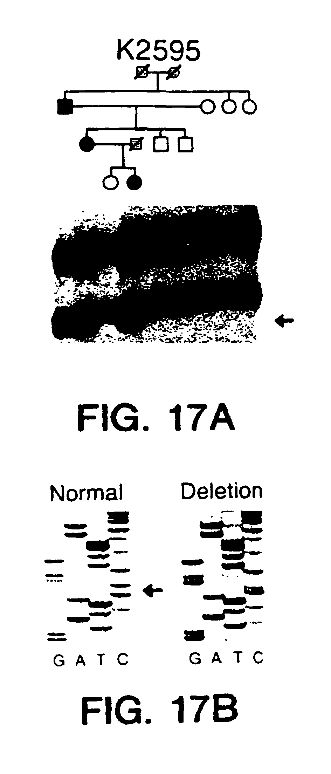

Figures 17A-C. Pedigree structure of K2595 is shown (Figure 17A). Deceased individuals are indicated by a slash. The result of SSCP analyses using primer pair 1-9 are shown beneath each individual (Figure 17A). Note that an aberrant SSCP conformer cosegregates with the disease in this family. DNA sequence shows a single base-pair deletion (△1261) (Figure 17B). This deletion results in a frameshift followed by a stop codon 12 amino acids downstream (Figure 17C). The deleted nucleotide is indicated with an arrow.

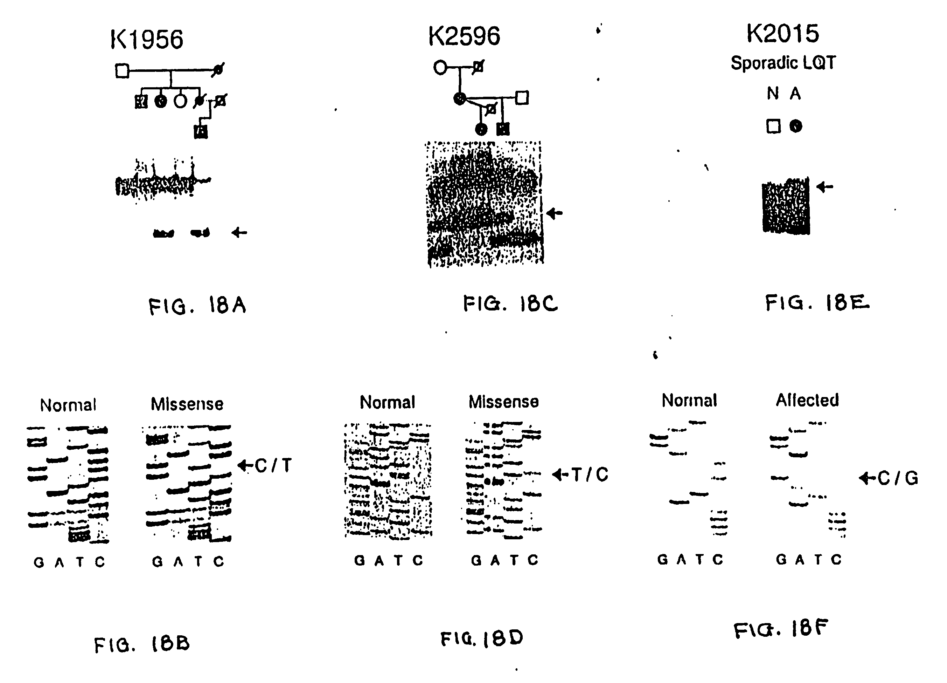

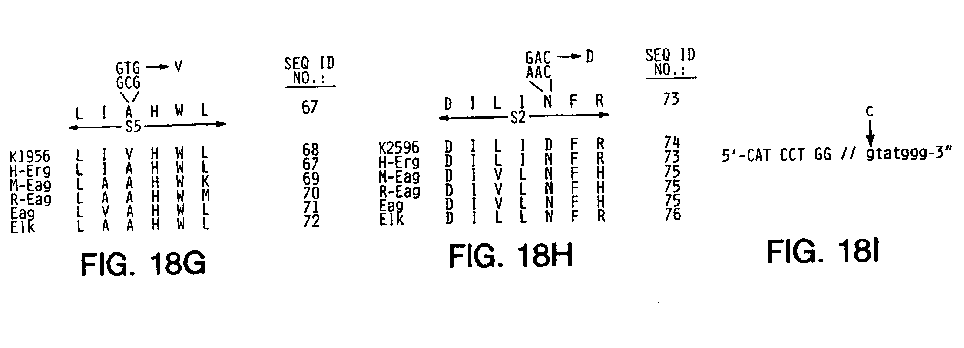

Figures 18A-I. *HERG* point mutations identified in three LQT kindreds. Pedigree structure of K1956 (Figure 18A), K2596 (Figure 18C) and K2015 (Figure 18E) are shown. Below each pedigree, the results of SSCP analyses with primer pair 5-11 (K1956) (Figure 18B), primer pair 1-9 (K2596) (Figure 18D) and primer pair 4-12 (K2015) (Figure 18F) are shown. Aberrant SSCP conformers cosegregate with the disease in each kindred. DNA sequence analyses of the normal and aberrant conformers reveals a C to T substitution at position 1682 in K1956. This mutation results in substitution of valine for a highly conserved alanine residue at codon 561 (A561V) (Figure 18G). Analyses of K2596 reveals an A to G substitution at position 1408 (T to C substitution on the anti-sense strand is shown) (Figure 18D). This mutation results in substitution of aspartic acid for a conserved asparagine in the second transmembrane domain (N470D) (Figure 18H). Analyses of K2015 reveals a G to C substitution (C to G substitution on the anti-sense strand is shown) (Figure 18F). This mutation occurs in the splice-donor sequence of intron III (Figure 18I). Coding sequences are upper case and intronic sequences are lower case. Note that the G to C substitution disrupts the splice-donor site. (*HERG,* M-eag, *elk,* Warmke and Ganetzky, 1994; R-eag; Ludwig et al., 1994).

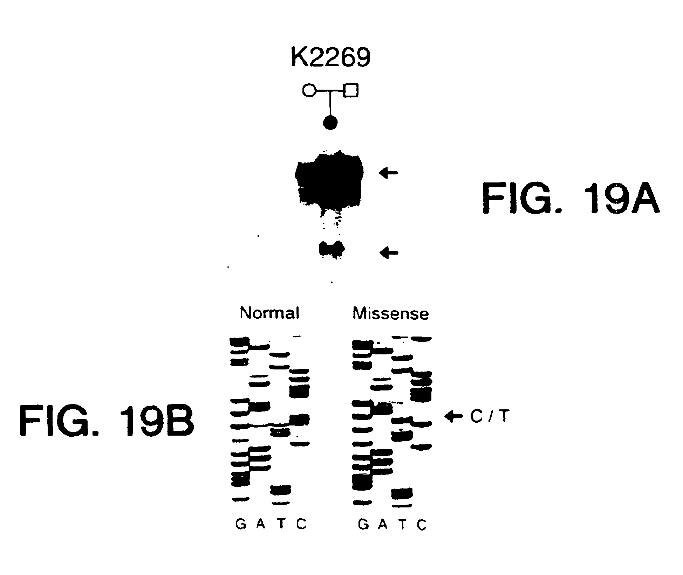

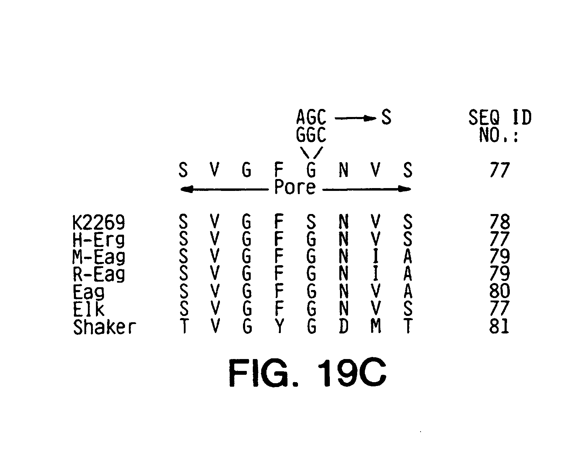

Figures 19A-C. *De novo* mutation of *HERG* in a sporadic case of LQT. Pedigree structure of K2269 (Figure 19A) and SSCP analyses (primer pair 14-16) (Figure 19A) showing an aberrant conformer in a sporadic case of LQT. DNA sequence analyses identified a G to A substitution at position 1882 of the cDNA sequence (C to T substitution on the antisense-strand is shown) (Figure 19B). Note that this mutation results in the substitution of a serine for a highly conserved glycine residue at codon 628 (G628S) (Figure 19C). This amino acid sequence is known to be critical for potassium ion selectivity.

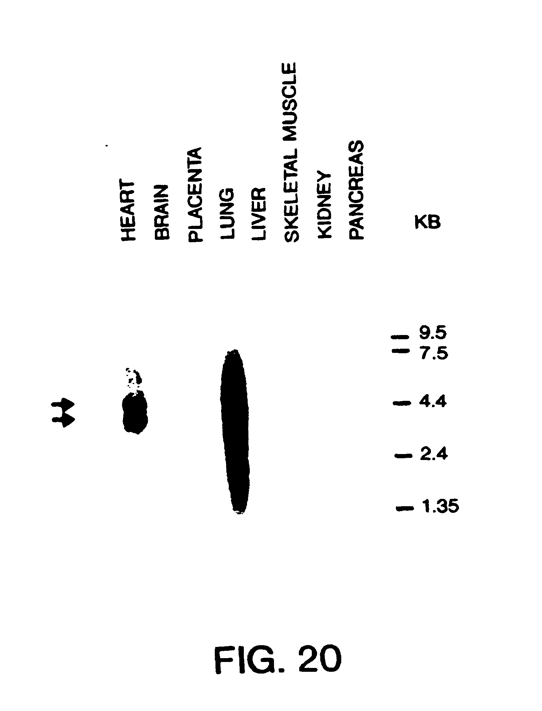

Figure 20. Northern blot analysis of *HERG* mRNA showing strong expression in the heart. A Northern blot (Clonetech, poly $A^+$ RNA, 2 mg/lane) was probed using an *HERG* cDNA containing nucleotides 679 to 2239 of the coding sequence. Two cardiac mRNAs of -4.1 and 4.4 kb are indicated. Background in mRNA extracted from lung was high, but no specific bands were identified.

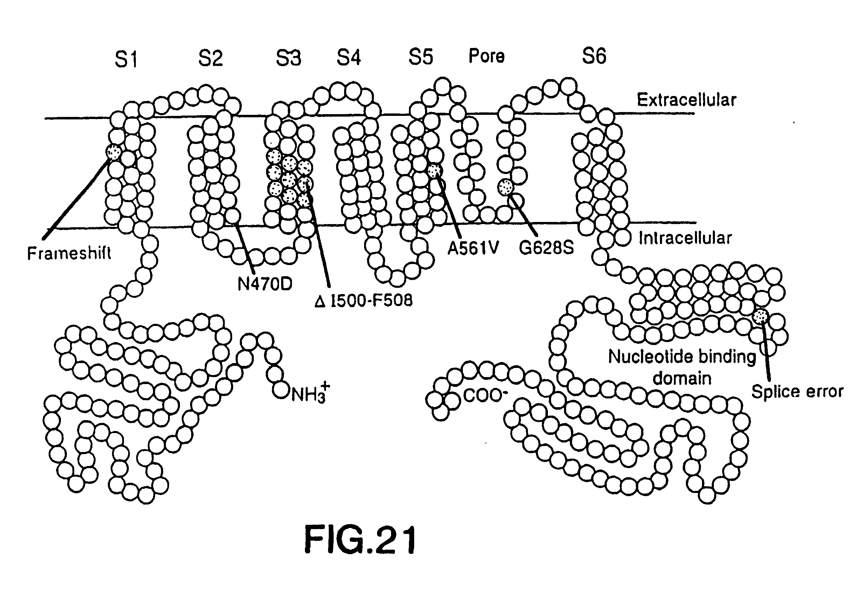

Figure 21. Schematic representation of the predicted topology of the protein encoded by ***HERG*** and the location of LQT-associated mutations. For simplicity, not all amino acids are shown. SSCP analyses identified *HERG* mutations in four of the 14 chromosome 7-linked kindreds.

The present invention is directed to the determination that LQT maps to both the *SCN5A* and *HERG* genes and that molecular variants of these genes cause or are involved in the pathogenesis of hereditary LQT. Furthermore, the $K^+$ channel encoded by the *HERG* gene is associated with the acquired form of LQT. More specifically, the present invention relates to mutations in the *SCNSA* and *HERG* genes and their use in the diagnosis of LQT and to the monitoring and adjustment of $K^+$ levels in patients with familial LQT or in patients undergoing drug therapy for cardiac arrhythmias or who are on other medication which may cause acquired LQT. The present invention is further directed to methods of screening humans for the presence of *SCN5A* and *HERG* gene variants which cause LQT. Since LQT can now be detected earlier (i.e., before symptoms appear) and more definitively, better treatment options will be available in those individuals identified as having hereditary LQT.

The present invention provides methods of screening the *SCN5A* and *HERG* genes to identify mutations. Such methods may further comprise the step of amplifying a portion of the *SCN5A* or *HERG* gene, and may further include a step of providing a set of polynucleotides which are primers for amplification of said portion of the *SCN5A* or *HERG* gene. The method is useful for identifying mutations for use in either diagnosis of LQT or prognosis of LQT.

Long QT syndrome is an inherited or an acquired disorder that causes sudden death from cardiac arrhythmias, specifically *torsade de pointes* and ventricular fibrillation. LQT was previously mapped to three loci: *LQT1* on chromosome 11p15.5, *LQT2* on 7q35-36 and *LQT3* on 3p21-24. It is a discovery of the present invention that there is a genetic linkage between *LQT3* and polymorphisms within *SCN5A*, the cardiac sodium channel gene. It is also a discovery of the present invention that there is a genetic linkage between *LQT2* and polymorphisms within *HERG.* It is a further discovery of the present invention that there is a connection between the $K^+$ channel encoded by the *HERG* gene and the acquired form of LQT. SSCP and DNA sequence analyses reveal identical intragenic deletions of *SCN5A* in affected members of two unrelated LQT families. The deleted sequences reside in a region that is important for channel inactivation. These data suggest that mutations in *SCN5A* cause chromosome 3-linked LQT, and indicate a likely cellular mechanism for this disorder. SSCP and DNA sequence analyses revealed two distinct intragenic deletions of *HERG* in two affected individuals. One mutation caused a 27 bp deletion beginning at position 1498. This deletion disrupts the third membrane spanning domain (S3) of *HERG.* The other deletion was of a single base at position 1261. This results in a frameshift in sequences encoding the first membrane spanning domain (SI), leading to a new stop codon within 12 amino acids. Similar analyses also found three distinct missense mutations in three other affected individuals and a splice-donor mutation in another affected individual.

Proof that the *SCN5A* and *HERG* genes are involved in causing hereditary LQT is obtained by finding sequences in DNA extracted from affected kindred members which create abnormal *SCN5A* or *HERG* gene products or abnormal levels of the gene products. Such LQT susceptibility alleles will co-segregate with the disease in large kindreds. They will also be present at a much higher frequency in non-kindred individuals with LQT than in individuals in the general population. The key is to find mutations which are serious enough to cause obvious disruption to the normal function of the gene product. These mutations can take a number of forms. The most severe forms would be frame shift mutations or large deletions which would cause the gene to code for an abnormal protein or one which would significantly alter protein expression. Less severe disruptive mutations would include small in-frame deletions and nonconservative base pair substitutions which would have a significant effect on the protein produced, such as changes to or from a cysteine residue, from a basic to an acidic amino acid or vice versa, from a hydrophobic to hydrophilic amino acid or vice versa, or other mutations which would affect secondary or tertiary protein structure. Silent mutations or those resulting in conservative amino acid substitutions would not generally be expected to disrupt protein function.

According to the diagnostic and prognostic method of the present invention, alteration of the wild-type *SCN5*A or *HERG* gene is detected. In addition, the method can be performed by detecting the wild-type *SCN5*A or *HERG* gene and confirming the lack of a cause of LQT as a result of these two loci. "Alteration of a wild-type gene" encompasses all forms of mutations including deletions, insertions and point mutations in the coding and noncoding regions. Deletions may be of the entire gene or of only a portion of the gene. Point mutations may result in stop codons, frameshift mutations or amino acid substitutions. Somatic mutations are those which occur only in certain tissues and are not inherited in the germline. Germline mutations can be found in any of a body's tissues and are inherited. Point mutational events may occur in regulatory regions, such as in the promoter of the gene, leading to loss or diminution of expression of the mRNA. Point mutations may also abolish proper RNA processing, leading to loss of expression of the *SCN5A* or *HERG* gene product, or to a decrease in mRNA stability or translation efficiency.

The presence of hereditary LQT may be ascertained by testing any tissue of a human for mutations of the *SCN5A* and/or *HERG* genes. For example, a person who has inherited a germline *SCN5A* or *HERG* mutation would be prone to develop LQT. This can be determined by testing DNA from any tissue of the person's body. Most simply, blood can be drawn and DNA extracted from the cells of the blood. In addition, prenatal diagnosis can be accomplished by testing fetal cells, placental cells or amniotic cells for mutations of the *SCN5A* and/or *HERG* genes. Alteration of a wild-type *SCN5A* or *HERG* allele, whether, for example, by point mutation or deletion, can be detected by any of the means discussed herein.

There are several methods that can be used to detect DNA sequence variation. Direct DNA sequencing, either manual sequencing or automated fluorescent sequencing can detect sequence variation. Another approach is the single-stranded conformation polymorphism assay (SSCP) (Orita et al., 1989). This method does not detect all sequence changes, especially if the DNA fragment size is greater than 200 bp, but can be optimized to detect most DNA sequence variation. The reduced detection sensitivity is a disadvantage, but the increased throughput possible with SSCP makes it an attractive, viable alternative to direct sequencing for mutation detection on a research basis. The fragments which have shifted mobility on SSCP gels are then sequenced to determine the exact nature of the DNA sequence variation. Other approaches based on the detection of mismatches between the two complementary DNA strands include clamped denaturing gel electrophoresis (CDGE) (Sheffield et al., 1991), heteroduplex analysis (HA) (White et al., 1992) and chemical mismatch cleavage (CMC) (Grompe et al., 1989). None of the methods described above will detect large deletions, duplications or insertions, nor will they detect a regulatory mutation which affects transcription or translation of the protein. Other methods which might detect these classes of mutations such as a protein truncation assay or the asymmetric assay, detect only specific types of mutations and would not detect missense mutations. A review of currently available methods of detecting DNA sequence variation can be found in a recent review by Grompe (1993). Once a mutation is known, an allele specific detection approach such as allele specific oligonucleotide (ASO) hybridization can be utilized to rapidly screen large numbers of other samples for that same mutation.

A rapid preliminary analysis to detect polymorphisms in DNA sequences can be performed by looking at a series of Southern blots of DNA cut with one or more restriction enzymes, preferably with a large number of restriction enzymes. Each blot contains a series of normal individuals and a series of LQT cases. Southern blots displaying hybridizing fragments (differing in length from control DNA when probed with sequences near or including the *SCN5A* or *HERG* loci) indicate a possible mutation. If restriction enzymes which produce very large restriction fragments are used, then pulsed field gel electrophoresis (PFGE) is employed.

Detection of point mutations may be accomplished by molecular cloning of the *SCN5A* or *HERG* alleles and sequencing the alleles using techniques well known in the art.

There are six well known methods for a more complete, yet still indirect, test for confirming the presence of a susceptibility allele: 1) single stranded conformation analysis (SSCP) (Orita et al., 1989); 2) denaturing gradient gel electrophoresis (DGGE) (Wartell et al., 1990; Sheffield et al., 1989); 3) RNase protection assays (Finkelstein et al., 1990; Kinszler et al., 1991); 4) allele-specific oligonucleotides (ASOs) (Conner et al., 1983); 5) the use of proteins which recognize nucleotide mismatches, such as the *E. coli* mutS protein (Modrich, 1991); and 6) allele-specific PCR (Rano and Kidd, 1989). For allele-specific PCR, primers are used which hybridize at their 3' ends to a particular *SCN5A* or *HERG* mutation. If the particular mutation is not present, an amplification product is not observed. Amplification Refractory Mutation System (ARMS) can also be used, as disclosed in European Patent Application Publication No. 0332435 and in Newton et al., 1989. Insertions and deletions of genes can also be detected by cloning, sequencing and amplification. In addition, restriction fragment length polymorphism (RFLP) probes for the gene or surrounding marker genes can be used to score alteration of an allele or an insertion in a polymorphic fragment. Such a method is particularly useful for screening relatives of an affected individual for the presence of the mutation found in that individual. Other techniques for detecting insertions and deletions as known in the art can be used.

In the first three methods (SSCP, DGGE and RNase protection assay), a new electrophoretic band appears. SSCP detects a band which migrates differentially because the sequence change causes a difference in single-strand, intramolecular base pairing. RNase protection involves cleavage of the mutant polynucleotide into two or more smaller fragments. DGGE detects differences in migration rates of mutant sequences compared to wild-type sequences, using a denaturing gradient gel. In an allele-specific oligonucleotide assay, an oligonucleotide is designed which detects a specific sequence, and the assay is performed by detecting the presence or absence of a hybridization signal. In the mutS assay, the protein binds only to sequences that contain a nucleotide mismatch in a heteroduplex between mutant and wild-type sequences.

Mismatches, according to the present invention, are hybridized nucleic acid duplexes in which the two strands are not 100% complementary. Lack of total homology may be due to deletions, insertions, inversions or substitutions. Mismatch detection can be used to detect point mutations in the gene or in its mRNA product. While these techniques are less sensitive than sequencing, they are simpler to perform on a large number of samples. An example of a mismatch cleavage technique is the RNase protection method. In the practice of the present invention, the method involves the use of a labeled riboprobe which is complementary to the human wild-type *SCN5A* or *HERG* gene coding sequence. The riboprobe and either mRNA or DNA isolated from the person are annealed (hybridized) together and subsequently digested with the enzyme RNase A which is able to detect some mismatches in a duplex RNA structure. If a mismatch is detected by RNase A, it cleaves at the site of the mismatch. Thus, when the annealed RNA preparation is separated on an electrophoretic gel matrix, if a mismatch has been detected and cleaved by RNase A, an RNA product will be seen which is smaller than the full length duplex RNA for the riboprobe and the mRNA or DNA. The riboprobe need not be the full length of the mRNA or gene but can be a segment of either. If the riboprobe comprises only a segment of the mRNA or gene, it will be desirable to use a number of these probes to screen the whole mRNA sequence for mismatches.

In similar fashion, DNA probes can be used to detect mismatches, through enzymatic or chemical cleavage. See, e.g., Cotton et al., 1988; Shenk et al., 1975; Novack et al., 1986. Alternatively, mismatches can be detected by shifts in the electrophoretic mobility of mismatched duplexes relative to matched duplexes. See, e.g., Cariello, 1988. With either riboprobes or DNA probes, the cellular mRNA or DNA which might contain a mutation can be amplified using PCR (see below) before hybridization. Changes in DNA of the *SCN5A* or *HERG* gene can also be detected using Southern hybridization, especially if the changes are gross rearrangements, such as deletions and insertions.

DNA sequences of the *SCN5A* or *HERG* gene which have been amplified by use of PCR may also be screened using allele-specific probes. These probes are nucleic acid oligomers, each of which contains a region of the gene sequence harboring a known mutation. For example, one oligomer may be about 30 nucleotides in length, corresponding to a portion of the gene sequence.

By use of a battery of such allele-specific probes, PCR amplification products can be screened to identify the presence of a previously identified mutation in the gene. Hybridization of allele-specific probes with amplified *SCN5A* or *HERG* sequences can be performed, for example, on a nylon filter. Hybridization to a particular probe under stringent hybridization conditions indicates the presence of the same mutation in the tissue as in the allele-specific probe.

The most definitive test for mutations in a candidate locus is to directly compare genomic *SCNSA* or *HERG* sequences from patients with those from a control population. Alternatively, one could sequence messenger RNA after amplification, *e.g.,* by PCR, thereby eliminating the necessity of determining the exon structure of the candidate gene.

Mutations from patients falling outside the coding region of *SCN5A* or *HERG* can be detected by examining the non-coding regions, such as introns and regulatory sequences near or within the genes. An early indication that mutations in noncoding regions are important may come from Northern blot experiments that reveal messenger RNA molecules of abnormal size or abundance in patients as compared to control individuals.

Alteration of *SCN5A* or *HERG* mRNA expression can be detected by any techniques known in the art. These include Northern blot analysis, PCR amplification and RNase protection. Diminished mRNA expression indicates an alteration of the wild-type gene. Alteration of wild-type genes can also be detected by screening for alteration of wild-type *SCN5A* or *HERG* protein. For example, monoclonal antibodies immunoreactive with *SCN5A* or *HERG* can be used to screen a tissue. Lack of cognate antigen would indicate a mutation. Antibodies specific for products of mutant alleles could also be used to detect mutant gene product. Such immunological assays can be done in any convenient formats known in the art. These include Western blots immunohistochemical assays and ELISA assays. Any means for detecting an altered *SCNSA* or *HERG* protein can be used to detect alteration of wild-type *SCN5A* or *HERG* genes. Functional assays, such as protein binding determinations, can be used. In addition, assays can be used which detect *SCN5A* or *HERG* biochemical function. Finding a mutant *SCN5A* or *HERG* gene product indicates alteration of a wild-type *SCN5A* or *HERG* gene.

Mutant *SCN5A* or *HERG* genes or gene products can also be detected in other human body samples, such as serum, stool, urine and sputum. The same techniques discussed above for detection of mutant genes or gene products in tissues can be applied to other body samples. By screening such body samples, a simple early diagnosis can be achieved for hereditary LQT.

The primer pairs of the present invention are useful for determination of the nucleotide sequence of a particular *SCN5A* or *HERG* allele using PCR. The pairs of single-stranded DNA primers can be annealed to sequences within or surrounding the *SCN5A* or *HERG* gene on chromosomes 3 or 7 respectively in order to prime amplifying DNA synthesis of the gene itself. A complete set of these primers allows synthesis of all of the nucleotides of the gene coding sequences, i.e., the exons. The set of primers preferably allows synthesis of both intron and exon sequences. Allele-specific primers can also be used. Such primers anneal only to particular *SCN5A* or *HERG* mutant alleles, and thus will only amplify a product in the presence of the mutant allele as a template.

In order to facilitate subsequent cloning of amplified sequences, primers may have restriction enzyme site sequences appended to their 5' ends. Thus, all nucleotides of the primers are derived from *SCN5A* or *HERG* sequences or sequences adjacent to *SCN5A* or *HERG,* except for the few nucleotides necessary to form a restriction enzyme site. Such enzymes and sites are well known in the art. The primers themselves can be synthesized using techniques which are well known in the art. Generally, the primers can be made using oligonucleotide synthesizing machines which are commercially available. Given the sequences of *SCN5A* (Gellens et al., 1992) and *HERG* (Warmke and Ganetzky, 1994), design of particular primers is well within the skill of the art.

The nucleic acid probes provided by the present invention are useful for a number of purposes. They can be used in Southern hybridization to genomic DNA and in the RNase protection method for detecting point mutations already discussed above. The probes can be used to detect PCR amplification products. They may also be used to detect mismatches with the *SCN5A* or *HERG* gene or mRNA using other techniques.

It has been discovered that individuals with the wild-type *SCN5A* and *HERG* genes do not have hereditary LQT. However, mutations which interfere with the function of the *SCN5A* or *HERG* gene products are involved in the pathogenesis of LQT. Thus, the presence of an altered (or a mutant) *SCN5A* or *HERG* gene which produces a protein having a loss of function, or altered function, directly causes LQT which increases the risk of cardiac arrhythmias. In order to detect an *SCN5A* or *HERG* gene mutation, a biological sample is prepared and analyzed for a difference between the sequence of the allele being analyzed and the sequence of the wild-type allele. Mutant *SCN5A* or *HERG* alleles can be initially identified by any of the techniques described above. The mutant alleles are then sequenced to identify the specific mutation of the particular mutant allele. Alternatively, mutant alleles can be initially identified by identifying mutant (altered) proteins, using conventional techniques. The mutant alleles are then sequenced to identify the specific mutation for each allele. The mutations, especially those which lead to an altered function of the protein, are then used for the diagnostic and prognostic methods of the present invention.

The present invention enables treating patients with $K^+$ to decrease the chances of developing LQT and/or *torsade de pointes.* The modulation of HERG by extracellular $K^+$ ($[K^+]_e$) may have physiologic importance. During rapid heart rates, or ischemia, $K^+$ accumulates within intracellular clefts (Gintant et al., 1992). This elevation in $[K^+]_e$ would increase the contribution of HERG to net repolarizing current. HERG may be even more important, therefore, in modulation of action potential duration at high heart rates, or during the initial phase of ischemia.

Definitions

The present invention employs the following definitions:

"Probes". Polynucleotide polymorphisms associated with *SCN5A* and *HERG* alleles which predispose to LQT are detected by hybridization with a polynucleotide probe which forms a stable hybrid with that of the target sequence, under stringent to moderately stringent hybridization and wash conditions. If it is expected that the probes will be perfectly complementary to the target sequence, stringent conditions will be used. Hybridization stringency may be lessened if some mismatching is expected, for example, if variants are expected with the result that the probe will not be completely complementary. Conditions are chosen which rule out nonspecific/adventitious bindings, that is, which minimize noise. Since such indications identify neutral DNA polymorphisms as well as mutations, these indications need further analysis to demonstrate detection of an *SCN5A* or *HERG* susceptibility allele.

Probes for *SCN5A* or *HERG* alleles may be derived from the sequences of the *SCN5A* or *HERG* region or their cDNAs. The probes may be of any suitable length, which span all or a portion of the *SCN5A* or *HERG* region, and which allow specific hybridization to the region. If the target sequence contains a sequence identical to that of the probe, the probes may be short, e.g., in the range of about 8-30 base pairs, since the hybrid will be relatively stable under even stringent conditions. If some degree of mismatch is expected with the probe, i.e., if it is suspected that the probe will hybridize to a variant region, a longer probe may be employed which hybridizes to the target sequence with the requisite specificity.

The probes will include an isolated polynucleotide attached to a label or reporter molecule and may be used to isolate other polynucleotide sequences, having sequence similarity by standard methods. For techniques for preparing and labeling probes see, e.g., Sambrook et al., 1989 or Ausubel et al., 1992. Other similar polynucleotides may be selected by using homologous polynucleotides. Alternatively, polynucleotides encoding these or similar polypeptides may be synthesized or selected by use of the redundancy in the genetic code. Various codon substitutions may be introduced, e.g., by silent changes (thereby producing various restriction sites) or to optimize expression for a particular system. Mutations may be introduced to modify the properties of the polypeptide, perhaps to change the polypeptide degradation or turnover rate.

Probes comprising synthetic oligonucleotides or other polynucleotides of the present invention may be derived from naturally occurring or recombinant single- or double-stranded polynucleotides, or be chemically synthesized. Probes may also be labeled by nick translation, Klenow fill-in reaction, or other methods known in the art.

Portions of the polynucleotide sequence having at least about eight nucleotides, usually at least about 15 nucleotides, and fewer than about 6 kb, usually fewer than about 1.0 kb, from a polynucleotide sequence encoding *SCN5A* or *HERG* are preferred as probes. The probes may also be used to determine whether mRNA encoding *SCN5A* or *HERG* is present in a cell or tissue.

**"Regulatory sequences"** refers to those sequences normally within 100 kb of the coding region of a locus, but they may also be more distant from the coding region, which affect the expression of the gene (including transcription of the gene, and translation, splicing, stability or the like of the messenger RNA).

**"Substantial homology or similarity".** A nucleic acid or fragment thereof is "substantially homologous" ("or substantially similar") to another if, when optimally aligned (with appropriate nucleotide insertions or deletions) with the other nucleic acid (or its complementary strand), there is nucleotide sequence identity in at least about 60% of the nucleotide bases, usually at least about 70%, more usually at least about 80%, preferably at least about 90%, and more preferably at least about 95-98% of the nucleotide bases.

Alternatively, substantial homology or (similarity) exists when a nucleic acid or fragment thereof will hybridize to another nucleic acid (or a complementary strand thereof) under selective hybridization conditions, to a strand, or to its complement. Selectivity of hybridization exists when hybridization which is substantially more selective than total lack of specificity occurs. Typically, selective hybridization will occur when there is at least about 55% homology over a stretch of at least about 14 nucleotides, preferably at least about 65%, more preferably at least about 75%, and most preferably at least about 90%. See, Kanehisa, 1984. The length of homology comparison, as described, may be over longer stretches, and in certain embodiments will often be over a stretch of at least about nine nucleotides, usually at least about 20 nucleotides, more usually at least about 24 nucleotides, typically at least about 28 nucleotides, more typically at least about 32 nucleotides, and preferably at least about 36 or more nucleotides.

Nucleic acid hybridization will be affected by such conditions as salt concentration, temperature, or organic solvents, in addition to the base composition, length of the complementary strands, and the number of nucleotide base mismatches between the hybridizing nucleic acids, as will be readily appreciated by those skilled in the art. Stringent temperature conditions will generally include temperatures in excess of 30°C, typically in excess of 37°C, and preferably in excess of 45°C. Stringent salt conditions will ordinarily be less than 1000 mM, typically less than 500 mM, and preferably less than 200 mM. However, the combination of parameters is much more important than the measure of any single parameter. See, e.g., Wetmur & Davidson, 1968.

Probe sequences may also hybridize specifically to duplex DNA under certain conditions to form triplex or other higher order DNA complexes. The preparation of such probes and suitable hybridization conditions are well known in the art.

Preparation of recombinant or chemically synthesized nucleic acids: vectors transformation, host cells

Large amounts of the polynucleotides of the present invention may be produced by replication in a suitable host cell. Natural or synthetic polynucleotide fragments coding for a desired fragment will be incorporated into recombinant polynucleotide constructs, usually DNA constructs, capable of introduction into and replication in a prokaryotic or eukaryotic cell. Usually the polynucleotide constructs will be suitable for replication in a unicellular host, such as yeast or bacteria, but may also be intended for introduction to (with and without integration within the genome) cultured mammalian or plant or other eukaryotic cell lines. The purification of nucleic acids produced by the methods of the present invention are described, e.g., in Sambrook et al., 1989 or Ausubel et al., 1992.

The polynucleotides of the present invention may also be produced by chemical synthesis, e.g., by the phosphoramidite method described by Beaucage & Carruthers, 1981 or the triester method according to Matteucci et aL, 1981, and may be performed on commercial, automated oligonucleotide synthesizers. A double-stranded fragment may be obtained from the single-stranded product of chemical synthesis either by synthesizing the complementary strand and annealing the strand together under appropriate conditions or by adding the complementary strand using DNA polymerase with an appropriate primer sequence.

Polynucleotide constructs prepared for introduction into a prokaryotic or eukaryotic host may comprise a replication system recognized by the host, including the intended polynucleotide fragment encoding the desired polypeptide, and will preferably also include transcription and translational initiation regulatory sequences operably linked to the polypeptide encoding segment. Expression vectors may include, for example, an origin of replication or autonomously replicating sequence (ARS) and expression control sequences, a promoter, an enhancer and necessary processing information sites, such as ribosome-binding sites, RNA splice sites, polyadenylation sites, transcriptional terminator sequences, and mRNA stabilizing sequences. Such vectors may be prepared by means of standard recombinant techniques well known in the art and discussed, for example, in Sambrook et al., 1989 or Ausubel et al., 1992.

An appropriate promoter and other necessary vector sequences will be selected so as to be functional in the host, and may include, when appropriate, those naturally associated with *SCN5A* or *HERG* genes. Examples of workable combinations of cell lines and expression vectors are described in Sambrook et al., 1989 or Ausubel et al., 1992; see also, e.g., Metzger et al., 1988. Many useful vectors are known in the art and may be obtained from such vendors as Stratagene, New England Biolabs, Pmmega Biotech, and others. Promoters such as the trp, lac and phage promoters, tRNA promoters and glycolytic enzyme promoters may be used in prokaryotic hosts. Useful yeast promoters include promoter regions for metallothionein, 3-phosphoglycerate kinase or other glycolytic enzymes such as enolase or glyceraldehyde-3-phosphate dehydrogenase, enzymes responsible for maltose and galactose utilization, and others. Vectors and promoters suitable for use in yeast expression are further described in Hitzernan et al., EP 73,675A. Appropriate nonnative mammalian promoters might include the early and late promoters from SV40 (Fiers et al., 1978) or promoters derived from murine Molony leukemia virus, mouse tumor virus, avian sarcoma viruses, adenovirus II, bovine papilloma virus or polyoma. In addition, the construct may be joined to an amplifiable gene (e.g., DHFR) so that multiple copies of the gene may be made. For appropriate enhancer and other expression control sequences, see also *Enhancers and Eukaryotic Gene Expression,* Cold Spring Harbor Press, Cold Spring Harbor, New York (1983).

While such expression vectors may replicate autonomously, they may also replicate by being inserted into the genome of the host cell, by methods well known in the art.

Expression and cloning vectors will likely contain a selectable marker, a gene encoding a protein necessary for survival or growth of a host cell transformed with the vector. The presence of this gene ensures growth of only those host cells which express the inserts. Typical selection genes encode proteins that a) confer resistance to antibiotics or other toxic substances, e.g. ampicillin, neomycin, methotrexate, etc., b) complement auxotrophic deficiencies, or c) supply critical nutrients not available from complex media, e.g., the gene encoding D-alanine racemase for *Bacilli.* The choice of the proper selectable marker will depend on the host cell, and appropriate markers for different hosts are well known in the art.

The vectors containing the nucleic acids of interest can be transcribed *in vitro,* and the resulting RNA introduced into the host cell by well-known methods, e.g., by injection (see, Kubo et al., 1988), or the vectors can be introduced directly into host cells by methods well known in the art, which vary depending on the type of cellular host, including electroporation; transfection employing calcium chloride, rubidium chloride calcium phosphate, DEAE-dextran, or other substances; microprojectile bombardment; lipofection; infection (where the vector is an infectious agent, such as a retroviral genome); and other methods. See generally, Sambrook et al., 1989 and Ausubel et al., 1992. The introduction of the polynucleotides into the host cell by any method known in the art, including, *inter alia,* those described above, will be referred to herein as "transformation." The cells into which have been introduced nucleic acids described above are meant to also include the progeny of such cells.

Large quantities of the nucleic acids and polypeptides of the present invention may be prepared by expressing the *SCN5A* or *HERG* nucleic acids or portions thereof in vectors or other expression vehicles in compatible prokaryotic or eukaryotic host cells. The most commonly used prokaryotic hosts are strains of *Escherichia coli,* although other prokaryotes, such as *Bacillus subtilis* or *Pseudomonas* may also be used.

Mammalian or other eukaryotic host cells, such as those of yeast, filamentous fungi, plant, insect, or amphibian or avian species, may also be useful for production of the proteins of the present invention. Propagation of mammalian cells in culture is *per se* well known. See, Jakoby and Pastan (eds.), 1979. Examples of commonly used mammalian host cell lines are VERO and HeLa cells, Chinese hamster ovary (CHO) cells, and WI38, BHK, and COS cell lines, although it will be appreciated by the skilled practitioner that other cell lines may be appropriate, e.g., to provide higher expression, desirable glycosylation patterns, or other features.

Clones are selected by using markers depending on the mode of the vector construction. The marker may be on the same or a different DNA molecule, preferably the same DNA molecule. In prokaryotic hosts, the transformant may be selected, *e.g.*, by resistance to ampicillin, tetracycline or other antibiotics. Production of a particular product based on temperature sensitivity may also serve as an appropriate marker.

Prokaryotic or eukaryotic cells transformed with the polynucleotides of the present invention will be useful not only for the production of the nucleic acids and polypeptides of the present invention, but also, for example, in studying the characteristics of *SCN5A* and *HERG* polypeptides.

The probes and primers based on the *SCN5A* and *HERG* gene sequences disclosed herein are used to identify homologous *SCN5A* and *HERG* gene sequences and proteins in other species. These gene sequences and proteins are used in the diagnostic/prognostic, therapeutic and drug screening methods described herein for the species from which they have been isolated.

Methods of Use: Nucleic Acid Diagnosis and Diagnostic Kits

In order to detect the presence of an *SCN5A* or *HERG* allele predisposing an individual to LQT, a biological sample such as blood is prepared and analyzed for the presence or absence of susceptibility alleles of *SCN5A* or *HERG.* In order to detect the presence of LQT or as a prognostic indicator, a biological sample is prepared and analyzed for the presence or absence of mutant alleles of *SCN5A* and/or *HERG.* Results of these tests and interpretive information are returned to the health care provider for communication to the tested individual. Such diagnoses may be performed by diagnostic laboratories, or, alternatively, diagnostic kits are manufactured and sold to health care providers or to private individuals for self-diagnosis.

Initially, the screening method involves amplification of the relevant *SCN5A* or *HERG* sequences. In another preferred embodiment of the invention, the screening method involves a non-PCR based strategy. Such screening methods include two-step label amplification methodologies that are well known in the art. Both PCR and non-PCR based screening strategies can detect target sequences with a high level of sensitivity.

The most popular method used today is target amplification. Here, the target nucleic acid sequence is amplified with polymerases. One particularly preferred method using polymerase-driven amplification is the polymerase chain reaction (PCR). The polymerase chain reaction and other polymerase-driven amplification assays can achieve over a million-fold increase in copy number through the use of polymerase-driven amplification cycles. Once amplified, the resulting nucleic acid can be sequenced or used as a substrate for DNA probes.

When the probes are used to detect the presence of the target sequences the biological sample to be analyzed, such as blood or serum, may be treated, if desired, to extract the nucleic acids. The sample nucleic acid may be prepared in various ways to facilitate detection of the target sequence, e.g. denaturation, restriction digestion, electrophoresis or dot blotting. The targeted region of the analyte nucleic acid usually must be at least partially single-stranded to form hybrids with the targeting sequence of the probe. If the sequence is naturally single-stranded, denaturation will not be required. However, if the sequence is double-stranded, the sequence will probably need to be denatured. Denaturation can be carried out by various techniques known in the art.

Analyte nucleic acid and probe are incubated under conditions which promote stable hybrid formation of the target sequence in the probe with the putative targeted sequence in the analyte. The region of the probes which is used to bind to the analyte can be made completely complementary to the targeted region of human chromosome 3 or 7. Therefore, high stringency conditions are desirable in order to prevent false positives. However, conditions of high stringency are used only if the probes are complementary to regions of the chromosome which are unique in the genome. The stringency of hybridization is determined by a number of factors during hybridization and during the washing procedure, including temperature, ionic strength, base composition, probe length, and concentration of formamide. These factors are outlined in, for example, Maniatis et al., 1982 and Sambrook et al., 1989. Under certain circumstances, the formation of higher order hybrids, such as triplexes, quadraplexes, etc., may be desired to provide the means of detecting target sequences.

Detection, if any, of the resulting hybrid is usually accomplished by the use of labeled probes. Alternatively, the probe may be unlabeled, but may be detectable by specific binding with a ligand which is labeled, either directly or indirectly. Suitable labels, and methods for labeling probes and ligands are known in the art, and include, for example, radioactive labels which may be incorporated by known methods (e.g., nick translation, random priming or kinasing), biotin, fluorescent groups, chemiluminescent groups (e.g., dioxetanes, particularly triggered dioxetanes), enzymes, antibodies and the like. Variations of this basic scheme are known in the art, and include those variations that facilitate separation of the hybrids to be detected from extraneous materials and/or that amplify the signal from the labeled moiety. A number of these variations are reviewed in, e.g., Matthews & Kricka, 1988; Landegren et al., 1988; Mittlin, 1989; U.S. Patent 4,868,105; and in EPO Publication No. 225,807.

As noted above, non-PCR based screening assays are also contemplated in this invention. This procedure hybridizes a nucleic acid probe (or an analog such as a methyl phosphonate backbone replacing the normal phosphodiester), to the low level DNA target. This probe may have an enzyme covalently linked to the probe, such that the covalent linkage does not interfere with the specificity of the hybridization. This enzyme-probe-conjugate-target nucleic acid complex can then be isolated away from the free probe enzyme conjugate and a substrate is added for enzyme detection. Enzymatic activity is observed as a change in color development or luminescent output resulting in a $10^3$-$10^6$ increase in sensitivity. For an example relating to the preparation of oligodeoxynucleotide-alkaline phosphatase conjugates and their use as hybridization probes, see Jablonski et al., 1986.

Two-step label amplification methodologies are known in the art. These assays work on the principle that a small ligand (such as digoxigenin, biotin, or the like) is attached to a nucleic acid probe capable of specifically binding *SCN5A* or *HERG.* Allele specific probes are also contemplated within the scope of this example and exemplary allele specific probes include probes encompassing the predisposing mutations of this patent application.

In one example, the small ligand attached to the nucleic acid probe is specifically recognized by an antibody-enzyme conjugate. In one embodiment of this example, digoxigenin is attached to the nucleic acid probe. Hybridization is detected by an antibody-alkaline phosphatase conjugate which turns over a chemiluminescent substrate. For methods for labeling nucleic acid probes according to this embodiment see Martin et al., 1990. In a second example, the small ligand is recognized by a second ligand-enzyme conjugate that is capable of specifically complexing to the first ligand. A well known embodiment of this example is the biotin-avidin type of interactions. For methods for labeling nucleic acid probes and their use in biotin-avidin based assays see Rigby et al., 1977 and Nguyen et al., 1992.

It is also contemplated within the scope of this invention that the nucleic acid probe assays of this invention will employ a cocktail of nucleic acid probes capable of detecting *SCN5A* and/or *HERG.* Thus, in one example to detect the presence of *SCN5A* or *HERG* in a cell sample, more than one probe complementary to the gene is employed and in particular the number of different probes is alternatively two, three, or five different nucleic acid probe sequences. In another example, to detect the presence of mutations in the *SCN5A* or *HERG* gene sequence in a patient, more than one probe complementary to these genes is employed where the cocktail includes probes capable of binding to the allele-specific mutations identified in populations of patients with alterations in *SCN5A* or *HERG.* In this embodiment, any number of probes can be used, and will preferably include probes corresponding to the major gene mutations identified as predisposing an individual to LQT.

Methods of Use: Peptide Diagnosis and Diagnostic Kits

The presence of LQT can also be detected on the basis of the alteration of wild-type *SCN5A* or *HERG* polypeptide. Such alterations can be determined by sequence analysis in accordance with conventional techniques. More preferably, antibodies (polyclonal or monoclonal) are used to detect differences in, or the absence of *SCNSA* or *HERG* peptides. Techniques for raising and purifying antibodies are well known in the art and any such techniques may be chosen to achieve the preparations claimed in this invention. In a preferred embodiment of the invention, antibodies will immunoprecipitate *SCN5A* or *HERG* proteins from solution as well as react with these proteins on Western or immunoblots of polyacrylamide gels. In another preferred embodiment, antibodies will detect *SCN5A* or *HERG* proteins in paraffin or frozen tissue sections, using immunocytochemical techniques.

Preferred embodiments relating to methods for detecting *SCN5A* or *HERG* or their mutations include enzyme linked immunosorbent assays (ELISA), radioimmunoassays (RIA), immunoradiometric assays (IRMA) and immunoenzymatic assays (IEMA), including sandwich assays using monoclonal and/or polyclonal antibodies. Exemplary sandwich assays are described by David et al., in U.S. Patent Nos. 4,376.110 and 4,486,530, hereby incorporated by reference.

Methods of Use: Gene Therapy

According to the present invention, a method is also provided of supplying wild-type *SCN5A* or *HERG* function to a cell which carries mutant *SCN5A* or *HERG* alleles. Supplying such a function should allow normal functioning of the recipient cells. The wild-type gene or a part of the gene may be introduced into the cell in a vector such that the gene remains extrachromosomal. In such a situation, the gene will be expressed by the cell from the extrachromosomal location. More preferred is the situation where the wild-type gene or a part thereof is introduced into the mutant cell in such a way that it recombines with the endogenous mutant gene present in the cell. Such recombination requires a double recombination event which results in the correction of the gene mutation. Vectors for introduction of genes both for recombination and for extrachromosomal maintenance are known in the art, and any suitable vector may be used. Methods for introducing DNA into cells such as electroporation, calcium phosphate co-precipitation and viral transduction are known in the art, and the choice of method is within the competence of the practitioner.

As generally discussed above, the *SCN5A* or *HERG* gene or fragment, where applicable, may be employed in gene therapy methods in order to increase the amount of the expression products of such genes in cells. It may also be useful to increase the level of expression of a given LQT gene even in those heart cells in which the mutant gene is expressed at a "normal" level, but the gene product is not fully functional.

Gene therapy would be carried out according to generally accepted methods, for example, as described by Friedman, 1991. Cells from a patient would be first analyzed by the diagnostic methods described above, to ascertain the production of *SCN5A or HERG* polypeptide in the cells. A virus or plasmid vector (see further details below), containing a copy of the *SCN5A* or *HERG* gene linked to expression control elements and capable of replicating inside the cells, is prepared. Suitable vectors are known, such as disclosed in U.S. Patent 5,252,479 and PCT published application WO 93/07282. The vector is then injected into the patient. If the transfected gene is not permanently incorporated into the genome of each of the targeted cells, the treatment may have to be repeated periodically.

Gene transfer systems known in the art may be useful in the practice of the gene therapy methods of the present invention. These include viral and non viral transfer methods. A number of viruses have been used as gene transfer vectors. including papovaviruses (e.g., SV40, Madzal: et al.. 1992), adenovirus (Berkner. 1992: Berkner et al., 1988; Gorziglia and Kapikian. 1992; Quantin et al., 1992; Rosenfeld et al., 1992; Wilkinson et al., 1992; Stratford-Perricaudet et al., 1990), vaccinia virus (Moss, 1992), adeno-associated virus (Muzyczka, 1992; Ohi et al., 1990), herpesviruses including HSV and EBV (Margolskee, 1992; Johnson et al., 1992; Fink et al., 1992; Breakfield and Geller, 1987; Freese et al., 1990), and retroviruses of avian (Brandyopadhyay and Temin, 1984; Petropoulos et al., 1992), murine (Miller, 1992; Miller et al., 1985; Sorge et al., 1984; Mann and Baltimore, 1985; Miller et al., 1988), and human origin (Shimada et al., 1991; Helseth et al., 1990; Page et al., 1990; Buchschacher and Panganiban, 1992). Most human gene therapy protocols have been based on disabled murine retroviruses.

Nonviral gene transfer methods known in the art include chemical techniques such as calcium phosphate coprecipitation (Graham and van der Eb, 1973; Pellicer et al., 1980); mechanical techniques, for example microinjection (Anderson et al., 1980; Gordon et al., 1980; Brinster et al., 1981; Constantini and Lacy, 1981); membrane fusion-mediated transfer via liposomes (Felgner et al., 1987; Wang and Huang, 1989; Kaneda et al., 1989; Stewart et al., 1992; Nabel et al., 1990; Lim et al., 1992); and direct DNA uptake and receptor-mediated DNA transfer (Wolff et al., 1990; Wu et al., 1991; Zenke et al., 1990; Wu et al., 1989; Wolff et al., 1991; Wagner et al., 1990; Wagner et al., 1991; Cotten et al., 1990; Curiel et al., 1991a; Curiel et al., 1991 b).

In an approach which combines biological and physical gene transfer methods, plasmid DNA of any size is combined with a polylysine-conjugated antibody specific to the adenovirus hexon protein, and the resulting complex is bound to an adenovirus vector. The trimolecular complex is then used to infect cells. The adenovirus vector permits efficient binding, internalization, and degradation of the endosome before the coupled DNA is damaged.

Liposome/DNA complexes have been shown to be capable of mediating direct *in vivo* gene transfer. While in standard liposome preparations the gene transfer process is nonspecific, localized *in vivo* uptake and expression have been reported in tumor deposits, for example, following direct *in situ* administration (Nabel, 1992).

Gene transfer techniques which target DNA directly to heart tissue is preferred. Receptor-mediated gene transfer, for example, is accomplished by the conjugation of DNA (usually in the form of covalently closed supercoiled plasmid) to a protein ligand via polylysine. Ligands are chosen on the basis of the presence of the corresponding ligand receptors on the cell surface of the target cell/tissue type. These ligand-DNA conjugates can be injected directly into the blood if desired and are directed to the target tissue where receptor binding and internalization of the DNA-protein complex occurs. To overcome the problem of intracellular destruction of DNA, coinfection with adenovirus can be included to disrupt endosome function.

The therapy is as follows: patients who carry an *SCNSA* or *HERG* susceptibility allele are treated with a gene delivery vehicle such that some or all of their heart precursor cells receive at least one additional copy of a functional normal *SCN5A* or *HERG* allele. In this step, the treated individuals have reduced risk of LQT to the extent that the effect of the susceptible allele has been countered by the presence of the normal allele.

<u>Methods of Use: Transformed Hosts</u>

Animals for testing therapeutic agents can be selected after mutagenesis of whole animals or after treatment of germline cells or zygotes. Such treatments include insertion of mutant *SCN5A* or *HERG* alleles, usually from a second animal species, as well as insertion of disrupted homologous genes. Alternatively, the endogenous *SCN5A* or *HERG* gene(s) of the animals may be disrupted by insertion or deletion mutation or other genetic alterations using conventional techniques (Capecchi, 1989; Valancius and Smithies, 1991; Hasty et al., 1991; Shinkai et aL, 1992; Mombaerts et al., 1992; Philpott et al., 1992; Snouwaert et al., 1992; Donehower et al., 1992). After test substances have been administered to the animals, the presence of LQT must be assessed. If the test substance prevents or suppresses the appearance of LQT, then the test substance is a candidate therapeutic agent for treatment of LQT. These animal models provide an extremely important testing vehicle for potential therapeutic products.

<u>Methods of Preventing LQT and *Torsade de Pointes*</u>

There is a variety of ways for LQT to develop. Mutations in specific genes, e.g. *HERG* and *SCN5A,* can cause LQT. Treatment with any of a variety of drugs can also cause LQT. These drugs include those being taken to treat cardiac arrhythmias and also other drugs including antihistamines and some antibiotics such as erythromycin. Regardless of whether the LQT is a result of mutations (hereditary or familial LQT) or drug induced (acquired LQT), it is due to an effect on an ion channel. The drugs interact with the $K^+$ channel $I_{Kr}$, the major subunit of which is encoded by *HERG,* thereby affecting $K^+$ flow in cardiac cells. Mutations in *HERG* also can affect $K^+$ flow through this channel. This can result in long QT syndrome and may lead to *torsade de pointes.* It has been found that elevation of extracellular $K^+$ causes an increase in outward HERG current. This is a paradoxical effect, since an increase of extracellular $K^+$ lowers the chemical driving force for outward $K^+$ flux and therefore, would be expected to decrease, rather than increase, outward current. This observation indicates that increasing extracellular $K^+$ will activate this $K^+$ channel. This activation can prevent LQT which could otherwise develop from at least partial inactivation of the channel as a result of a mutation in *HERG* or a result of drug treatment. A normal extracellular physiological $K^+$ concentration, as measured in serum, in humans is in the range of about 3.5-4.5 mM. Values in the range of 3-5 mM are frequently seen, less frequently values in the range 2-3 or 5-7 mM are seen. Occasionally values lower than 2 mM or higher than 7 mM are seen. It was found that the HERG current at an extracellular $K^+$ concentration of 5 mM is 40% greater than the current seen at 2 mM. Potentiation of this $K^+$ channel by increasing extracellular $K^+$ levels is beneficial. During rapid heart rates, or ischemia, $K^+$ accumulates within intracellular clefts. Raising extracellular $K^+$ should increase the outward current thereby reducing this intracellular accumulation. Monitoring extracellular $K^+$ levels in persons with hereditary forms of LQT or those on medications which can cause acquired LQT, will allow physicians to prescribe added $K^+$ to those patients with lower than normal or even at normal extracellular $K^+$ levels. By increasing these extracellular $K^+$ levels to at least normal levels of 3.5-4.5 mM, preferably above normal levels to 4.5-5.5 mM, most preferably to about 5 mM $K^+$, the development of LQT and/or *torsade de pointes* will be inhibited. This new knowledge of the causes of LQT will lead to a system of monitoring extracellular $K^+$ levels in patients at risk of developing LQT, either hereditary or acquired, and administering $K^+$ to those with low or even normal extracellular $K^+$ levels. Such treatment will lead to the prevention of LQT and/or *torsades de pointes.*

Two strategies have been utilized herein to identify LQT genes, a candidate gene approach and positional cloning. Positional information is now available for three LQT loci with *LQT1* having been mapped to chromosome 11p15.5 (Keating et al., 1991 a; Keating et al., 1991b), *LQT2* to 7q35-36 and *LQT3* to 3p21-24 (Jiang et al., 1994). The candidate gene approach relies on likely mechanistic hypotheses based on physiology. Although little is known about the physiology of LQT, the disorder is associated with prolongation of the QT interval on electrocardiograms, a sign of abnormal cardiac repolarization. This association suggests that genes encoding ion channels, or their modulators, are reasonable candidates for LQT. This hypothesis is now supported by the discovery that chromosome 7-linked LQT results from mutations in *HERG,* a cardiac potassium channel gene. A neuroendocrine calcium channel gene *(CACNLI A2;* Chin et al., 1991; Seino et al., 1992) and a gene encoding a-GTP-binding protein that modulates potassium channels *(GNAI2;* Weinstein et al., 1988; Magovcevic et al., 1992) became candidates for *LQT3* based on their chromosomal location. Subsequent linkage analyses, however, have excluded these genes (Wang and Keating, unpublished data).

In theory, mutations in a cardiac sodium channel gene could cause LQT. Voltage-gated sodium channels mediate rapid depolarization in ventricular myocytes, and also conduct a small current during the plateau phase of the action potential (Attwell et al., 1979). Subtle abnormalities of sodium channel function (e.g., delayed sodium channel inactivation or altered voltage-dependence of channel inactivation) could delay cardiac repolarization, leading to QT prolongation and arrhythmias. In 1992, Gellens and colleagues cloned and characterized a cardiac sodium channel gene, *SCN5A* (Gellens et al., 1992). The structure of this gene was similar to other, previously characterized sodium channels, encoding a large protein of 2016 amino acids. These channel proteins contain four homologous domains (DI-DIV), each of which contains six putative membrane spanning segments (S1-S6). *SCN5A* was recently mapped to chromosome 3p21, making it an excellent candidate gene for *LQT3* (George et al., 1995).

The present invention has used genotypic analyses to show that *SCN5A* is tightly linked to *LQT3* in three unrelated families (details provided in the Examples). The same intragenic deletion of *SCN5A* appears in affected members of two of these families. Both families are North American of European descent, one German and the other English. Examination of a genealogy database failed to reveal a relationship between these families for more than eight generations. Furthermore, genotypic analyses of these kindreds indicated different haplotypes on the disease chromosome as shown in Figure 1. Thus it is unlikely these families are related. This deletion is due to a nine base pair deletion resulting in a deletion of three amino acids, $K_{1505}$-$P_{1506}$-$Q_{1507}$ (KPQ) in the cytoplasmic linker between DIII and DIV. These deletions disrupted sequences within a region of known importance for sodium channel inactivation, suggesting a likely cellular mechanism for chromosome 3-linked LQT.

Additional evidence supports a role for *SCN5A* in the pathogenesis of LQT. Pharmacologic data, for example, suggest that abnormal cardiac sodium channel function could cause LQT. Toxins and drugs that slow the rate of sodium channel inactivation, or shift the voltage-dependence of channel activation or inactivation, can prolong cardiac action potential duration and induce arrhythmias (Honerjager, 1982). Molecular genetic studies also support a link between sodium channel inactivation and clinical electrophysiologic abnormalities. Studies of hyperkalemic periodic paralysis and paramyotonia congenita indicate that missense mutations in the skeletal muscle sodium channel gene *(SCN4A)* cause myotonia (Fontaine et al., 1990; Ptacek et al., 1991, 1992; Rojas et al., 1991; McClatchey et al., 1992b, 1992c; Ebers et al., 1991; Rudolph et al., 1992; Lerche et al., 1993). Physiologic data show that these mutations affect sodium channel inactivation, leading to repetitive depolarizations, consistent with the myotonic phenotype (Yang et al., 1994). By analogy, similar mutations in the cardiac sodium channel gene could cause a phenotype like LQT.

The mutations in *HERG,* a cardiac potassium channel gene, cause the chromosome 7-linked form of hereditary LQT (details provided in Examples). The mutations identified in *HERG,* and the biophysics of potassium channel alpha subunits, suggest that chromosome 7-linked hereditary LQT results from dominant-negative mutations and a resultant reduction in functional channels. In chromosome 3-linked LQT, by contrast, the LQT-associated deletions identified in *SCN5A* are likely to result in functional cardiac sodium channels with altered properties, such as delayed inactivation or altered voltage-dependence of channel inactivation. Delayed sodium channel inactivation would increase inward sodium current, depolarizing the membrane. This affect is similar to the altered membrane potential expected from *HERG* mutations where outward potassium current is decreased. It is unlikely that more deleterious mutations of *SCN5A* would cause LQT. A reduction of the total number of cardiac sodium channels, for example, would be expected to reduce action potential duration, a phenotype opposite that of LQT. The present invention describes mutations which cause the deletion of three amino acids, KPQ, in the cytoplasmic linker between DIII and DIV. The KPQ sequence is highly conserved, suggesting the presence of genetic pressure for its conservation during evolution (Figure 2). The cytoplasmic peptide segment that links DIII and DIV is the region responsible for fast inactivation (West et al., 1992). Heterologous expression of neural sodium channels in the form of two polypeptides lacking this DIIIIDIV linker results in a sodium current with greatly slowed inactivation (Stuhmer et al., 1989). Site-directed mutagenesis studies of this region in *SCN4A* have focused on another amino acid triplet, $I_{1488}$-$F_{1489}$-$M_{1490}$ (IFM).

Mutations of these amino acids to glutamine residues eliminate sodium channel fast inactivation, but leave slow inactivation intact (West et al., 1992). It will be of interest to determine if the KPQ deletion has a similar functional effect on cardiac sodium channel inactivation.

The data predict a likely cellular mechanism for chromosome 3-linked LQT. Delayed myocellular sodium channel inactivation would prolong action potential duration and the QT interval. Excessive prolongation could result in reactivation of L-type calcium or sodium channels, thereby leading to secondary depolarizations, a likely mechanism of *torsades de pointes* (Antzelevitch and Sicouri, 1994).

No mutation has yet been identified in *SCN5A* in kindred 2171, the third chromosome 3-linked LQT family. Although the disease phenotype in this kindred appears to be linked to *SCN5A,* SSCP analyses of sequences encoding the putative inactivation region failed to show a deletion or other anomalies. Presumably, the disease in this family results from *SCN5A* mutations in another region of this approximately 35 kb gene. Mutational analyses of hyperkalemic periodic paralysis and paramyotonia congenita families have demonstrated several other regions of *SCN4A* that are important for channel inactivation (Fontaine et al., 1990; Ptacek et al., 1991, 1992; Rojas et al., 1991; McClatchey et al., 1992b, 1992c; Ebers et al., 1991; Rudolph et al., 1992; Lerche et al., 1993).

Although LQT kindreds 2321 and 2322 appear unrelated, both had the same KPQ deletion. The haplotype of the affected chromosome in each family was distinct, but this does not exclude the possibility of a distant relationship. Continued genotypic analysis of sequences near *SCN5A* may help determine if these deletions arose separately, or in a single progenitor.

Presymptomatic diagnosis of LQT has depended on identification of QT prolongation on electrocardiograms. Unfortunately, electrocardiograms are rarely performed in young, healthy individuals. In addition, many LQT gene carriers have relatively normal QT intervals, and the first sign of disease can be a fatal cardiac arrhythmia (Vincent et al., 1992). Now that two LQT genes have been identified, genetic testing for this disorder can be contemplated. This will require continued mutational analyses and identification of additional LQT genes. With more detailed phenotypic analyses, phenotypic differences between the varied forms of LQT may be discovered. These differences may be useful for diagnosis and treatment.

The identification of the association between the *SCN5A* and *HERG* gene mutations and hereditary LQT permits the early presymptomatic screening of individuals to identify those at risk for developing LQT. To identify such individuals, the *SCN5A* and/or *HERG* alleles are screened for mutations either directly or after cloning the alleles. The alleles are tested for the presence of nucleic acid sequence differences from the normal allele using any suitable technique, including but not limited to, one of the following methods: fluorescent *in situ* hybridization (FISH), direct DNA sequencing, PFGE analysis, Southern blot analysis, single stranded conformation analysis (SSCP), linkage analysis, RNase protection assay, allele specific oligonucleotide (ASO) dot blot analysis and PCR-SSCP analysis. For example, either (1) the nucleotide sequence of both the cloned alleles and normal *SCN5A* gene or appropriate fragment (coding sequence or genomic sequence) are determined and then compared, or (2) the RNA transcripts of the *SCN5A* gene or gene fragment are hybridized to single stranded whole genomic DNA from an individual to be tested, and the resulting heteroduplex is treated with Ribonuclease A (RNase A) and run on a denaturing gel to detect the location of any mismatches. Two of these methods can be carried out according to the following procedures.

The alleles of the *SCN5A* or *HERG* gene in an individual to be tested are cloned using conventional techniques. For example, a blood sample is obtained from the individual. The genomic DNA isolated from the cells in this sample is partially digested to an average fragment size of approximately 20 kb. Fragments in the range from 18-21 kb are isolated. The resulting fragments are ligated into an appropriate vector. The sequences of the clones are then determined and compared to the normal *SCN5A* gene.

Alternatively, polymerase chain reactions (PCRs) are performed with primer pairs for the 5' region or the exons of the *SCNSA* or *HERG* gene. PCRs can also be performed with primer pairs based on any sequence of the normal *SCNSA* or *HERG* gene. For example, primer pairs for one of the introns can be prepared and utilized. Finally, PCR can also be performed on the mRNA. The amplified products are then analyzed by single stranded conformation polymorphisms (SSCP) using conventional techniques to identify any differences and these are then sequenced and compared to the normal gene sequence.

Individuals can be quickly screened for common *SCN5A* or *HERG* gene variants by amplifying the individual's DNA using suitable primer pairs and analyzing the amplified product, e.g., by dot-blot hybridization using allele-specific oligonucleotide probes.

The second method employs RNase A to assist in the detection of differences between the normal *SCN5A* or *HERG* gene and defective genes. This comparison is performed in steps using small (-500 bp) restriction fragments of the *SCN5A* or *HERG* gene as the probe. First, the *SCN5A* or *HERG* gene is digested with a restriction enzyme(s) that cuts the gene sequence into fragments of approximately 500 bp. These fragments are separated on an electrophoresis gel, purified from the gel and cloned individually, in both orientations, into an SP6 vector (e.g., pSP64 or pSP65). The SP6-based plasmids containing inserts of the *SCN5A* or *HERG* gene fragments are transcribed *in vitro* using the SP6 transcription system, well known in the art, in the presence of [$\alpha$-$^{32}$P]GTP, generating radiolabeled RNA transcripts of both strands of the gene.

Individually, these RNA transcripts are used to form heteroduplexes with the allelic DNA using conventional techniques. Mismatches that occur in the RNA:DNA heteroduplex, owing to sequence differences between the *SCN5A* or *HERG* fragment and the *SCN5A* or *HERG* allele subclone from the individual, result in cleavage in the RNA strand when treated with RNase A. Such mismatches can be the result of point mutations or small deletions in the individual's allele. Cleavage of the RNA strand yields two or more small RNA fragments, which run faster on the denaturing gel than the RNA probe itself.

Any differences which are found, will identify an individual as having a molecular variant of the *SCN5A* or *HERG* gene and the consequent presence of long QT syndrome. These variants can take a number of forms. The most severe forms would be frame shift mutations or large deletions which would cause the gene to code for an abnormal protein or one which would significantly alter protein expression. Less severe disruptive mutations would include small in-frame deletions and nonconservative base pair substitutions which would have a significant effect on the protein produced, such as changes to or from a cysteine residue, from a basic to an acidic amino acid or vice versa, from a hydrophobic to hydrophilic amino acid or vice versa, or other mutations which would affect secondary or tertiary protein structure. Silent mutations or those resulting in conservative amino acid substitutions would not generally be expected to disrupt protein function.

Genetic testing will enable practitioners to identify individuals at risk for hereditary LQT at, or even before, birth. Presymptomatic diagnosis of LQT will enable prevention of these disorders. Existing medical therapies, including beta adrenergic blocking agents, may prevent and delay the onset of problems associated with the disease. Finally, this invention changes our understanding of the cause and treatment of common heart disease like cardiac arrhythmias which account for 11 % of all natural deaths. Existing diagnosis has focused on measuring the QT interval from electrocardiograms. This method is not a fully accurate indicator of the presence of long QT syndrome. The present invention is a more accurate indicator of the presence of the disease.

The Association between HERG and Acquired LQT

HERG Encodes a $K^{\pm}$ Channel with Inward Rectification Properties Similar to $I_{Kr}$. To determine the physiologic properties of HERG, a full-length cDNA was cloned and characterized. This was prepared for expression in *Xenopus* oocytes. The characteristics of the expressed channel were studied in oocytes 2-6 days after cRNA injection using standard two-microelectrode voltage clamp techniques. HERG current was activated in response to test potentials > -50 mV. The magnitude of HERG current increased progressively with test potentials up to -10 mV (Figure 3A), then progressively decreased with test potentials ≥ 0 mV (Figure 3B). Deactivation of current (tail current) was assessed after return of the membrane to the holding potential of -70 mV. The amplitude of the tail currents progressively increased after depolarization and saturated at +10 mV. The HERG current-voltage (I-V) relationship determined for 10 oocytes is shown in Figure 3C. Peak outward current decreased with incremental depolarization, indicating that HERG is an inward rectifier. The voltage-dependence of channel activation was assessed by plotting the relative amplitude of tail currents as a function of test potential (Figure 3D). HERG reached half-maximal activation at a potential of -15.1 mV. These data define HERG as a delayed rectifier $K^+$ channel with a voltage-dependence of activation and rectification properties nearly identical to $I_{Kr}$ (Sanguinetti and Jurkiewicz, 1990; Shibasaki, 1987; Yang et al., 1994). These properties are unlike any other cardiac current.

To further characterize HERG, the time-course of current activation and deactivation was determined. The time-course for the onset of current (activation) was best fit with a single exponential function (Figure 4A). The rate of activation increased with incremental changes in test potentials from -40 to +50 mV. Deactivating currents were best fit with a biexponential function (Figure 4B), similar to $I_{Kr}$ (Chinn, 1993; Yang et al., 1994). The time constants for HERG current activation, and the fast phase of deactivation, were a bell-shaped function of test potential (Figure 4C). The relative amplitude of the fast component of deactivation varied from 0.77 at -30 mV to 0.2 at -120 mV (Figure 4D). The kinetics of HERG current are slower than $I_{Kr}$ (Sanguinetti and Jurkiewicz, 1990; Shibasaki, 1987; Yang et al., 1994), but exhibit an identical voltage-dependence.

HERG Current is Activated by Extracellular $K^{\pm}$. The $K^+$-selectivity of HERG was determined by measuring the reversal potential of currents in oocytes bathed in ND96 solution containing different concentrations of KCl (0.5 - 20 mM). Tail currents were measured at a variable test potential after current activation by a pulse to +20 mV (Figures 5A and 5B). The voltage at which the tail current reversed from an inward to an outward current was defined as the reversal potential, $E_{rev}$. This varied with extracellular $K^+$ concentration ($[K^+]_e$), as predicted by the Nernst equation (58 mV change for a 10-fold increase in $[K^+]_e$) for $[K^+]_e \geq 5$ mM. $E_{rev}$ varied over the entire range of $[K^+]_e$ in a manner well-described by the Goldman-Hodgkin-Katz current equation (Figure 5C). These data indicate that HERG is selectively permeable to $K^+$ over $Na^+$ by a factor of 143.

A hallmark feature of cardiac $I_{Kr}$ is its modulation by $[K^+]_e$ (Sanguinetti and Jurkiewicz, 1992). The effect of $[K^+]_e$ on the magnitude of HERG current is shown in Figures 6A-C. HERG current increased in direct proportion to $[K^+]_e$, although the shape of the I-V relationship was not altered (Figure 6D). The $[K^+]_e$-dependence of HERG current was determined by comparing the peak outward current at +20 mV in oocytes bathed in solutions containing 0.5 to 20 mM KCl. Over this range, HERG current amplitude varied as a linear function of $[K^+]_e$ (Figure 6E). Unlike most other $K^+$ currents, the magnitude of outward HERG current is paradoxically reduced upon removal of extracellular $K^+$.

Rectification of HERG Current Results from Rapid Channel Inactivation. Inward rectification of $IK_r$ is hypothesized to result from voltage-dependent inactivation that is more rapid than activation (Sanguinetti and Jurkiewicz, 1990; Shibasaki, 1987). The net result of these two competing processes is a reduced current magnitude relative to that predicted from the steady-state activation variable and the electrochemical driving force for outward $K^+$ flux. It is hypothesized that peak tail currents do not exhibit similar rectification after strong depolarizations (see Figure 3) because the channels recover from fast inactivation much more rapidly than the time-course of deactivation. If this interpretation is correct, it should be possible to measure the time-course of recovery from fast inactivation during the onset of tail current. Figure 7 shows the results of this experiment. Tail currents were recorded at several test potentials, each preceded by a prepulse to +40 mV (Figure 7A). The voltage-dependence of the time constant for recovery from fast inactivation is plotted in Figure 7B. Recovery was slowest at -30 mV (t = 18.6 msec) and became faster with incremental increases or decreases in test potential. The bell-shaped relationship between the time constant for recovery from inactivation and membrane potential peaked at the same voltage (-30 mV) as the relationship describing the voltage-dependence of HERG current activation and deactivation (Figure 4C). Although the onset of fast inactivation could not be quantified (because it occurred much faster than activation), it is likely that the descending limb of the curve in Figure 6B (from -20 to +20 mV) also describes the voltage-dependence of rapid inactivation. These data indicate that inward rectification of HERG current results from an inactivation process that is much more rapid than the time course of activation.

The voltage-dependence of channel rectification was determined by comparison of the fully-activated I-V relationship for HERG current (Figure 7C) with the I-V relationship expected for an ohmic conductor. The dotted line in Figure 7C was extrapolated from a linear fit of current amplitudes measured at -90 to -120 mV, and described the I-V relationship that would occur in the absence of inward rectification (ohmic conduction). The slope of this line defined the maximal conductance of HERG in this oocyte (118 μS), and was used to calculate the voltage-dependence of channel rectification (Figure 7D). Rectification was half-maximal at -49 mV, and the relationship had a slope factor of 28 mV. The half-point was very similar to $I_{Kr}$ in rabbit nodal cells and the slope factor was nearly identical to $I_{Kr}$ in guinea pig myocytes (Table 1).

Table 1

Comparison of the properties of HERG and $I_{Kr}$

| Current | Inward Rectification | Current-voltage relation: *peak* (mV) | Current-voltage relation: *full rectification* (mV) | Activation: *threshold* (mV) | Activation: *half-point* (mV) | Activation: *slope factor* (mV) |
|---|---|---|---|---|---|---|
| HERG[1] | + | 0 | +60 | -50 | -15 | 7.9 |
| $I_{Kr}$ (guinea pig heart)[2] | + | 0 | +60 | -50 | -22 | 7.5 |
| $I_{Kr}$ (rabbit heart)[3] | + | n.d. | n.d. | -50 | -25 | 7.4 |
| $I_{Kr}$ (mouse AT-1 cells)[4] | + | 10 | +55 | -50 | +1 | n.d. |

| Current | Inactivation: *half-point* (mV) | Inactivation: *slope factor* (mV) | Modulation of current by $[K^+]_e$ | Blockers: $La^{3+}, Co^{2+}$ | | Blockers: *methanesulfonanilides* E-4031 | *methanesulfonanilides* MK-499 | *methanesulfonanilides* dofetilide |
|---|---|---|---|---|---|---|---|---|
| HERG | -49 | +28 | + | + | | (1 μM - no effect) | | n.d. |
| $I_{Kr}$ (guinea pig) | -9 | +22 | + | + | $IC_{50}$ = | 397 nM | 44 nM | 32 nM |
| $I_{Kr}$ (rabbit) | -68 | n.d. | n.d. | n.d. | $IC_{50}$ = | < 1 μM | n.d. | 4 nM |
| $I_{Kr}$ (AT-1 cells) | n.d. | n.d. | n.d. | + | $IC_{50}$ = | n.d. | n.d. | 12 nM |

n.d. = not determined

References:

[1] This study; [2] (Jurkiewicz and Sanguinetti, 1993; Lynch et al., 1994; Sanguinetti and Jurkiewicz, 1990; Sanguinetti and Jurkiewicz, 1991); [3] (Carmeliet, 1992; Shibasaki, 1987); [4] (Yang et al., 1994)

Steady-state HERG current at any given test potential ($V_t$) can be defined:

$$I_{HERG} = G \cdot n \cdot R \cdot (V_t - E_{rev})$$

where: G = maximal conductance of HERG current; n = activation variable; R = rectification variable; $E_{rev}$ = reversal potential.

HERG Current Is Blocked by Lanthanum and Cobalt, but Not Affected by Methanesulfonanilides or Cyclic Nucleotides. $I_{Kr}$ of cardiac myocytes is blocked by 10 - 100 μM lanthanum ($La^{3+}$), 2 mM cobalt ($Co^{2+}$) (Balser et al., 1990; Sanguinetti and Jurkiewicz, 1990), and nM concentrations of several methanesulfonanilide antiarrhythmic drugs, such as E-4031 (Sanguinetti and Jurkiewicz, 1990) and MK-499 (Lynch et al., 1994). It was determined whether HERG current is also blocked by these cations and drugs. At a test potential of 0 mV, 10 μM $La^{3+}$ reduced HERG current by 92±3% (n = 4, Figure 8). At least part of the blocking effect of $La^{3+}$ resulted from screening of negative membrane surface charge (Sanguinetti and Jurkiewicz, 1990), as indicated by the 40 mV positive shift in both the peak of the I-V relationship (Figure 8C) and the isochronal activation curve (Figure 8D). HERG was also partially blocked (52%) by 2 mM $Co^{2+}$ (n = 2). However, neither E-4031 nor MK-499 at a concentration of 1 μM blocked HERG current, even after incubating the oocytes for up to 4 hours in these drugs.

The HERG channel contains a segment homologous to a cyclic nucleotide binding domain near its carboxyl terminus (Warmke and Ganetzky, 1994). To determine if HERG was sensitive to cyclic nucleotides, the effects of 8-Br-cAMP and 8-Br-cGMP on expressed HERG current were tested. These membrane permeant analogs of endogenous cyclic nucleotides have been shown to increase the magnitude of other channels expressed in *Xenopus* oocytes (Blumenthal and Kaczmarek, 1992; Bruggemann et al., 1993). Neither compound had a significant effect on current magnitude or voltage-dependence of channel activation at a concentration of 1 mM within 30 min of application (data not shown).

HERG Encodes Subunits of Cardiac $I_{Kr}$ Channels. The above results show that *HERG* encodes the major subunit of the cardiac $I_{Kr}$ channel. HERG expressed in oocytes induces a current that shares most of the distinguishing characteristics defining $I_{Kr}$ in cardiac myocytes (Table 1). These include: 1) inward rectification of the I-V relationship, with a peak near 0 mV; 2) voltage dependence of activation; 3) paradoxical modulation of current by $[K^+]_e$; and 4) block by $La^{3+}$ and $Co^{2+}$. The kinetics of activation and deactivation of HERG current are much slower than $I_{Kr}$ in mouse AT-1 cells measured at room temperature (Yang, et al., 1994). This difference may indicate that some other endogenous factor, or an additional channel subunit modulates the gating of $I_{Kr}$ channels in cardiac cells. In addition, HERG is not activated by 8-Br-cAMP, consistent with the finding that isoproterenol does not increase $I_{Kr}$ in cardiac myocytes (Sanguinetti et al., 1991). Co-assembly of HERG subunits in oocytes, presumedly as homotetramers (MacKinnon, 1991), therefore, can reconstitute the major biophysical properties of cardiac $I_{Kr}$. No other channel shares all these characteristics.

The only major difference between HERG current and $I_{Kr}$ is that HERG is not blocked by methanesulfonanilide drugs (E-4031, MK-499), potent and specific blockers of $I_{Kr}$ in isolated cardiac myocytes (Lynch et al., 1994; Sanguinetti and Jurkiewicz, 1990). This suggests that the $I_{Kr}$ channel and the methanesulfonanilide receptor are separate, but interacting, proteins. A similar phenomenon has been described for the $K_{ATP}$ channel, recently isolated from mammalian heart (Ashford et al., 1994). When this channel (rc$K_{ATP}$-1) is expressed in HEK293 cells, it has all the biophysical characteristics of the native channel (Ashford et al., 1994), including modulation by intracellular nucleotides. However, the channel is not blocked by glibenciamide, a drug that inhibits $K_{ATP}$ channels in cardiac myocytes (Ashford et al., 1994). It may be possible to isolate the methanesulfonanilide receptor biochemically using known high affinity probes such as dofetilide or MK-499. Co-expression of HERG channels with the methanesulfonanilide receptor will enable detailed studies of the interaction between these two molecules.

The Mechanism of HERG Rectification Is Rapid Channel Inactivation. A unique feature of $I_{Kr}$ is inward rectification of the I-V relationship. The cardiac inward rectifier, $I_{KI}$, also exhibits intense inward rectification, but this occurs over a much more negative voltage range. Under normal physiologic conditions, peak outward $I_{KI}$ occurs at -60 mV, whereas $I_{Kr}$ peaks at 0 mV. The mechanism of $I_{KI}$ rectification results from both a voltage-dependent gating mechanism and block of outward current by intracellular $Mg^{2+}$ (Vandenberg, 1987) and spermine (Fakler et al., 1995). In contrast, it was postulated that inward rectification of $I_{Kr}$ results from voltage-dependent inactivation that occurs much faster than activation (Shibasaki, 1987). The kinetics of fast inactivation are difficult to resolve in macroscopic current recordings of myocytes and, therefore, were calculated based on kinetics of single channel activity (Shibasaki, 1987). In this study, it was possible to resolve the time-course for recovery from inactivation of macroscopic HERG current because of the large signal-to-noise ratio and the relatively slow channel gating kinetics at room temperature. The rapid onset of, and recovery from, fast inactivation explains the marked inward rectification of the I-V relationship for HERG. For example, at a test potential of +20 mV, HERG activates with a time constant of 230 msec, but simultaneously inactivates with a time constant of 12 msec. Thus, inactivation is complete before activation of current has reached a significant level, resulting in a much reduced current amplitude. Recovery from inactivation occurs so fast, relative to deactivation, that tail current amplitudes are not significantly affected after repolarization. Our findings support Shibasaki's hypothesis that the mechanism of rectification for $I_{Kr}$ (and HERG) is rapid, voltage-dependent inactivation.

Rectification of HERG current was half-maximal ($V_{1/2}$) at -49 mV, and had a slope factor of 28 mV. The slope factor of HERG rectification was similar to $I_{Kr}$ measured in guinea pig myocytes (22 mV). The $V_{1/2}$ of HERG rectification was more negative than that estimated in guinea pig (Table 1). However, the voltage-dependence of $I_{Kr}$ rectification in guinea pig myocytes was difficult to measure because of overlap with a much larger $I_{KI}$ at negative test potentials. The absence of overlapping current in rabbit nodal cells, and in oocytes expressing HERG, allowed more accurate measure of the voltage-dependence of channel rectification, and these determinations were similar (Table 1). Single channel analyses of expressed HERG will enable a more detailed description of voltage-dependent gating and fast inactivation.

The $[K^+]_e$-Dependence of HERG Current May Modulate Duration of Cardiac Action Potentials. Elevation of $[K^+]_e$ caused an increase in outward HERG current. This is a paradoxical effect, since an increase of $[K^+]_e$ lowers the chemical driving force for outward $K^+$ flux and therefore, would be expected to decrease, rather than increase, outward current. The same phenomenon has been described for $I_{Kr}$ (Sanguinetti and Jurkiewicz, 1992; Scamps and Carmeliet, 1989), but not for any other cardiac channel, except $I_{K1}$. However, $I_{K1}$ is activated almost instantly with hyperpolarization, whereas HERG, like $I_{Kr}$, is relatively slowly activated by depolarization, and not activated by hyperpolarization.

The modulation of HERG (and $I_{Kr}$) by $[K^+]_e$ may have physiologic importance. During rapid heart rates, or ischemia, $K^+$ accumulates within intracellular clefts (Gintant et al., 1992). This elevation in $[K^+]_e$ would increase the contribution of HERG ($I_{Kr}$) to net repolarizing current. HERG ($I_{Kr}$) may be even more important, therefore, in modulation of action potential duration at high heart rates, or during the initial phase of ischemia.

The mechanism of HERG modulation by $[K^+]_e$ is not yet known, but may be similar to that described for another cloned $K^+$ channel, RCK4. The amplitude of RCK4 is also increased with elevation of $[K^+]_e$ (Pardo et al., 1992). Single channel analyses revealed that elevation of $[K^+]_e$ increased the number of channels available to open, but had no effect on single-channel conductance, mean open time, or gating charge (Pardo et al., 1992). Moreover, it was demonstrated that substitution of a single lysine, located near the pore of the channel, to a tyrosine residue (K533Y) eliminated this effect. A similar $[K^+]_e$-dependent increase in current was created by substitution of a single amino acid near the pore domain of Shaker B channels (Lopez-Barneo et al., 1993). Future experiments will determine if $K^+$ modulates single HERG channels by a similar mechanism.

Mutation of HERG and Drug-induced Block of $IK_r$:A Mechanistic Link Between Inherited and Acquired LQT. Inherited LQT, and the more common (drug-induced) acquired form of the disorder, are associated with *torsade de pointes,* a polymorphic ventricular tachyarrhythmia. It was recently shown that mutations in *HERG* cause chromosome 7-linked LQT, likely by a dominant-negative inhibition of HERG function (Curran et al., 1995). It should be noted that there are likely to be several different mechanisms that account for acquired and inherited LQT. For example, it was recently demonstrated that mutations in *SCN5A,* the cardiac sodium channel gene, cause chromosome 3-linked LQT (Wang et al., 1995). The discovery that HERG forms the $I_{Kr}$ channel provides a logical explanation for the observation that block of $I_{Kr}$ by certain drugs can provoke the same arrhythmia (*torsades de pointes)* as observed in familial LQT.

The present findings may have important clinical implications. It was found that changes in $[K^+]_e$ over a physiologic range significantly modulated the amplitude of HERG current. For example, elevation of $[K^+]_e$ from a level of 2 mM to a new level of 5 mM increased HERG current by 40%. Modest hypokalemia, a common clinical problem, would have a significant effect on HERG current. This may explain the association between hypokalemia and acquired LQT (Roden, 1988). Furthermore, hypokalemia *per se* has been associated with ventricular arrhythmias (Curry et al., 1976). Medications *(e.g.,* sotalol, dofetilide) that decrease $I_{Kr}$ can be effective antiarrhythmic agents because they modestly lengthen cardiac action potentials, thereby inhibiting re-entrant arrhythmias. In the setting of hypokalemia, however, this effect would be exaggerated, leading to excessive action potential prolongation and induction of *torsade de pointes.* Modest elevation of serum $[K^+]$ in patients given these antiarrhythmic medications, or in patients given other drugs which can cause acquired LQT (e.g., antihistamines or antibiotics such as erythromycin) or in individuals with chromosome 7-linked LQT, should help prevent LQT and *torsade de pointes.*

In summary, it has been demonstrated that *HERG* encodes the major subunit forming $I_{Kr}$ channels. This discovery suggests that the molecular mechanism of chromosome 7-linked LQT, and certain acquired forms of the disorder, can result from dysfunction of the same ion channel.

The present invention is further detailed in the following Examples, which are offered by way of illustration and are not intended to limit the invention in any manner. Standard techniques well known in the art or the techniques specifically described below are utilized.

EXAMPLE 1

Methods for Phenotypic Evaluation

For the *SCN5A* studies, three previously-described LQT kindreds (K2171, K2321 and K2322) were studied (Jiang et al., 1994). For the *HERG* studies, LQT kindreds were ascertained from medical clinics throughout North America.

Phenotypic criteria were identical to those used in previous studies (Keating et al., 1991a; Keating et al., 1991b; Keating, 1992). Individuals were evaluated for LQT based on the QT interval corrected for heart rate (QTc; Bazette, 1920), and the presence of syncope, seizures, and aborted sudden death. Informed consent was obtained from each individual, or their guardians, in accordance with local institutional review board guidelines. Phenotypic data were interpreted without knowledge of genotype. Symptomatic individuals with a corrected QT interval (QTc) of 0.45 seconds or greater and asymptomatic individuals with a QTc of 0.47 seconds or greater were classified as affected. Asymptomatic individuals with a QTc of 0.41 seconds or less were classified as unaffected. Asymptomatic individuals with QTc between 0.41 and 0.47 seconds and symptomatic individuals with QTc of 0.44 seconds or less were classified as uncertain.

EXAMPLE 2

Linkage Analysis

Pairwise linkage analysis was performed using MLINK in LINKAGE v5.1 (Lathrop et al., 1985). Assumed values of 0.90 for penetrance and 0.00 for LQT gene frequency were used. Gene frequency was assumed to be equal between males and females.

EXAMPLE 3

Isolation of *SCN5A* Genomic Clones and Partial Characterization of Genomic Structure .

*SCN5A* probes were generated using the products of PCR reactions with human genomic DNA and primer pairs based on *SCN5A* cDNA sequences. One primer pair, 5'ACTTTCATCGTACTGAATAAAGGCAA3' (SEQ ID NO:1) and 5'GAGTGAACCAGAATCTTCACAGC3' (SEQ ID NO:2), was designed from 5' coding sequences and yielded the predicted product of 118 bp.

The second primer pair, 5'GGACCGTGAGTCCATCGTGTGA3' (SEQ ID NO:3) and 5'AGCCCATTCACAACATATACAGTCT3' (SEQ ID NO:4), was derived from the 3' non-coding sequences and yielded a product of 336 bp.

The third primer pair, 5'AGCAACTTCATCCCAGCTGCTGAG3' (SEQ ID NO:5) and 5'CTCCCAGCATCTCAGGTCAAGTG3' (SEQ ID NO:6), was based on 3' non-coding sequences and yielded a product of 297 bp. These PCR products were purified from 2% agarose gels, radiolabeled to high specific activity and used to screen a human genomic P1 library (Sternberg, 1990).

To characterize the genomic structure of *SCN5A* exons encoding the cytoplasmic linker between DIII-DIV, sequencing primers based on cDNA sequences and predicted genomic structure (McClatchey et al., 1992a; Gellens et al., 1992) were designed. The primer pair for presumed exon 21 (based on the structure of *SCN4A)* was 5'TATGAAGAGCAGCCTCAGTGGGAA3' (SEQ ID NO:7) and 5'CTTTTTCTTCTGTTGGTTGAAGTTG3' (SEQ ID NO:8). Primers for presumed exon 22 were 5'TTAGGGGGCCAGGACATCTTC3' (SEQ ID NO:9) and 5'CAGGGGCCGTGGGATGGGCTTCTGG3' (SEQ ID NO:10). Primers for presumed exon 23 were 5'CACCATATTCAAGCAGATCAG3' (SEQ ID NO:11) and 5'CTGCGCCACTACTACTTCACC3' (SEQ ID NO:12). These primers were used to determine intronic sequences from *SCN5A* clones as described (Wang and Keating, 1994).

EXAMPLE 4

Isolation of *HERG* Genomic and cDNA Clones

*HERG* probes were generated using the products of PCR reactions with human genomic DNA and primer pairs 1-10, 6-13 and 15-17 (see Table 2). These products were cloned, radiolabeled to high specific activity and used to screen a human genomic P1 library (Sternberg, 1990). Positive clones were purified, characterized and used for FISH and DNA sequence analyses. An *HERG* genomic clone containing domains S1-S3 and intron I (Curran et al., 1995) was used to screen ~$10^6$ recombinants of a human hippocampal cDNA library (Stratagene, library #936205). A single, partially processed cDNA clone that contained nucleotides 32-2398 of *HERG* coding sequence was identified. A second screen of this library was performed using the coding portion of this cDNA. This screen produced a second clone containing *HERG* coding sequence from nucleotides 1216 through the 3' untranslated region (UTR), and included a poly-$A^+$ region. These two cDNAs were ligated using an *XhoI* site at position 2089. To recover the 5' region of *HERG,* ~$10^6$ clones of a human heart cDNA library (Stratagene, library #936207) were screened with the composite hippocampal cDNA. A single clone containing the 5'-UTR through nucleotide 2133 was isolated. This clone was combined with the hippocampal composite at a *BglII* site (nucleotide 1913) to produce a full-length *HERG* cDNA.

EXAMPLE 5

YAC-based Mapping of *HER*G

A PCR assay specific for the 3' untranslated region of *HERG* (employing primers 5'GCTGGGCCGCTCCCCTTGGA3' (SEQ ID NO:13) and 5'GCATCTTCATTAATTATTCA3' (SEQ ID NO:14) and yielding a 309-bp product) was used to screen a collection of YAC clones highly enriched for human chromosome 7 (Green et al., in press). Two positive YAC clones were identified (yWSS2193 and yWSS17S9), both were contained within a larger contig that includes YACs positive for the genetic marker D7S505 (Green et al., 1994).

EXAMPLE 6

Fluorescent *In Situ* Hybridization

Metaphase chromosome spreads were prepared from normal cultured lymphocytes (46 X,Y) by standard procedures of colcemid arrest, hypotonic treatment and acetic acid-methanol fixation. *HERG* P1 clone 16B4 was labeled by incorporation of biotin-14-dATP (BioNick System, Gibco-BRL), hybridized to metaphase spreads and detected with streptavidin-Cy3 according to standard methods (Lichter et al., 1988). To identify chromosome 7, a digoxigenin-labeled centromere-specific α-satellite probe (Oncor) was co-hybridized and detected with antidigoxigenin-FITC. Chromosomes were counterstained with DAPI and visualized directly on the photomicroscope.

EXAMPLE 7

SSCP Analysis

Genomic DNA samples were amplified by PCR and used in SSCP analyses as described (Orita et al., 1989; Ptacek et al., 1991). Primer pairs used for this study are shown in Tables 2 and 3. Annealing temperature was 62°C (for the *SCN5A* studies) or 58°C (for the *HERG* studies) for all PCR reactions. For *SCN5A,* reactions (10 μl) were diluted with 50 μl of 0.1% SDS/1mM EDTA and 50 μl of 95% formamide dye. For *HERG,* reactions (10 μl) were diluted with 40 μl of 0.1% SDS/lmM EDTA and 30μl of 95% formamide dye. Diluted products were denatured by heating at 94°C or 100°C for 5 or 10 minutes, and 3-5 μl of each sample were separated by electrophoresis on either 7.5% or 10% non-denaturing polyacrylamide gels (50 acrylamide:1 Bis-acrylamide) at 4°C. Electrophoresis was carried out at 40-50 watts for 2 to 5 hours. Gels were transferred to 3MM filter paper, dried and exposed to X-ray film at -80°C for 12-36 hours.

TABLE 2

*HERG* PCR Primers

| Name | Position | Sequence | SEQ ID NO: |
|---|---|---|---|
| 1 L | 1147-1166 | GACGTGCTGCCTGAGTACAA | 21 |
| 2 L | 1291-1312 | TTCCTGCTGAAGGAGACGGAAG | 22 |
| 3 L | 1417-1437 | ACCACCTACGTCAATGCCAAC | 23 |
| 4 L | INTRON I | TGCCCCATCAACGGAATGTGC | 24 |
| 5 L | 1618-1636 | GATCGCTACTCAGAGTACG | 25 |
| 6 L | 1802-1823 | GCCTGGGCGGCCCCTCCATCAA | 26 |
| 7 R | 1446-1426 | CACCTCCTCGTTGGCATTGAC | 27 |
| 8 R | 1527-1503 | GTCGAAGGGGATGGCGGCCACCATG | 28 |
| 9 R | INTRON I | TACACCACCTGCCTCCTTGCTGA | 29 |
| 10 R | 1643-1623 | GCCGCGCCGTACTCTGAGTAG | 30 |
| 11 R | 1758-1736 | CAGCCAGCCGATGCGTGAGTCCA | 31 |
| 12 R | INTRON II | GCCCGCCCCTGGGCACACTCA | 32 |
| 13 R | 2034-2016 | CAGCATCTGTGTGTGGTAG | 33 |
| 14 R | INTRON III | GGCATTTCCAGTCCAGTGC | 34 |
| 15 L | 2259-2278 | CCTGGCCATGAAGTTCAAGA | 35 |
| 16 L | 2214-2233 | GCACTGCAAACCCTTCCGAG | 36 |
| 17 R | 2550-2529 | GTCGGAGAACTCAGGGTACATG | 37 |

All primers are shown in 5' to 3' direction. Sense-strand oligonucleotides are indicated with an "L" and anti-sense oligonucleotides are indicated with an "R". cDNA sequence was obtained from the Genbank database, nucleotide numbering begins with the initiator methionine.

TABLE 3

PCR Primers Used to Define *SCN5A* Polymorphisms and Mutations

| Primer | Sequence | SEQ ID NO: | Region Amplified | Exon |
|---|---|---|---|---|
| 1 L | GCCTGTCTGATCTCCCTGTGTGA | 38 | DIII/S6; IDIII-IV | 21 |
| 2R | ACCCAGCCCAGTGGGGAGCTGGT | 39 | | |
| 3 L | CCATGCTGGGGCCTCTAAAACC | 40 | IDIII-IV | 22 |
| 4 R | GGCTCTGATGGCTGGCCATGTG | 41 | | |
| 5 L | CCCAGCGAGCACTTTCCATTTG | 42 | IDIII-IV; DIV/S1-S3 | 23 |
| 6 R | GCTTCTCCGTCCAGCTGACTTGTA | 43 | | |
| 7 L | GAGCCCAGCCGTGGGCATCCT | 44 | DIV/S6 | 24 |
| 8 R | GTCCCCACTCACCATGGGCAG | 45 | | |

All primers are shown in 5' to 3' direction. Sense-strand oligonucleotides are indicated with an "L" and anti-sense oligonucleotides are indicated with an "R".

D = domain; ID = interdomain; S = membrane spanning segment.

EXAMPLES 8

Sequence Analysis of SSCP Conformers

Normal and aberrant SSCP conformers were cut directly from dried gels and eluted in 75-100 μl of distilled water at either 37°C or 65°C for 30 minutes. Ten μl of the eluted DNA was used as template for a second PCR reaction using the original primer pair. Products were fractionated in 2% low-melting temperature agarose gels (FMC), and DNA fragments were purified and sequenced directly by cycle sequencing (Wang and Keating, 1994). Alternatively, purified PCR products were cloned into pBluescript II SK$^+$ (Stratagene) using the T-vector method as described (Marchuk et al., 1990). Plasmid DNA samples were purified and sequenced by the dideoxy chain termination method using SequiTherm Polymerase (Epicentre Technologies) or as previously described (Curran et al., 1993a).

EXAMPLE 9

Northern Analysis

A multiple tissue Northern blot containing ~2 μg/lane of poly-A$^+$ mRNA was purchased from Clonetech (Human MTN blot 1). A high specific activity (>1.5 x $10^9$ cpm/μg DNA), radiolabeled *HERG* cDNA fragment containing nucleotides 679-2239 of the coding sequence was prepared by random hexamer priming as described (Feinberg and Vogelstein, 1983). Probe was added to the hybridization solution at final concentration of 5 x $10^6$ cpm/ml. Hybridization was carried out at 42°C for 24 hours in 20 ml of Quickhyb solution (Stratagene). Final washes were carried out at 65°C for 30 minutes in a solution of 0.1% SDS/0.1X SSC.

EXAMPLE 10

Linkage Analysis of *SCN5A*

To determine the chromosomal location of an LQT gene, linkage analysis was performed. Previously 3 LQT families had been linked to chromosome 3p (Jiang et al., 1994). In that study two families, K2171 and K2321 showed complete linkage to markers at D3S1100 and D3S1298. The combined pairwise lod scores of the three linked families were 6.72 and 6.39 respectively, at θ=0.001. Haplotype analysis performed in families K2171, K2321 and K2322 had further refined the position of this locus. Obligate recombinants were identified at D3S1211 and D3S1767, defining the telomeric and centromeric flanking markers respectively. The interval between these flanking loci is estimated to be 17.6 centiMorgans (Jiang et al., 1994).

In 1995, *SCN5A* was mapped to chromosome 3p21 (George et al., 1995). To test the candidacy of *SCN5A* for LQT, single-stranded conformation polymorphism (SSCP) analyses were used to identify polymorphisms within this gene, and then linkage analyses in chromosome 3-linked families were performed. Since the genomic structure of *SCN5A* was unknown, oligonucleotide primer pairs were designed from published *SCN5A* cDNA sequences (Gellens et al., 1992) based on the assumption that the genomic structure of *SCN5A* would be similar to the known structure of *SCN4A,* the skeletal muscle sodium channel gene (McClatchey et al., 1992a). To facilitate this work, two genomic P1 clones were identified and partially characterized. These clones spanned the entire *SCN5A* gene. Primers based on sequences predicted to encode the cytoplasmic region between DIII and DIV were synthesized. This region corresponds to exons 21-23 of SCN4A and is known to be critical for channel inactivation. These primers were used to characterize the flanking introns by cycle sequencing. Additional primers were designed to these intronic sequences for SSCP analyses (Table 3). Primer pairs which gave appropriately sized products from genomic DNA were used to screen DNA samples from patients. An aberrant SSCP conformer was identified using primer pair 7-8 (Table 3). The normal and aberrant bands were cloned and sequenced. The aberrant conformer resulted from a C to T substitution at position 3 of codon 1819 (cDNA nucleotide 5607; Gellens et al., 1992). This substitution did not affect the predicted amino acid sequence of *SCN5A.* The observed heterozygosity for this polymorphism was <0.50. The *SCN5A* polymorphism was used for genotypic analyses in chromosome 3-linked families (Figure 1). No recombination events between *SCN5A* and LQT were identified in any of these families. The maximum combined two-point lod score for all chromosome 3-linked families was 2.74 at a recombination fraction of 0.0 (Table 4).

TABLE 4 Pairwise Lod Scores an Recombination Fractions for Linkage of *LQT3* with SCN5A

| Marker | Kindred | Recombination Fraction | | | | | | |
|---|---|---|---|---|---|---|---|---|
| | | 0.001 | 0.01 | 0.05 | 0.10 | 0.20 | 0.30 | 0.40 |
| SCN5A/3-4 | K2171 | 0.00 | 0.00 | 0.00 | 0.00 | 0.00 | 0.00 | 0.00 |
| | K2321 | 1.50 | 1.47 | 1.35 | 1.20 | 0.87 | 0.53 | 0.18 |
| | K2322 | 4.03 | 3.95 | 3.62 | 3.20 | 2.34 | 1.47 | 0.61 |
| | *TOTAL* | 5.53 | 5.43 | 4.97 | 4.40 | 3.22 | 2.00 | 0.79 |
| | $Z_{max}$ = 5.54. θ = 0.0 | | | | | | | |
| *SCN5A*/7-8 | K2171 | 0.82 | 0.80 | 0.74 | 0.65 | 0.46 | 0.26 | 0.08 |
| | K2321 | 0.12 | 0.12 | 0.10 | 0.08 | 0.04 | 0.02 | 0.00 |
| | K2322 | 1.80 | 1.78 | 1.66 | 1.48 | 1.05 | 0.60 | 0.20 |
| | *TOTAL* | 2.74 | 2.70 | 2.50 | 2.20 | 1.55 | 0.87 | 0.28 |
| | $Z_{max}$ = 2.74, θ = 0.0 | | | | | | | |

Lod scores were calculated assuming autosomal dominant inheritance with a penetrance of 0.90 for all kindreds, as indicated by segregation analysis of these and other LQT kindreds. Assumed were a disease allele frequency of 0.001 and that female and male recombination frequencies were equal. $Z_{max}$ indicates maximum log score. θ indicates estimated recombination fraction at $Z_{max}$.

When primer pair 3-4 was used in SSCP analyses, an anomalous conformer was identified in DNA samples from affected members of kindreds 2321 and 2322 (Figure 1). By contrast, only the normal conformer was seen in DNA samples from unaffected members of these families. The combined two-point lod score for linkage between this anomaly and *LQT3* was 5.54 (Table 4). Again, no recombination was observed between *SCN5A* and *LQT3,* indicating that these loci are tightly linked.

The mobility shift between the aberrant and normal SSCP conformers identified in kindreds 2321 and 2322 was large, suggesting the possibility of a small deletion. To test this hypothesis, the conformers were separated by electrophoresis on denaturing polyacrylamide gels (Figures 9 and 10). These data demonstrated the presence of two products of 235 bp and 244 bp. The 235 bp product was only seen in affected individuals. Furthermore, this aberrant conformer was not observed in more than 500 control individuals. These data indicate the presence of a disease-associated deletion within *SCN5A.*

To determine the effect of the LQT-associated deletion on *SCN5A* structure, the normal and aberrant SSCP conformers were amplified and cycle sequencing was performed. These experiments revealed the presence of a 9 bp deletion beginning at nucleotide 4661 of the cDNA (Figure 11). The aberrant and normal SSCP conformers were also cloned and sequenced. These experiments confirmed the size and location of the deletion. This deletion disrupts the coding sequence, resulting in a deletion of three conserved amino acids, $K_{1505}$-$P_{1506}$-$Q_{1507}$ (KPQ) in the cytoplasmic linker between DIII and DIV (Figures 2 and 12).

DNA sequence analyses of the aberrant conformer in kindred 2321 indicated that the intragenic deletion was identical to that found in kindred 2322. One possible explanation for the identical deletions in these two kindreds is that they are distantly related. Both families were North American of European descent, one German and the other English. Examination of a genealogy database failed to reveal a relationship between these families for more than eight generations. Furthermore, genotypic analyses of these kindreds indicated different haplotypes on the disease chromosome (Fig. 1). The presence of identical deletions in two apparently unrelated LQT families strongly suggests that *SCN5A* is *LQT3.*

EXAMPLE 11

Linkage Analysis of *HERG*

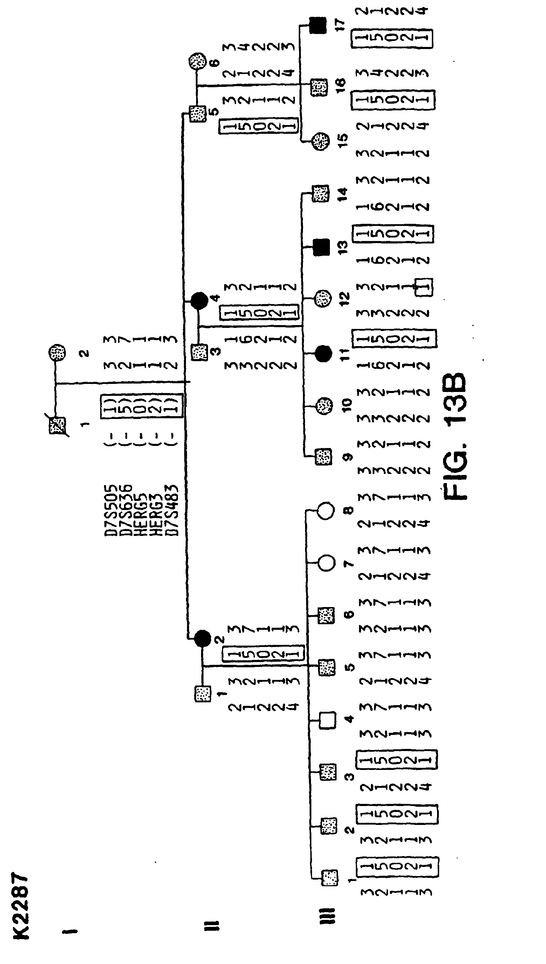

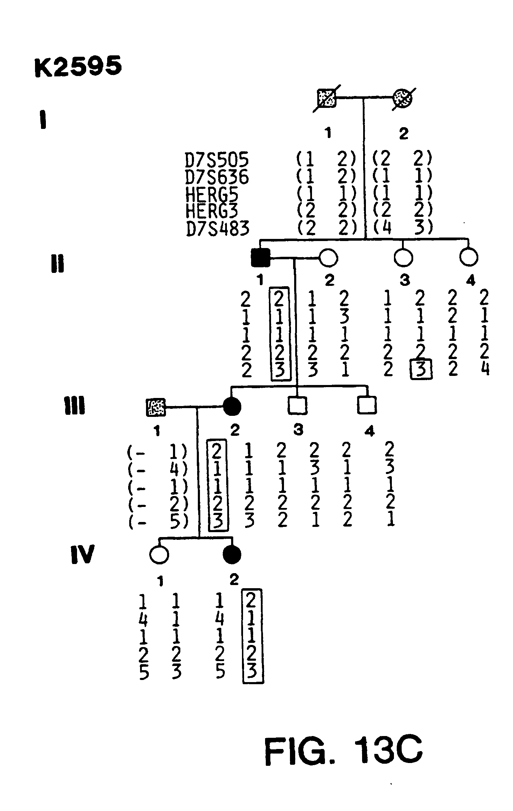

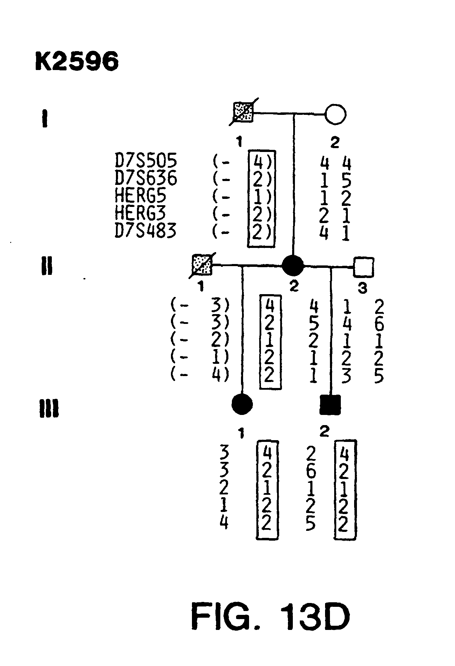

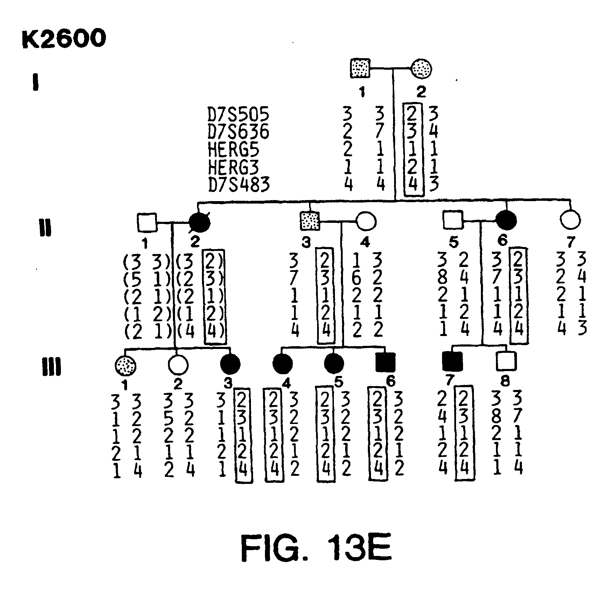

*LQT2* is linked to markers on chromosome 7q35-36. To determine the relative frequency of the three known LQT loci *(LQT1, LQT2, LQT3).* linkage analyses were performed in families with this disorder. Five LQT families were identified and phenotypically characterized (Fig. 13). These families were unrelated and of varying descent, including Mexican (Spanish), German. English, and Danish. In each case, an autosomal dominant pattern of inheritance was suggested by inspection of the pedigree. Affected individuals were identified by the presence of QT prolongation on electrocardiograms and, in some cases, a history of syncope or aborted sudden death. No patients had signs of congenital neural hearing loss, a finding associated with the rare, autosomal recessive form of LQT, or other phenotypic abnormalities. Genotype analyses with polymorphic markers linked to the known LQT loci suggested that the disease phenotype in these families was linked to polymorphic markers on chromosome 7q35-36 (Fig. 14). The maximum combined two-point lod score for these five families was 5.13 at D7S636 ($\theta$=0.0; Table 5). When combined with a previous study (Jiang et al., 1994; Wang et al., 1995), the maximum combined two-point lod score for the fourteen chromosome 7-linked families was 26.14, also at D7S636 ($\theta$=0.0; Table 5). Haplotype analyses were consistent with previous studies, placing *LQT2* between D7S505 and D7S483 (Fig. 14; Wang et al., 1995), localizing this gene to chromosome 7q35-36.

*HERG* maps to chromosome 7q35-36. *HERG* was previously mapped to chromosome 7 (Warmke and Ganetzky, 1994). To test the candidacy of this gene, the localization of *HERG* was-refined using two physical mapping techniques. First, *HERG* was mapped on a set of yeast artificial chromosome (YAC) contigs constructed for chromosome 7 (Green et al., 1994). *HERG* was localized to the same YAC as D7S505, a polymorphic marker that was tightly linked to *LQT2* (Table 5). Second, *HERG* was mapped to chromosome 7q35-36 using fluorescent *in situ* hybridization (FISH) with a P1 genomic clone containing *HERG.*

TABLE 5 Maximum Pairwise Lod Scores and Recombination Fractions for Linkage of *LQT2* with *HERG,* and Polymorphic Markers on Chromosome 7

| | Families From Present Study | | Families Studied To Date | |
|---|---|---|---|---|
| Locus | $Z_{max}$ | $\theta$ | $Z_{max}$ | $\theta$ |
| D7SS05 | 4.40 | 0.0 | 22.91 | 0.009 |
| D7S636 | 5.13 | 0.0 | 26.14 | 0.00 |
| *HERG* 3-8 | 0.11 | 0.0 | 6.34 | 0.00 |

Table continued

| Locus | Families From Present Study $Z_{max}$ | Families From Present Study θ | Families Studied To Date $Z_{max}$ | Families Studied To Date θ |
|---|---|---|---|---|
| *HERG* 5-11 | 3.55 | 0.0 | 9.64 | 0.00 |
| D7S483 | 2.48 | 0.0 | 22.42 | 0.00 |

Markers are shown in chromosomal order (centromere to telomere, Gyapay et al., 1994). The first column (families from present study) indicates combined lod scores for the five families described in this study. The second column (families studied to date) indicates combined log scores from the five families studied here, and nine families from previous study (Jiang et al., 1994). $Z_{max}$ indicates maximum lod score. θ indicates estimated recombination fraction at $Z_{max}$.

To determine if *HERG* was genetically linked to the LQT locus, SSCP analyses were used to identify polymorphisms within *HERG,* and linkage analyses were performed in the chromosome 7-linked families. Two aberrant SSCP conformers were identified in DNA samples from patients and controls using primer pairs 5-11, and 3-8 (Figure 15). These conformers were cloned and sequenced. One abnormal conformer resulted from a C to T substitution at position 3 of codon 489 (cDNA nucleotide 1467, observed heterozygosity = 0.37). The second abnormal conformer resulted from an A to G substitution at position 3 of codon 564 (cDNA nucleotide 1692, observed heterozygosity = 0.44). Neither substitution affected the predicted amino acid sequence of *HERG. HERG* polymorphisms were used for genotypic analyses in chromosome 7-linked families (Figure 13). No recombination events between *HERG* and LQT were identified in any of these families. The maximum combined lod score for the 14 families was 9.64 (θ = 0.0; Table 5). These data indicate that *HERG* is completely linked to *LQT2*.

<u>HERG intragenic deletions associated with LQT in two families</u>. To test the hypothesis that *HERG* is *LQT2*, SSCP analyses were used to screen for mutations in affected individuals. Since the genomic structure of *HERG* was unknown, oligonucleotide primer pairs were designed from published (Warmke and Ganetzky, 1994) *HERG* cDNA sequences (Fig. 15, Table 2). In most cases, single products of expected size were generated. For primer pairs 1-10, 6-13, and 15-17, however, products of greater than expected size were obtained, suggesting the presence of intronic sequences. To examine this possibility, these larger products were cloned and sequenced. DNA sequence analyses identified three introns at positions 1557/1558, 1945/1946, and 2398/2399 of the cDNA sequence (Fig. 15). These boundaries were confirmed by direct DNA sequencing of *HERG* genomic clones containing *HERG* (data not shown). To facilitate SSCP analyses, additional primers were designed to intronic sequences.

As indicated previously, SSCP analyses using primer pair 3-8 identified an A to G polymorphism within *HERG* (cDNA nucleotide 1692). Analysis of kindred 2287 (K2287) using this SSCP polymorphism defined a pattern of genotypes consistent with a null allele (Fig. 13). Possible explanations for these findings included multiple misinheritances, a possibility not supported by previous genotypic analyses, DNA sample errors, base-pair substitutions, or a deletion. To test the hypothesis that the genotypic data were due to a small deletion, PCR analyses of K2287 were repeated using a new primer pair (3-9) flanking the previous set of primers. These experiments identified two products of 170 bp and 143 bp in affected members of K2287 (Figs. 16A and 16B). By contrast, only a single product of 170 bp was observed in unaffected members of this kindred. Furthermore, only the 170 bp band was seen in DNA samples from more than 200 unaffected individuals. The 143 bp and 170 bp products were cloned from affected individual II-2. Direct sequence analyses of the aberrant PCR product revealed the presence of a 27 bp deletion beginning at position 1498 (ΔI500-F508). This deletion disrupts the third membrane spanning domain (S3) of *HERG.*

To further test the hypothesis that *HERG* is *LQT2,* more SSCP analyses were performed in additional kindreds. SSCP using the primer pair 1-9 identified an aberrant conformer in affected individuals of K2595 (Fig. 17A). Analyses of more than 200 unaffected individuals failed to show this anomaly. The normal and aberrant conformers were cloned and sequenced, revealing a single base deletion at position 1261 (Δ1261). This deletion results in a frameshift in sequences encoding the first membrane spanning domain (S1), leading to a new stop codon within 12 amino acids (Fig. 17B). The identification of intragenic deletions of *HERG* in two LQT families suggests that *HERG* mutations can cause LQT.

<u>Three *HERG* point mutations associated with LQT</u>. To identify additional *HERG* mutations in *LQT2*, further SSCP analyses were performed in linked kindreds and sporadic cases. Three aberrant SSCP conformers were identified in affected members of K1956, K2596 and K2015 (Fig. 18A). In each case, the normal and aberrant conformers were cloned and sequenced. In K1956, a C to T substitution at position 1682 was identified. This mutation results in substitution of valine for a highly conserved alanine at codon 561 (A561 V), altering the fifth membrane spanning domain (S5) of the *HERG* protein (Fig. 18B). In K2596, an A to G substitution was identified at position 1408. This mutation results in substitution of aspartic acid for a conserved asparagine at codon 470 (N470D), located in the second membrane spanning domain (S2; Fig. 18B). In K2015, a G to C substitution was identified. This substitution disrupts the splice-donor sequence of intron III, affecting the cyclic nucleotide binding domain (Fig. 18B). None of the aberrant conformers was identified in DNA samples from more than 200 unaffected individuals.

*De novo* mutation of *HERG* a sporadic case of LQT. To substantiate that *HERG* mutations cause LQT, SSCP was used to screen for mutations in sporadic cases. Primer pair 4-12 identified an aberrant conformer in affected individual II-1 of K2269 (Fig. 19A). This conformer was not identified in either parent or in more than 200 unaffected individuals. Direct DNA sequencing of the aberrant conformer identified a G to A substitution at position 1882. This mutation results in substitution of serine for a highly conserved glycine at codon 628 (G628S) (Fig. 19B), altering the pore forming domain. Genotype analysis of this kindred using nine informative STR polymorphisms confirmed maternity and paternity. The identification of a *de novo* mutation in a sporadic case demonstrates that *HERG* is *LQT2.*

*HERG* is expressed in the heart. *HERG* was originally identified from a hippocampal cDNA library (Warmke and Ganetzky, 1994). To determine the tissue distribution of *HERG* mRNA, partial cDNA clones were isolated and used in Northern analyses. Northern analyses showed strongest hybridization to heart mRNAs, with faint signals in brain, liver, and pancreas (Fig. 20). Non-specific hybridization was also seen in lung, possibly due to genomic DNA contamination. The size of the bands observed in cardiac mRNA was consistent with the predicted size of *HERG.* Two bands, of ~4.1 and 4.4 kb were identified, possibly due to alternative splicing or the presence of a second related mRNA. These data indicate that *HERG* is strongly expressed in the heart, consistent with its involvement in LQT.

Mutations in *HERG* are one cause of LQT. It can be concluded that mutations in *HERG* cause the chromosome 7-linked form of LQT. Several lines of evidence support this conclusion. First, linkage analyses were used to map an LQT locus *(LQT2)* to chromosome 7q35-36 in 14 families. Second, physical and genetic mapping were used to place *HERG* in the same chromosomal region as *LQT2.* Third, it was demonstrated that *HERG* is expressed in the heart. Fourth, intragenic deletions of *HERG* associated with LQT in two families were identified. Fifth, four *HERG* point mutations in LQT patients were identified. Finally, one of the point mutations arose *de novo* and occurs within a highly conserved region encoding the potassium-selective pore domain.

The data suggest a likely molecular mechanism for chromosome 7-linked LQT. Although the function of *HERG* was not known, analyses of its predicted amino acid sequence indicated that it encodes a potassium channel α-subunit. Potassium channels are formed from four α-subunits (MacKinnon, 1991), either as homo- or hetero-tetramers (Covarrubias et al., 1991). These biophysical observations suggest that combination of normal and mutant *HERG* α-subunits could form abnormal *HERG* channels. This raises the possibility that *HERG* mutations have a dominant-negative effect on potassium channel function.

The mutations that were identified are consistent with a dominant-negative mechanism (Fig. 21). Two mutations result in premature stop codons and truncated proteins (Δ1261 and the splice-donor mutation). In the first case, only the amino terminus and a portion of the first membrane spanning domain (S1) remain. In the second, the carboxyl end of the protein is truncated, leaving all membrane spanning domains intact. *HERG* contains a cyclic nucleotide binding domain near the carboxyl terminus, and in both mutations this domain is deleted. In another mutation, an in-frame deletion of nine amino acids disrupts the third membrane spanning domain (△I500-F508). Two missense mutations also affect membrane spanning domains, A561V in the S5 domain and N470D in S2. Both mutations affect amino acids conserved in the *eag* family of potassium channels and likely alter the protein's secondary structure. The *de novo* missense mutation, G628S, occurs in the pore-forming domain. This domain is highly conserved in all potassium channel α-subunits. This mutation affects a conserved amino acid that is of known importance for ion selectivity. When this substitution was introduced into *Shaker* H4, potassium ion selectivity was lost (Heginbotham et al., 1994). As discussed above, these mutations could induce the loss of *HERG* function.

The data have implications for the mechanism of arrhythmias in LQT. Two hypotheses for LQT have previously been proposed (Schwartz et al., 1994). One suggests that a predominance of left autonomic innervation causes abnormal cardiac repolarization and arrhythmias. This hypothesis is supported by the finding that arrhythmias can be induced in dogs by removal of the right stellate ganglion. In addition, anecdotal evidence suggests that some LQT patients are effectively treated by β-adrenergic blocking agents and by left stellate ganglionectomy (Schwartz et al., 1994). The second hypothesis for LQT-related arrhythmias suggests that mutations in cardiac-specific ion channel genes, or genes that modulate cardiac ion channels, cause delayed myocellular repolarization. Delayed myocellular repolarization could promote reactivation of L-type calcium channels, resulting in secondary depolarizations (January and Riddle, 1989). These secondary depolarizations are the likely cellular mechanism of *torsade de pointes* arrhythmias (Surawicz, 1989). This hypothesis is supported by the observation that pharmacologic block of potassium channels can induce QT prolongation and repolarization-related arrhythmias in humans and animal models (Antzelevitch and Sicouri, 1994). The discovery that one form of LQT results from mutations in a cardiac potassium channel gene supports the myocellular hypothesis.

The presence of a cyclic nucleotide binding domain in *HERG* suggests a mechanism for the link between altered autonomic nervous activity and arrhythmias in LQT. β-adrenergic receptor activation increases intracellular cAMP and enhances L-type $Ca^{2+}$ channel function. Cyclic AMP may also activate *HERG,* thereby increasing net outward current and accelerating the rate of myocellular repolarization. Dominant-negative mutations of *HERG* might interrupt the normal modulation of *HERG* function by cAMP, thereby permitting a predominant effect on L-type $Ca^{2+}$ channel function. The resulting imbalance would increase the likelihood that enhanced sympathetic tone could induce $Ca^{2+}$ channel-dependent secondary depolarizations, the probable cellular mechanism of *torsades de pointes.* β-adrenergic blocking agents could act by interrupting the effect of cAMP on L-type $Ca^{2+}$ channels, possibly explaining the beneficial effects of β-blockers in some LQT patients.

The relative frequency of the three LQT loci is not yet known. In this study, five,new families with autosomal dominant LQT were identified, and all were linked to chromosome 7. This brings the total number of chromosome 7-linked families to 14. To date, seven families have been linked to chromosome 11 (*LQT1*), 14 families to chromosome 7 (*LQT2*), three families to chromosome 3 (*LQT3*) and three families remain unlinked (Keating et al., 1991 a,b; Jiang et al., 1994). Although preliminary, these data suggest that *LQT2* is a common form of inherited LQT.

This work may have important clinical implications. Recently, presymptomatic diagnosis has been possible in large families using linkage analysis. Most cases of LQT are sporadic and therefore genetic testing using linkage analysis is not feasible. Continued mutational analyses of *LQT2* and *LQT3,* will facilitate genetic testing for these forms of LQT. Identification and characterization of genes responsible for other forms of LQT will be necessary for the development of generalized diagnostic tests. Improved diagnostic capacity may enable rational therapy. For example, chromosome 7-linked LQT patients may respond to potassium channel activators, like pinacidil.

EXAMPLE 12

Generation of Polyclonal Antibody against SCN5A or HERG

Segments of *SCN5A* or *HERG* coding sequence are expressed as fusion protein in *E. coli.* The overexpressed protein is purified by gel elution and used to immunize rabbits and mice using a procedure similar to the one described by Harlow and Lane, 1988. This procedure has been shown to generate Abs against various other proteins (for example, see Kraemer et al., 1993).

Briefly, a stretch of *SCN5A* or *HERG* coding sequence is cloned as a fusion protein in plasmid PET5A (Novagen, Inc., Madison, WI). After induction with IPTG, the overexpression of a fusion protein with the expected molecular weight is verified by SDS/PAGE. Fusion protein is purified from the gel by electroelution. Identification of the protein as the *SCN5A* or *HERG* fusion product is verified by protein sequencing at the N-terminus. Next, the purified protein is used as immunogen in rabbits. Rabbits are immunized with 100 μg of the protein in complete Freund's adjuvant and boosted twice in 3 week intervals, first with 100 μg of immunogen in incomplete Freund's adjuvant followed by 100 μg of immunogen in PBS. Antibody containing serum is collected two weeks thereafter.

This procedure is repeated to generate antibodies against the mutant forms of the *SCN5A* or *HERG* gene. These antibodies, in conjunction with antibodies to wild type *SCN5A* or *HERG,* are used to detect the presence and the relative level of the mutant forms in various tissues and biological fluids.

EXAMPLE 13

Generation of Monoclonal Antibodies Specific for *SCN5A* or *HERG*

Monoclonal antibodies are generated according to the following protocol. Mice are immunized with immunogen comprising intact *SCN5A* or *HERG* or *SCN5A* or *HERG* peptides (wild type or mutant) conjugated to keyhole limpet hemocyanin using glutaraldehyde or EDC as is well known.

The immunogen is mixed with an adjuvant. Each mouse receives four injections of 10 to 100 μg of immunogen and after the fourth injection blood samples are taken from the mice to determine if the serum contains antibody to the immunogen. Serum titer is determined by ELISA or RIA. Mice with sera indicating the presence of antibody to the immunogen are selected for hybridoma production.

Spleens are removed from immune mice and a single cell suspension is prepared (see Harlow et al., 1988). Cell fusions are performed essentially as described by Kohler and Milstein, 1975. Briefly, P3.65.3 myeloma cells (American Type Culture Collection, Rockville, MD) are fused with immune spleen cells using polyethylene glycol as described by Harlow et al., 1988. Cells are plated at a density of $2x10^5$ cells/well in 96 well tissue culture plates. Individual wells are examined for growth and the supernatants of wells with growth are tested for the presence of *SCN5A* or *HERG* specific antibodies by ELISA or RIA using wild type or mutant *SCN5A* or *HERG* target protein. Cells in positive wells are expanded and subcloned to establish and confirm monoclonality.

Clones with the desired specificities are expanded and grown as ascites in mice or in a hollow fiber system to produce sufficient quantities of antibody for characterization and assay development.

EXAMPLE 14

Sandwich Assay for *SCN5A or HER*G

Monoclonal antibody is attached to a solid surface such as a plate, tube, bead, or particle. Preferably, the antibody is attached to the well surface of a 96-well ELISA plate. 100 μl sample (e.g., serum, urine, tissue cytosol) containing the *SCN5A* or *HERG* peptide/protein (wild-type or mutants) is added to the solid phase antibody. The sample is incubated for 2 hrs at room temperature. Next the sample fluid is decanted, and the solid phase is washed with buffer to remove unbound material. 100 μl of a second monoclonal antibody (to a different determinant on the *SCN5A* or *HERG* peptide/protein) is added to the solid phase. This antibody is labeled with a detector molecule (e.g., $^{125}$I, enzyme, fluorophore, or a chromophore) and the solid phase with the second antibody is incubated for two hrs at room temperature. The second antibody is decanted and the solid phase is washed with buffer to remove unbound material.

The amount of bound label, which is proportional to the amount of *SCN5A* or *HERG* peptide/protein present in the sample, is quantitated. Separate assays are performed using monoclonal antibodies which are specific for the wild-type *SCN5A* or *HERG* as well as monoclonal antibodies specific for each of the mutations identified in *SCN5A* or *HERG.*

EXAMPLE 15

Construction of an *HERG* Expression Plasmid and Transcription of cRNA

To facilitate HERG expression in *Xenopus* oocytes, the *HERG* cDNA was subcloned into a poly-A$^+$ expression vector and the 5' and 3' UTRs reduced to minimal lengths. The final *HERG* expression construct contains cDNA sequence from nucleotides -6 through 3513 in the pSP64 plasmid vector (Promega). Before use in expression experiments, the *HERG* construct was characterized by restriction mapping and DNA sequence analyses. Complementary RNAs for injection into oocytes were prepared with the mCAP RNA Capping Kit (Stratagene) following linearization of the expression construct with *EcoR*I.

EXAMPLE 16

Isolation of Oocytes and Injection of RNA

*Xenopus* frogs were anesthetized by immersion in 0.2% tricaine for 15-30 min. Ovarian lobes were digested with 2 mg/ml Type 1A collagenase (Sigma) in $Ca^{2+}$-free ND96 solution for 1.5 hours to remove follicle cells. Stage IV and V oocytes (Dumont, 1972) were injected with *HERG* cRNA (0.05 mg/ml. 50 nl), then cultured in Barth's solution supplemented with 50 μg/ml gentamycin and 1 mM pyruvate at 18°C. Barth's solution contained (in mM): 88 NaCl, 1 KCl, 0.4 $CaCl_2$, 0.33 $Ca(NO_3)_2$, 1 $MgSO_4$, 2.4 $NaHCO_3$, 10 HEPES; pH 7.4.

EXAMPLE 17

Two-microelectrode Voltage Clamp of Oocytes

Unless indicated, oocytes were bathed in ND96 solution. This solution contained (in mM): 96 NaCl, 2 KCl, 1 $MgCl_2$, 1.8 $CaCl_2$, 5 HEPES; pH 7.6. In some experiments, KCl was varied by equimolar substitution with NaCl. Currents were recorded at room temperature (21-23°C) using standard two-microelectrode voltage clamp techniques. Glass microelectrodes were filled with 3 M KCl and their tips broken to obtain tip resistances of 1 - 3 MΩ for the voltage-recording electrode and 0.6 - 1 MΩ for the current-passing electrode. Oocytes were voltage-clamped with a Dagan TEV-200 amplifier. Voltage commands were generated using pClamp software (ver. 6, Axon Instruments), a 486DX2 personal computer and a TL-1 D/A interface (Axon Instruments). Current signals were digitally sampled at a rate equal to 2-4 times the lowpass cut-off frequency (-3 db) of a 4-pole Bessel filter. Unless indicated, currents were corrected for leak and capacitance using standard, on-line P/3 leak subtraction. The oocyte membrane potential was held at -70 mV between test pulses, applied at a rate of 1 - 3 pulses/min. Data analyses, including exponential fitting of current traces, were performed using pCLAMP. Fits of appropriate data to a Boltzmann function, or Goldman-Hodgkin-Katz constant field equation (Goldman, 1943; Hodgkin and Katz, 1949) were performed using Kaleidagraph (Synergy Software). Data are expressed as the mean ± SEM (n = number of oocytes).

## LIST OF REFERENCES

Anderson, et al. (1980). Proc. Natl. Acad. Sci. USA 77, 5399-5403.
Antzelevitch, C. and Sicouri, S. (1994). J. Am. Col. Card. 23, 259-277.
Ashford, M. L. J., et al. (1994). Nature 370, 456-459.
Attwell, D., et al. (1979). Pflugers Arch. *379*, 137-142.
Ausubel, F.M., et al. (1992). Current Protocols in Molecular Biology, (J. Wiley and Sons, N.Y.)
Balser, J. R., et al. (1990). J. Gen. Physiol. 96, 835-863.
Bazette, H. C. (1920). Heart 7,353-370.
Beaucage & Carruthers (1981). Tetra. Letts. 22, 1859-1862.
Benhorin, J., et al. (1993). Science 260, 1960-1962.
Berkner, et al. (1988). BioTechniques 6, 616-629.
Berkner (1992). Curr. Top. Microbiol. Immunol.*158*, 39-61.
Blumenthal, E. M. and Kaczmarek, L. K. (1992). J. Neuroscience *12*, 290-296.
Bonner, T. I., et al. (1987). Science *237,* 527-532.
Brandyopadhyay and Temin (1984). Mol. Cell. Biol. *4*, 749-754.
Breakfield and Geller (1987). Mol. Neurobiol. *1*, 337-371.
Brinster, et al. (1981). Cell *27*,223-231.
Bruggemann, A., et al. (1993). Nature *365*, 445-448.
Buchschacher and Panganiban (1992). J. Virol. *66*, 2731-2739.
Capecchi, M.R. (1989). Science *244,* 1288.
Cariello (1988). Human Genetics *42*, 726.
Carmeliet. E. (1992). J. Pharm. Exp. Ther. 262, 809-817.
Chin, H., et al. (1991). Genomics *11*, 914-919.
Chinn, K. (1993). J. Pharmacol. Exp. Therap. 264, 553-560.
Conner, B.J., et al. (1983). Proc. Natl. Acad. Sci. USA 80, 278-282.
Constantini and Lacy (1981). Nature 294, 92-94.
Cotten, et al. (1990). Proc. Natl. Acad. Sci. USA 87, 4033-4037.
Cotton, et al. (1988). Proc. Natl. Acad. Sci. USA 85, 4397.
Covarrubias, M., et al. (1991). Neuron 7, 763-773.
Curiel, et al. (1991a). Hum. Gene Ther. 3, 147-154.
Curiel, et al. (1991 b). Proc. Natl. Acad. Sci. USA 88, 8850-8854.
Curran, M. E., et al. (1993a). Cell 73, 159-168.
Curran, M. E., et al. (1993b). J. Clin. Invest. 92, 799-803.
Curran, M. E., et al. (1995). Cell 80, 795-804.
Curry, P., et al. (1976). Lancet *II,* 231-233.
Donehower, L.A., et al. (1992). Nature *356,*215.
Dumont, J. N. (1972). J. Morphol. *136,* 153-180.
Ebers, G. C., et al. (1991). Ann. Neurol. 30, 810-816.
*Enhancers and Eukaryotic Gene Expression,* Cold Spring Harbor Press, Cold Spring Harbor, New York (1983).
Fakler, B., et al. (1995). Cell *80,* 149-154.
Feinberg, A. P. and Vogelstein, B. A. (1983). Anal. *Biochem. 132,* 6-13.
Felgner, et al. (1987). Proc. Natl. Acad. Sci. USA 84, 7413-7417.
Fiers, et al. (1978). Nature *273,* 113.
Fink, et al. (1992). Hum. Gene Ther. 3, 11-19.
Finkelstein. J., et al. (1990). Genomics 7. 167-172.
Follmer, C. H., et al. (1992). Am. J. Physiol. *262*, C75-C83.
Fontaine, B., et al. (1990). Science *250*, 1000-1002.
Freese, et al. (1990). Biochem. Pharmacol. *40*, 2189-2199.
Friedman, T. (1991). In Therapy for Genetic Diseases, T. Friedman, ed., Oxford University Press, pp. 105-121.
Gellens, M., et al. (1992). Proc. Natl. Acad. Sci. USA *89*, 554-558.
George, A.L., et al. (1995). Cytogenet. Cell. Genet. *68*, 67-70.
Gintant, G.A., et al. (1992). Time-dependent Outward Currents in the Heart. In The Heart and Cardiovascular System, H.A. Fozzard, R.B. Jennings, E. Haber, A.M. Katz and H.E. Morgan (eds.). New York, Raven Press, pp. 1121-1169.
Goldman, D. E. (1943). J. Gen. Physiol. *27*, 37-60.

Gordon, et al. (1980). Proc. Natl. Acad. Sci. USA *77*, 7380-7384.
Gorziglia and Kapikian (1992). J. Virol. *66*, 4407-4412.
Graham and van der Eb (1973). Virology *52*, 456-467.
Green, E. D., et al. (1994). Hum. Mol. Genet. *3*, 489-501.
Green, E. D., et al. Genomics, in press.
Grompe, M., 1993. Nature Genetics *5*, 111-117.
Grompe, M., et al., 1989. Proc. Natl. Acad. Sci. USA *86,* 5855-5892.
Gyapay, G., et al. (1994). Nat. Genet. *7*, 246-339.
Harlow & Lane (1988). Antibodies: A Laboratory Manual (Cold Spring Harbor Laboratory, Cold Spring Harbor, N.Y.).
Hasty, P., K., et al. (1991). Nature *350*, 243.
Heginbotham, L., et al. (1994). Biophys. J. *66*, 1061-1067
Helseth, et al. (1990). J. Virol. *64*, 2416-2420.
Hodgkin. A. L. and Katz. B. (1949). J. Physiol (Lond.) I*08*, 37-77.
Honerjager, P. (1982). Rev. Physiol. Biochem. Pharmacol. *92*, 1-74.
Innis et al. (1990). PCR Protocols: A Guide to Methods and Applications, (Academic Press, San Diego, Cal.).
Jablonski, E., et al. (1986). Nuc. Acids Res. *14*, 6115-6128.
Jakoby, W.B. and Pastan, I.H. (eds.) (1979). Cell Culture. Methods in Enzymology, volume *58* (Academic Press, Inc., Harcourt Brace Jovanovich (New York)).
January, C. T. and Riddle, J. M. (1989). Circ. Res. *64*, 977-990.
Jervell, A. and Lange-Nielson, F. (1957). Am. Heart J. *54*, 59-78.
Jiang, C., et al. (1994). Nat. Genet. *8,* 141-147.
Johnson, et al. (1992). J. Virol. *66*, 2952-2965.
Jurkiewicz, N. K. and Sanguinetti, M. C. (1993). Circ. Res. *72*, 75-83.
Kaneda, et al. (1989). J. Biol. Chem. *264*, 12126-12129.
Kanehisa (1984). Nucl. Acids Res.*12*, 203-213.
Kannel, W. B., et al. (1987). Am. Heart J. *113*, 799-804.
Keating, M. T., et al. (1991a). Science *252*, 704-706.
Keating, M. T., et al. (1991b). Am. J. Hum. Genet. *49*, 1335-1339.
Keating, M. T. (1992). Circulation *85*, 1973-1986.
Kinszler, K.W., et al. (1991). Science *251,* 1366-1370.
Koch, M. C., et al. (1992). Science *257*, 797-800,
Kohler, G. and Milstein, C. (1975). Nature *256*, 495-497.
Kraemer, F.B. et al. (1993). J. Lipid Res. *34*, 663-672.
Kubo, T., et al. (1988). FEBS Letts. *241*, 119.
Landegren. et al. (1988). Science *242*, 229.
Lathrop. G. M., et al. (1985), Am. J. Hum. Genet. *37*, 482-498.
Lerche, H., et al. (1993). J. Physiol. *470*, 13-22.
Lichter, P., et al. (1988). Hum. Genet. *80*, 224-234.
Lim, et al. (1992). Circulation *83*, 2007-2011.
Lopez-Barneo, J., et al. (1993). Receptors and Channels *1*, 61-71.
Ludwig, J., et al. (1994). EMBO J. *13*, 4451-4458.
Lynch, J. J., et al. (1994). J. Pharmacol. Exp. Ther. *269*, 541-554.
MacKinnon, R. (1991). Nature *350*, 232-235.
MacKinnon, R., et al. (1993). Science *262,* 757-759.
Madzak,etal.(1992). J. Gen. Virol. *73*, 1533-1536.
Magovcevic, I., et al. (1992). Genomics *12*, 125-129.
Maniatis, T. et al. (1982). Molecular Cloning: A Laboratory Manual (Cold Spring Harbor Laboratory, Cold Spring Harbor, N.Y.).
Mann and Baltimore (1985). J. Virol. *54,* 401-407.
Marchuk, D., et al. (1990). Nucl. Acids Res. *19*, 1154.
Margolskee (1992). Curr. Top. Microbiol. Immunol. *158,* 67-90.
Martin, R., et al. (1990). BioTechniques *9,* 762-768.
Matteucci, M.D. and Caruthers, M.H. (1981). J. Am. Chem. Soc. *103, 3185.*
Matthews & Kricka (1988). Anal. Biochem. *169*, 1.
McClatchey, A., et al. (1992a). Hum. Mol. Genet. *1*, 521-527.
McClatchey, A., et al. (1992b). Cell *68,* 769-774.
McClatchey, A., et al. (1992c). Nature Genet. *2*, 148-152.
Mettlin, C., et al. (1990). American Journal of Epidemiology *131*, 973-983.

Metzger, et al. (1988). Nature *334*, 31-36.
Miller (1992). Curr. Top. Microbiol. Immunol. *158*, 1-24.
Miller, et al. (1985). Mol. Cell. Biol. *5*. 431-437.
Miller, et al. (1988). J. Virol. *62*, 4337-4345.
Modrich, P. (1991). Ann. Rev. Genet. *25*, 229-253.
Mombaerts, P., et al. (1992). Cell *68*, 869.
Moss, A.J., et al. (1991). Circulation *84*, 1136-1144.
Moss (1992). Curr. Top. Microbiol. Immunol. *158*, 25-38.
Muzyczka (1992). Curr. Top. Microbiol. Immunol. *158*, 97-123.
Nabel, et al. (1990). Science *249*, 1285-1288.
Nabel (1992). Hum. Gene Ther. *3*, 399-410.
Newton, C.R., et al. (1989). Nucl. Acids Res. *17*, 2503-2516.
Nguyen, Q., et al. (1992). BioTechniques *13*, 116-123.
Novack, et al. (1986). Proc. Natl. Acad. Sci. USA *83*, 586.
Ohi, et al. (1990). Gene *89*, 279-282.
Orita, M., et al. (1989). Proc. Natl. Acad. Sci. USA *86*, 2766-2770.
Page, et al. (1990). J. Virol. *64*, 5370-5276.
Pardo, L. A., et al. (1992). Proc. Natl. Acad. Sci. USA *89*, 2466-2470.
Pellicer, et al. (1980). Science *209*, 1414-1422.
Petropoulos, et al. (1992). J. Virol. *66*, 3391-3397.
Philpott, K.L., et al. (1992). Science *256*, 1448.
Ptacek, L. J., et al. (1991). Cell *67*, 1021-1027.
Ptacek. L.J., et al. (1992). Neuron *8*, 891-897.
Quantin, et al. (1992). Proc. Natl. Acad. Sci. USA *89*, 2581-2584.
Rano & Kidd (1989). Nucl. Acids Res. *17*, 8392.
Rigby, P.W.J., et al. (1977). J. Mol. Biol. *113*, 237-251.
Roden, D.M. (1988). Arrhythmogenic Potential of Class III Antiarrhythmic Agents: Comparison with Class I Agents. In Control of Cardiac Arrhythmias by Lengthening Repolarization, B.N. Singh (ed.). Mt. Kisco, NY, Futura Publishing Co., pp. 559-576.
Rojas, C., et al. (1991). Nature *354*, 387-389.
Romano, C. (1965). Congenital cardiac arrhythmia. Lancet 1658-659.
Rosenfeld, et al. (1992). Cell *68*, 143-155.
Roy, N., et al. (1994). Nat. Genet. *8*, 113-114.
Rudolph, J. A., et al. (1992). Nature Genet. *2*, 144-147.
Sambrook, J., et al. (1989). Molecular Cloning: A Laboratory Manual, 2nd Ed. (Cold Spring Harbor Laboratory, Cold Spring Harbor, N.Y.).
Sanguinetti, M. C. and Jurkiewicz, N. K. (1990). Am. J. Physiol. *259*, H1881-H1889.
Sanguinetti, M. C. and Jurkiewicz, N. K. (1990). J. Gen. Physiol. *96*, 195-215.
Sanguinetti, M. C. and Jurkiewicz, N. K. (1991). Am. J. Physiol. *260,* H393-H399.
Sanguinetti, M. C. and Jurkiewicz, N. K. (1992). Pflugers Archiv *420*,180-186.
Sanguinetti, M. C., et al. (1991). Circ. Res. *68*, 77-84.
Scamps, F. and Carmeliet, E. (1989). Am. J. Physiol. *257*, C1086-C1092.
Schwartz, P. J., et al. (1975). Am. Heart J. *109*, 378-390.
Schwartz, P. J., et al. (1994). The long QT syndrome. In Cardiac Electrophysiology: from cell to bedside. D. P. Zipes and J. Jalife eds. (W.B. Sanders Company) pp.788-811.
Seino, S., et al. (1992). Genomics *13*, 1375-1377.
Sheffield, V.C., et al. (1989). Proc. Natl. Acad. Sci. USA *86,* 232-236.
Sheffield, V.C., et al., 1991. Am. J. Hum. Genet. *49*,699-706.
Shibasaki, T. (1987). J. Physiol. *387*, 227-250.
Shimada, et al. (1991). J. Clin. Invest. *88*, 1043-1047.
Shinkai, Y., et al. (1992). Cell *68*, 855.
Snouwaert, J.N., et al. (1992). Science *257*, 1083.
Sorge, et al. (1984). Mol. Cell. Biol. *4*, 1730-1737.
Stemberg, N. (1990). Proc. Natl. Acad. Sci. USA *87*, 103-107.
Stewart, et al. (1992). Hum. Gene Ther. *3*, 267-275.
Stratford-Perricaudet, et al. (1990). Hum. Gene Ther. *1*, 241-256.
Stuhmer, W., et al. (1989). Nature *339*, 597-603.
Surawicz, B. (1989). J. Am. Coll. Cardiol. *14*, 172-184.

Towbin, J. A., et al. (1994). Circulation *90*, 2635-2644.
Temple, B. L., et al. (1987). Science *237*, 770-775.
Valancius, V. & Smithies, O. (1991). Mol. Cell Biol. *11*, 1402.
Vandenberg, C. A. (1987). Proc. Natl. Acad. Sci. USA *84*, 2560-2564.
Vincent, G. M., et al. (1992). N. Engl. J. Med. *327*, 846-852.
Wagner, et al. (1991). Proc. Natl. Acad. Sci. USA *88,* 4255-4259.
Wagner, et al. (1990). Proc. Natl. Acad. Sci. USA *87,* 3410-3414.
Wang and Huang (1989). Biochemistry *28*, 9508-9514.
Wang, Q. and Keating, M. T. (1994). BioTechniques *17*,282-284.
Wang, Q., et al. (1995). Cell *80,* 805-811.
Ward, O. C. (1964). J. Ir. Med. Assoc. *54*, 103-106.
Warmke, J. E. and Ganetzky. B. (1994). Proc. Natl. Acad. Sci. *91*, 3438-3442.
Wartell, R.M., et al. (1990). Nucl. Acids Res. *18*, 2699-2705.
Weinstein. L.S.. et al. (1988). FEBS Letters *232,* 333-340.
West, J., et al. (1992). Proc. Natl. Acad. Sci. USA *89*, 10910-10914.
Wetmur & Davidson (1968). J. Mol. Biol. *31*, 349-370.
White, M.B., et al. (1992). Genomics *12*, 301-306.
Wilkinson, et al. (1992). Nucleic Acids Res. *20*, 2233-2239.
Willich, S. N., et al. (1987). Am. J. Cardiol. *60*, 801-806.
Wolff, et al. (1990). Science *247*, 1465-1468.
Wolff, et al. (1991). BioTechniques *11*, 474-485.
Wu, et al. (1989). J. Biol. Chem. *264*, 16985-16987.
Wu; et al. (1991). J. Biol. Chem. *266*,14338-14342.
Yang, N., et al. (1994). Proc. Natl. Acad. Sci. USA 91, 12785-12789.
Yang, T., et al. (1994). Circ. Res. *75*, 870-878.
Zenke, et al. (1990). Proc. Natl. Acad. Sci. USA *87*, 3655-3659.
Zipes, D.P. (1987). Am. J. Cardiol. *59*, 26E.31E.

Patents and Patent Applications:

European Patent Application Publication No. 0332435.
EPO Publication No. 225,807.
Hitzeman et al., EP 73,675A.
PCT published application WO 93/07282.
U.S. Patent 4,3 76,110.
U.S. Patent 4,486,530.
U.S. Patent 4,868,105.
U.S. Patent 5,252,479.

## Claims

**1.** An isolated DNA consisting essentially of DNA coding for a mutant SCN5A polypeptide which causes long QT syndrome wherein said mutant SCN5A polypeptide contains a mutation which is an alteration of any one or more nucleotides encoding amino acid residues 1505, 1506 or 1507 of the SCN5A polypeptide, the alteration being selected from deletion, insertion and point mutation.

**2.** An isolated DNA according to claim 1 wherein said mutation is the deletion of amino acid residues 1505-1507 of the SCN5A polypeptide.

**3.** An isolated DNA consisting essentially of DNA coding for a mutant HERG polypeptide which causes long QT syndrome wherein said mutant HERG polypeptide results from a mutation in a HERG gene which comprises at least one of the mutations in the following group: a deletion of bases 1498-1524, a deletion of base 1261, the presence of a T rather than a C at base position 1682, the presence of a G rather than an A at base position 1408, the presence of an A rather than a G at base position 1882, and the presence of a C rather than a G at the start of the splice donor site of intron III.

**4.** A nucleic acid probe which will hybridize to the DNA of claim 1 or claim 2 but will not hybridize to DNA encoding wild type SCN5A.

**5.** A nucleic acid probe which will hybridize to the DNA of claim 3 coding for a mutant HERG polypeptide but will not hybridize to DNA encoding wild type HERG.

**6.** A method for diagnosing a polymorphism which causes long QT syndrome comprising hybridizing the probe of any one of claims 4 or 5 to a patient's sample of DNA or RNA under conditions which only permit hybridization products which are fully complementary in the region of the mutation to form and determining the presence or absence of a signal indicating a hybridization product, the presence of a hybridization signal indicating the presence of long QT syndrome.

**7.** A method according to claim 6 wherein the patient's DNA or RNA has been amplified and said amplified DNA or RNA is hybridized with said probe.

**8.** A method according to claim 7 wherein hybridization is performed *in situ.*

**9.** A method for diagnosing a polymorphism which causes long QT syndrome comprising amplifying a region of an SCN5A gene or RNA encoding amino acids 1545-1507, measuring the size of the amplified region, and determining whether a deletion has occurred, said deletion being indicative of long QT syndrome.

**10.** The method of claim 9 wherein primers corresponding to SEQ ID Nos. 40 and 41 are used to amplify said region of the SCN5A gene.

**11.** A method for diagnosing a mutation which causes long QT syndrome comprising amplifying a region of a HERG gene or RNA encoding amino acid residues 500-508, measuring the size of the amplified region, and determining whether a deletion has occurred, said deletion being indicative of long QT syndrome.

**12.** The method of claim 11 wherein primers corresponding to SEQ ID Nos. 23 and 28 are used to amplify said region of the HERG gene.

**13.** A method for diagnosing a polymorphism which causes long QT syndrome comprising amplifying a region of a HERG gene or RNA surrounding base position 1261 and determining whether a deletion has occurred, said deletion being indicative of long QT syndrome.

**14.** The method of claim 13 wherein primers corresponding to SEQ ID Nos. 21 and 29 are used to amplify said region of the HERG gene.

**15.** A method for diagnosing a polymorphism which causes long QT syndrome, said polymorphism being selected from an alteration of any one or more nucleotides encoding amino acids residues 1505, 1506 or 1507 of the SCN5A polypeptide, the alteration being selected from deletion, insertion and point mutation; a deletion of bases 1498-1524 of HERG; a deletion of base 1261 of HERG; a T rather than a C at base 1682 of HERG; a G rather than an A at base 1408 of HERG; an A rather than a G at base 1882 of HERG; and a C rather than a G at the start of the splice donor site of intron III of HERG, said method comprising detecting a polymorphism by using a single-stranded conformation polymorphism technique.

**16.** The method of claim 15 wherein primers corresponding to SEQ ID Nos. 40 and 41 are used for diagnosing a polymorphism in SCNSA and a primer pair selected from the group consisting of (i) SEQ ID Nos. 23 and 29; (ii) SEQ ID Nos. 23 and 28; (iii) SEQ ID Nos. 21 and 29; (iv) SEQ ID Nos. 25 and 31; and (v) SEQ ID Nos. 24 and 32 are used for diagnosing a polymorphism in HERG.

**17.** The method of claim 16 wherein a 9 base pair deletion is seen in SCN5A, said 9 base pair deletion being indicative of long QT syndrome.

**18.** A method for diagnosing a polymorphism which causes long QT syndrome comprising amplifying a region of the SCN5A gene or RNA encoding amino acids 1505-1507 and sequencing the amplified gene or RNA wherein an alteration in or deletion of the DNA or RNA which results in an alteration or deletion of any one or more of amino acid residues 1505-1507 is indicative of long QT syndrome.

**19.** A method for diagnosing a mutation which causes long QT syndrome comprising amplifying a region of the HERG gene or RNA and sequencing the amplified gene or RNA wherein long QT syndrome is indicated by any one or more mutations of the following group: a deletion of base pairs 1498-1524, a deletion consisting of base pair 1261, a T at base position 1682, a G at base position 1408, a C at the start of the splice donor of intron III, and an A at base position 1882.

**20.** A method for diagnosing a polymorphism which causes long QT syndrome comprising identifying a mismatch between a patient's DNA SCN5A gene or RNA and a wild-type DNA or RNA probe wherein said probe hybridizes to a region of DNA encoding amino acid residues 1505-1507 of the SCN5A polypeptide.

**21.** A method for diagnosing a mutation which causes long QT syndrome comprising identifying a mismatch between a patient's DNA HERG gene or RNA and a wild-type DNA or RNA probe wherein said probe hybridizes to a region of DNA or RNA wherein said region is any one of the following group: a region comprising bases 1498-1524, a region comprising base 1261, a region comprising base 1682, a region comprising base 1408, a region comprising base 1882, and a region comprising a splice donor site of intron III.

**22.** The method of claim 20 or 21 wherein the mismatch is identified by an RNase assay.

**23.** A method for diagnosing long QT syndrome, said method comprising sequencing the SCN5A polypeptide, wherein a mutation in said polypeptide is indicative of long QT syndrome, wherein said mutation in said polypeptide is a deletion comprising amino acids 1505-1507.

**24.** A method for diagnosing long QT syndrome, said method comprising sequencing a HERG polypeptide, a mutation in said polypeptide being indicative of long QT syndrome, wherein said mutation in said polypeptide is caused by a DNA mutation, said DNA mutation being chosen from the group consisting of: deletion of bases 1498-1524, deletion of base 1261, substitution of a T for a C at base 1682, substitution of a G for an A at base 1408, substitution of an A for a G at base 1882, and substitution of a C for a G at the start of the splice donor site of intron III.

**25.** An antibody which binds to a mutant HERG polypeptide but not to a wild-type HERG polypeptide, wherein said mutant HERG polypeptide is a result of one of the mutations in the following group: deletion of bases 1498-1524, deletion of base 1261, substitution of a T for a C at base 1682, substitution of a G for an A at base 1408, substitution of an A for a G at base 1882, and substitution of a C for a G at the beginning of the splice donor site of intron III.

**26.** An antibody which binds to a mutant SCN5A polypeptide but not to wild-type SCN5A polypeptide, wherein said mutant SCN5A polypeptide is a result of the deletion of DNA encoding amino acids 1505-1507.

**27.** An antibody according to claim 25 or 26 wherein said antibody is a monoclonal antibody.

**28.** A method for diagnosing long QT syndrome said method consisting of an assay for the presence of mutant SCN5A or HERG polypeptide in a patient by reacting a patient's sample with an antibody of any one of claims 25 to 27, the presence of a positive reaction being indicative of long QT syndrome.

**29.** An isolated SCN5A polypeptide wherein said mutant SCN5A polypeptide comprises a mutation in an SCN5A gene, said mutation in said SCN5A gene consisting of a deletion of bases encoding amino acids 1505-1507 of said SCN5A polypeptide.

**30.** An isolated HERG polypeptide wherein said mutant HERG polypeptide comprises a mutation in a HERG gene, said mutation in said HERG gene being chosen from the group consisting of: deletion of bases 1498-1524, deletion of base 1261, substitution of a T for a C at base 1682, substitution of a G for an A at base 1408, substitution of an A for a G at base 1882, and substitution of a C for a G at the start of the splice donor site of intron III.

## Patentansprüche

**1.** Eine isolierte DNA, die im wesentlichen aus DNA besteht, die für ein mutiertes SCN5A Polypeptid kodiert, welches verlängertes QT Syndrom (long QT syndrome) auslöst, wobei besagtes mutiertes SCN5A Polypeptid eine Mutation beinhaltet, bei der es sich um die Änderung eines oder mehrerer beliebiger Nukleotide handelt, die die Aminosäurereste 1505, 1506 oder 1507 des SCN5A Polypeptides kodieren, wobei die Änderung ausgewählt ist aus Deletion, Insertion und Punktmutation.

**2.** Eine isolierte DNA gemäß Anspruch 1, wobei besagte Mutation die Deletion der Aminosäurereste 1505-1507 des SCN5A Polypeptides ist.

**3.** Eine isolierte DNA, die im wesentlichen aus DNA besteht, die für ein mutiertes HERG Polypeptid kodiert, welches verlängertes QT Syndrom (long QT syndrome) auslöst, wobei besagtes mutiertes HERG Polypeptid durch eine Mutation in einem HERG Gen entsteht, welches mindestens eine der Mutationen aus der folgenden Gruppe umfasst: eine Deletion der Basen 1498-1524, eine Deletion der Base 1261, die Anwesenheit eines T anstelle eines C an Basenposition 1682, die Anwesenheit eines G anstelle eines A an Basenposition 1408, die Anwesenheit eines A anstelle eines G an Basenposition 1882 und die Anwesenheit eines C anstelle eines G am Anfang der Spleißdonorstelle von Intron III.

**4.** Eine Nukleinsäureprobe, die an die DNA gemäß Anspruch 1 oder Anspruch 2 hybridisieren wird, aber nicht an DNA, die ein Wildtyp-SCN5A kodiert, hybridisieren wird.

**5.** Eine Nukleinsäureprobe, die an die DNA gemäß Anspruch 3, welche für ein mutiertes HERG Polypeptid kodiert, hybridisieren wird, aber nicht an DNA, die ein Wildtyp-HERG kodiert, hybridisieren wird.

**6.** Ein Verfahren zur Diagnose eines Polymorphismus, der verlängertes QT Syndrom (long QT syndrome) auslöst, das die Hybridisierung der Probe nach einem der Ansprüche 4 oder 5 an eine Patientenprobe aus DNA oder RNA umfasst, unter Bedingungen, die nur Hybridisierungsprodukte ermöglichen, die vollständig komplementär sind in dem Bereich, in dem die Mutation sich bildet, und die Bestimmung der Anwesenheit oder Abwesenheit eines Signals, welches auf ein Hybridisierungsprodukt hinweist, wobei die Anwesenheit eines Hybridisierungssignals auf das Vorliegen von verlängertem QT Syndrom (long QT syndrome) hinweist.

7. Ein Verfahren gemäß Anspruch 6, wobei die DNA oder RNA des Patienten amplifiziert wurde und besagte amplifizierte DNA oder RNA mit besagter Probe hybridisiert wird.

8. Ein Verfahren gemäß Anspruch 7, wobei die Hybridisierung *in situ* stattfindet.

9. Ein Verfahren zur Diagnose eines Polymorphismus, der verlängertes QT Syndrom (long QT syndrome) auslöst, das die Amplifizierung eines Bereichs eines SCN5A Gens oder einer SCN5A RNA, welcher Aminosäuren 1505-1507 kodiert, die Größenerfassung des amplifizierten Bereichs und die Bestimmung, ob eine Deletion aufgetreten ist, umfasst, wobei besagte Deletion auf verlängertes QT Syndrom (long QT syndrome) hinweist.

10. Das Verfahren gemäß Anspruch 9, wobei Primer, die den SEQ ID Nr.40 und 41 entsprechen, zur Amplifikation besagten Bereichs des SCN5A Gens verwendet werden.

11. Ein Verfahren zur Diagnose einer Mutation, die verlängertes QT Syndrom (long QT syndrome) auslöst, das die Amplifikation eines Bereichs eines HERG Gens oder einer HERG RNA, welcher Aminosäurereste 500-508 kodiert, die Größenerfassung des amplifizierten Bereichs und die Bestimmung, ob eine Deletion aufgetreten ist, umfasst, wobei besagte Deletion auf das verlängerte QT Syndrom (long QT syndrome) hinweist.

12. Das Verfahren gemäß Anspruch 11, wobei Primer, die den SEQ ID Nr. 23 und 28 entsprechen, zur Amplifikation besagten Bereichs des HERG Gens verwendet werden.

13. Ein Verfahren zur Diagnose eines Polymorphismus, der verlängertes QT Syndrom (long QT syndrome) auslöst, das die Amplifizierung eines Bereichs eines HERG Gens oder einer HERG RNA, welcher die Basenposition 1261 umgibt, und die Bestimmung, ob eine Deletion aufgetreten ist, umfasst, wobei besagte Deletion auf verlängertes QT Syndrom (long QT syndrome) hinweist.

14. Das Verfahren gemäß Anspruch 13, wobei Primer, die den SEQ ID Nr. 21 und 29 entsprechen, zur Amplifikation besagten Bereichs des HERG Gens verwendet werden.

15. Ein Verfahren zur Diagnose eines Polymorphismus, der verlängertes QT Syndrom (long QT syndrome) auslöst, wobei besagter Polymorphismus ausgewählt ist aus einer Änderung ein oder mehrerer beliebiger Nukleotide, die Aminosäurereste 1505, 1506 oder 1507 des SCN5A Polypeptids kodieren, wobei die Änderung ausgewählt ist aus Deletion, Insertion und Punktmutation; einer Deletion der Basen 1498-1524 von HERG; einer Deletion der Base 1261 von HERG; einem T anstelle eines C an der Base 1682 von HERG; einem G anstelle eines A an der Base 1408 von HERG; einem A anstelle eines G an der Base 1882 von HERG; und einem C anstelle eines G am Anfang der Spleißdonorstelle von Introns III von HERG, wobei besagtes Verfahren die Detektion eines Polymorphismus unter Verwendung der Einzelstrang-Konformations-Polymorphismus-Technik (single-stranded conformation polymorphism technique) umfasst.

16. Das Verfahren gemäß Anspruch 15, wobei Primer, die den SEQ ID Nr. 40 und 41 entsprechen, zur Diagnose eines Polymorphismus in SCN5A verwendet werden und ein Primerpaar ausgewählt aus der Gruppe bestehend aus (i) SEQ ID Nr. 23 und 29; (ii) SEQ ID Nr. 23 und 28; (iii) SEQ ID Nr. 21 und 29; (iv) SEQ ID Nr. 25 und 31; und (v) SEQ ID Nr. 24 und 32 zur Diagnose eines Polymorphismus in HERG verwendet wird.

17. Das Verfahren gemäß Anspruch 16, wobei eine 9-Basenpaar Deletion in SCN5A erkannt wird, wobei besagte 9-Basenpaar Deletion auf verlängertes QT Syndrom (long QT syndrome) hinweist.

18. Ein Verfahren zur Diagnose eines Polymorphismus, der verlängertes QT Syndrom (long QT syndrome) auslöst, das die Amplifikation eines Bereichs des SCN5A Gens oder der SCN5A RNA, welcher Aminosäuren 1505-1507 kodiert, und die Sequenzierung des amplifizierten Gens oder der amplifizierten RNA umfasst, wobei eine Änderung oder eine Deletion der DNA oder RNA, die zu einer Änderung oder Deletion einer oder mehrerer beliebiger Aminosäurereste 1505-1507 führt, auf verlängertes QT Syndrom (long QT syndrome) hinweist.

19. Ein Verfahren zur Diagnose eine Mutation, die verlängertes QT Syndrom (long QT syndrome) auslöst, das die Amplifikation eines Bereichs des HERG Gens oder der HERG RNA und die Sequenzierung des amplifizierten Gens oder der amplifizierten RNA umfasst, wobei auf verlängertes QT Syndrom (long QT syndrome) hingewiesen wird durch eine oder mehrere beliebige Mutationen der folgenden Gruppe: eine Deletion der Basenpaare 1498-1524, eine Deletion, die aus dem Basenpaar 1261 besteht, ein T an der Basenposition 1682, ein G an der Basenposition 1408, ein C am Anfang der Spleißdonorstelle des Intron III und ein A an der Basenposition 1882.

**20.** Ein Verfahren zur Diagnose eines Polymorphismus, der verlängertes QT Syndrom (long QT syndrome) auslöst, das die Identifizierung einer fehlerhaften Basenpaarung (mismatch) zwischen der DNA des SCN5A Gens oder RNA eines Patienten und einer Wildtyp-DNA- oder RNA-Probe umfasst, wobei besagte Probe an einen Bereich der DNA hybridisiert, der die Aminosäurereste 1505-1507 des SCN5A Polypeptides kodiert.

**21.** Ein Verfahren zur Diagnose einer Mutation, die verlängertes QT Syndrom (long QT syndrome) auslöst, das die Identifizierung einer fehlerhaften Basenpaarung (mismatch) zwischen der DNA des HERG Gens oder HERG RNA eines Patienten und einer Wildtyp DNA- oder RNA-Probe umfasst, wobei besagte Probe an einen Bereich der DNA oder RNA hybridisiert, wobei besagter Bereich ein beliebiger aus der folgenden Gruppe ist: ein Bereich, der die Basen 1498-1524 umfasst, ein Bereich, der die Base 1261 umfasst, ein Bereich, der die Base 1682 umfasst, ein Bereich, der die Base 1408 umfasst, ein Bereich, der die Base 1882 umfasst, und ein Bereich, der eine Spleißdonorstelle von Intron III umfasst.

**22.** Das Verfahren gemäß Anspruch 20 oder 21, wobei die fehlerhafte Basenpaarung (mismatch) durch einen RNase-Test bestimmt wird.

**23.** Ein Verfahren zur Diagnose von verlängertem QT Syndrom (long QT syndrome), wobei besagtes Verfahren die Sequenzierung des SCN5A Polypeptids umfasst, wobei eine Mutation in besagtem Polypeptid auf verlängertes QT Syndrom (long QT syndrome) hinweist, wobei besagte Mutation in besagtem Polypeptid eine Deletion ist, die die Aminosäuren 1505-1507 umfasst.

**24.** Ein Verfahren zur Diagnose von verlängertem QT Syndrom (long QT syndrome), wobei besagtes Verfahren die Sequenzierung eines HERG Polypeptids umfasst, wobei eine Mutation in besagtem Polypeptid auf verlängertes QT Syndrom (long QT syndrome) hinweist, wobei besagte Mutation in besagtem Polypeptid durch eine DNA Mutation hervorgerufen wird, wobei besagte DNA Mutation ausgewählt ist aus der Gruppe bestehend aus: Deletion der Basen 1498-1524, Deletion der Base 1261, Substitution eines C durch ein T an der Base 1682, Substitution eines A durch ein G an der Base 1408, Substitution eines G durch ein A an der Base 1882 und Substitution eines G durch ein C am Anfang der Spleißdonorstelle von Intron III.

**25.** Ein Antikörper, der an ein mutiertes HERG Polypeptid aber nicht an ein Wildtyp-HERG Polypeptid bindet, wobei besagtes mutiertes HERG Polypeptid aus einer der Mutationen aus der folgenden Gruppe resultiert: Deletion der Basen 1498-1524, Deletion der Base 1261, Substitution eines C durch ein T an der Base 1682, Substitution eines A durch ein G an der Base 1408, Substitution eines G durch ein A an der Base 1882 und Substitution eines G durch ein C am Beginn der Spleißdonorstelle von Intron III.

**26.** Ein Antikörper, der an ein mutiertes SCN5A Polypeptid, aber nicht an ein Wildtyp-SCN5A Polypeptid bindet, wobei besagtes mutiertes SCN5A Polypeptid aus der Deletion von DNA resultiert, die Aminosäuren 1505-1507 kodiert.

**27.** Ein Antikörper gemäß Anspruch 25 oder 26, wobei besagter Antikörper ein monoklonaler Antikörper ist.

**28.** Ein Verfahren zur Diagnose des verlängerten QT Syndroms (long QT syndrome), wobei besagtes Verfahren in einem Test auf das Vorliegen eines mutierten SCN5A oder HERG Polypeptides in einem Patienten besteht, bei dem man einen Antikörper gemäß einem der Ansprüche 25 bis 27 mit einer Patientenprobe reagieren läßt, wobei das Vorliegen einer positiven Reaktion auf verlängertes QT Syndrom (long QT syndrome) hinweist.

**29.** Ein isoliertes SCN5A Polypeptid, wobei besagtes mutiertes SCN5A Polypeptid eine Mutation in einem SCN5A Gen umfasst, wobei besagte Mutation in besagtem SCN5A Gen in einer Deletion der Basen besteht, die Aminosäuren 1505-1507 des besagten SCN5A Polypeptids kodieren.

**30.** Ein isoliertes HERG Polypeptid, wobei besagtes mutiertes HERG Polypeptid eine Mutation in einem HERG Gen umfasst, wobei besagte Mutation in besagtem HERG Gen ausgewählt ist aus der Gruppe bestehend aus: Deletion der Basen 1498-1524, Deletion der Base 1261, Substitution eines C durch ein T an der Base 1682, Substitution eines A durch ein G an der Base 1408, Substitution eines G durch ein A an der Base 1882 und Substitution eines G durch ein C am Anfang der Spleißdonorstelle von Intron III.

## Revendications

1. Un ADN isolé consistant essentiellement en ADN codant pour un polypeptide SCN5A mutant qui provoque le syndrome du long QT, ledit polypeptide SCN5A mutant contenant une mutation qui est une altération d'un quelconque ou de plusieurs nucléotides codant pour des résidus d'acides aminés 1505, 1506 ou 1507 du polypeptide SCN5A, l'altération étant choisie parmi les délétion, insertion et mutation ponctuelles.

2. Un ADN isolé selon la revendication 1, dans lequel ladite mutation est la délétion des résidus d'acides aminés 1505-1507 du polypeptide SCN5A.

3. Un ADN isolé consistant essentiellement en ADN codant pour un polypeptide HERG mutant qui provoque le syndrome du long QT, ledit polypeptide HERG mutant résultant d'une mutation d'un gène HERG qui comprend au moins une des mutations du groupe comprenant: la délétion des bases 1498-1524, la délétion de la base 1261, la présence d'un T au lieu d'un C à la position de la base 1682, la présence d'un G au lieu d'un A à la position de la base 1408, la présence d'un A au lieu d'un G à la position de la base 1882 et la présence d'un C à la place d'un G au départ du site donneur d'épissure de l'intron III.

4. Une sonde d'acide nucléique qui s'hybride à l'ADN selon la revendication 1 ou 2, mais ne s'hybride pas à l'ADN codant pour le SCN5A de type naturel.

5. Une sonde d'acide nucléique qui s'hybride à l'ADN selon la revendication 3 codant pour un polypeptide HERG mutant mais ne s'hybride pas à l'ADN codant pour le HERG de type sauvage.

6. Une méthode de diagnostic d'un polymorphisme qui provoque le syndrome du long QT comprenant l'hybridation de la sonde selon l'une quelconque des revendications 4 ou 5 à un échantillon d'ADN ou d'ARN d'un patient dans des conditions qui ne permettent que l'hybridation de produits qui sont totalement complémentaires dans la région de mutation pour former et déterminer la présence ou l'absence d'un signal indiquant un produit d'hybridation, la présence du signal d'hybridation indiquant la présence du syndrome de long QT.

7. Une méthode selon la revendication 6, dans laquelle l'ADN ou l'ARN du patient a été amplifié et ledit ADN ou ARN amplifié est hybridé avec ladite sonde.

8. Une méthode selon la revendication 7, dans laquelle on effectue l'hybridation *in situ.*

9. Une méthode de diagnostic d'un polymorphisme qui provoque le syndrome du long QT comprenant l'amplification d'une région d'un gène de SCN5A ou d'ARN codant pour les acides aminés 1505-1507, la mesure de la taille de la région amplifiée et la détermination du fait qu'une délétion s'est produite, ladite délétion étant indicative du syndrome du long QT.

10. La méthode selon la revendication 9, dans laquelle les amorces correspondant aux SEQ ID n° 40 et 41 sont utilisées pour amplifier ladite région du gène SCN5A.

11. Une méthode de diagnostic d'une mutation qui provoque le syndrome du long QT comprenant l'amplification d'une région du gène de HERG ou d'un ARN codant pour des résidus d'acides aminés 500-508, la mesure de la taille de la région amplifiée, et la détermination si une délétion s'est produite, ladite délétion étant indicative du syndrome du long QT.

12. La méthode selon la revendication 11, dans laquelle on utilise des amorces correspondant aux SEQ ID N° 23 et 28 pour amplifier ladite région du gène de HERG.

13. Une méthode de diagnostic d'un polymorphisme qui provoque le syndrome du long QT comprenant l'amplification d'une région du gène de HERG ou de l'ARN entourant la position de la base 1261 et la détermination si la délétion s'est produite, ladite délétion étant indicative du syndrome du long QT.

14. La méthode selon la revendication 13, dans laquelle les amorces correspondantes aux SEQ ID n° 21 et 29 servent pour l'amplification de la région du gène de HERG.

**15.** Une méthode de diagnostic d'un polymorphisme qui provoque le syndrome du long QT, ledit polymorphisme étant sélectionné dans le groupe comprenant:

- l'altération d'un quelconque ou de plusieurs nucléotides codant pour des résidus d'acides aminés 1505, 1506 ou 1507 du polypeptide SCN5A, l'altération étant choisie parmi les délétion, insertion et mutation ponctuelles;
- la délétion des bases 1498-1524 de HERG;
- la délétion de la base 1261 de HERG;
- un T au lieu d'un C à la position de la base 1682 de HERG;
- un G au lieu d'un A à la position de la base 1408 de HERG;
- un A au lieu d'un G à la position de la base 1882 de HERG; et
- un C au lieu d'un G au départ du site donneur d'épissure de l'Intron III de HERG;

ladite méthode comprenant la détection d'un polymorphisme à l'utilisation d'une technique de polymorphisme de conformation monocaténaire.

**16.** La méthode selon la revendication 15, dans laquelle les amorces correspondant aux SEQ ID N°40 et 41 sont utilisés pour le diagnostic d'un polymorphisme dans SCN5A et une paire d'amorces choisies dans le groupe comprenant:

(i) SEQ ID n° 23 et 29;
(ii) SEQ ID n° 23 et 28;
(iii) SEQ ID n° 21 et 29;
(iv) SEQ ID n° 25 et 31; et
(v) SEQ ID n° 24 et 32

est utilisée pour les diagnostics de polymorphisme dans HERG.

**17.** La méthode selon la revendication 16, dans laquelle on observe une délétion de la paire de bases 9 dans du SCN5A, ladite délétion de la paire de bases 9 étant indicative d'un syndrome de long QT.

**18.** Une méthode de diagnostic d'un polymorphisme qui provoque le syndrome du long QT, comprenant l'amplification d'une région du gène SCN5A ou d'un ARN codant pour les acides aminés 1505-1507 et le séquençage du gène amplifié ou de l'ARN dans laquelle une altération ou une délétion de l'ADN ou de l'ARN qui conduit à une altération ou à une délétion d'un ou plusieurs des résidus acides aminés 1505-1507 est indicative du syndrome de long QT.

**19.** Une méthode de diagnostic d'une mutation qui provoque le syndrome du long QT comprenant l'amplification d'une région du gène de HERG ou d'un ARN et le séquençage du gène amplifié ou ARN dans lequel le syndrome du long QT est indiqué par une ou plusieurs mutations choisies dans le groupe formé par: une délétion de la paire de bases 1498-1524, une délétion consistant en la paire des bases 1261, un T à la position de la base 1682, un G à la position de la base 1408, un C au départ du donneur d'épissure de l'intron III et un A à la position de la base 1882.

**20.** Une méthode de diagnostic d'un polymorphisme qui provoque le syndrome du long QT, comprenant l'identification d'un mésappareillement entre un gène SCN5A d'ADN ou d'ARN d'un patient et d'une sonde d'ADN ou d'ARN de type sauvage, dans laquelle ladite sonde s'hybride à une région d'ADN codant pour des résidus acides aminés 1505-1507 du polypeptide SCN5A.

**21.** Une méthode de diagnostic d'une mutation qui provoque le syndrome du long QT, comprenant l'identification d'un mésappareillement entre le gène de HERG d'ADN ou d'ARN d'un patient et une sonde d'ADN ou d'ARN de type sauvage, ladite sonde s'hybridant à une région de l'ADN ou de l'ARN, ladite région étant l'une quelconque du groupe formé par les régions suivantes: une région comprenant les bases 1498-1524, une région comprenant la base 1261, une région comprenant la base 1682, une région comprenant la base 1408, une région comprenant la base 1882, et une région comprenant le site donneur d'épissure de l'intron III.

**22.** La méthode selon la revendication 20 ou 21, dans laquelle le mésappareillement est identifié par un test de RNase.

**23.** Une méthode de diagnostic du syndrome de long QT, ladite méthode comprenant le séquençage d'un polypeptide SCN5A, la mutation dans ledit polypeptide étant indicative du syndrome de long QT, et ladite mutation dudit polypeptide étant une délétion comprenant les acides aminés 1505-1507.

**24.** Une méthode de diagnostic du syndrome de long QT, ladite méthode comprenant le séquençage d'un polypeptide HERG, une mutation dudit polypeptide étant indicative du syndrome de long QT, ladite mutation dudit polypeptide étant provoquée par une mutation de l'ADN, ladite mutation de l'ADN étant choisie dans le groupe comprenant: la délétion des bases 1498-1524, la délétion de la base 1261, le remplacement d'un C par un T à la position de la base 1682, le remplacement d'un A par un G à la position de la base 1408, le remplacement d'un G par un A à la position de la base 1882, et remplacement d'un G par un C au départ du site donneur d'épissure de l'intron III.

**25.** Un anticorps qui se fixe à un polypeptide HERG mutant mais non à un polypeptide HERG de type sauvage, ledit polypeptide HERG mutant étant le résultat de l'une des mutations du groupe comprenant: la délétion des bases 1498-1524, délétion de la base 1261, le remplacement d'un C par un T à la position de la base 1682, le remplacement d'un A par un G à la position de la base 1408, le remplacement d'un G par un A à la position de la base 1882, et le remplacement d'un G par un C au début du site donneur d'épissure de l'intron III.

**26.** Un anticorps qui se fixe à un polypeptide SCN5A mutant mais non à un polypeptide SCN5A de type sauvage, ledit polypeptide SCN5A mutant étant le résultat de la délétion d'un ADN codant pour les acides aminés 1505-1507.

**27.** Un anticorps selon la revendication 25 ou 26 dans lequel ledit anticorps est un anticorps monoclonal.

**28.** Un procédé de diagnostic du syndrome du long QT, ladite méthode consistant en un test pour déterminer la présence de polypeptide SCN5A ou HERG mutant chez un patient en faisant réagir un échantillon d'un patient avec un anticorps selon l'une quelconque des revendications 25 à 27, la présence d'une réaction positive étant indicative du syndrome de long QT.

**29.** Un polypeptide SCN5A isolé, dans lequel ledit polypeptide SCN5A mutant comprend une mutation au niveau d'un gène de SCN5A, ladite mutation au niveau dudit gène SCN5A consistant en une délétion de bases codant pour les acides aminés 1505-1507 du polypeptide SCN5A.

**30.** Un polypeptide HERG isolé, dans lequel ledit polypeptide HERG mutant comprend une mutation au niveau d'un gène du HERG, ladite mutation dudit gène du HERG étant choisi dans le groupe comprenant: la délétion des bases 1498-1524, la délétion de la base 1261, le remplacement d'un C par un T au niveau de la base 1682, le remplacement d'un A par un G au niveau de la base 1408, le remplacement d'un G par un A au niveau de la base 1882, et le remplacement d'un G par un C au départ du site donneur d'épissure de l'intron III.

K2322
D3S1298
SCNSA 7-5
SCNSA 3-4
D2S1100

FIG. 1A

K2321
D3S1298
SCNSA 7-8
SCNSA 3-4
D3S1100

FIG. 1C

K2171

FIG. 1B

```
SCN5A
human heart muscle        DNFNQQKKKLGGQ  DIFMTEEQKKYYNAMKKLGSKKPQKPIPRPLNKYQGFIFD

rat heart                 .............  ........................................

SCN4A
human skeletal muscle     ............K  ..............................Q..I..MVY.

SCN1A
human brain               .........F...  ..............................A..F..MV..

rat skeletal muscle       .........F..K  ..............................Q..I..MVY.

rat brain II              .........F...  ..............................A..F..MV..

rat brain I               .........F...  ..............................G..F..MV..

Fugu rubripes             .........F...  ..............................Q..I..MV..

Loligo opalescens         ........GA..S  LEV...DD.....K...N.Q....T.G..M.GF.IAEWM.H

Loligo bleekiri           .K.SFL...YD.TYL.M.L.PT.QN...TL....T....TVK..K..C.AVVY.

Drosophila melanogaster   ....MLRRSIE.V  LEM.L..S..H..T......R....V.K..I.HFLAMFY.

Heliothis virescens       ....E....AA.S  LEM....D.........M....L.A....KWRP.AIV.E
```

FIG. 2

Vt = -50 to -10 mV
800
nA
400
0
0
2500
5000
7500
msec

FIG.3A

Vt = 0 to +40 mV
1200
nA
800
400
0
0
2500
5000
7500
msec

FIG.3B

current (nA)
test potential (mV)
relative tail current
half-point = -15.1 mV
slope = 7.85 mV
test potential (mV)
time constant (msec)
activation
deactivation
test potential (mV)
time constant of deactivation (sec)
relative amplitude of fast component
fast time constant
slow time constant
mV
AMP-fast/(AMP-fast + slow)

FIG.3C

FIG.3D

FIG.4C

FIG.4D

Activation
750
nA
500
250
0
0
2500
5000
7500
msec

FIG.4A

Deactivation
1000
nA
0
0
8
16
sec

FIG.4B

2 mM K
800
+20
-70
nA
0
-800
0
2500
5000
7500
msec
FIG.5A
10 mM K
2000
1000
nA
0
-1000
FIG.5B
-40
-60
-80
-100
-120
$E_{rev}$(mV)
58 mV/decade
1
10
$[K]_0$(mM)
FIG.5C

1200
800
400
0
nA
10 mM K
+20
-70
0
msec
2500
5000
7500
800
400
0
nA
2 mM K
400
0
nA
0 mM K
1250
1000
750
500
250
0
current (nA)
10 K+
2 K+
0 K+
-40
-20
0
20
test potential (mV)
1000
800
600
400
200
0
current (nA)
0
5
10
15
20
[K+]0(mM)


FIG.6A

FIG.6B

FIG.6C

FIG.6D

FIG.6E

+40
+20
90 msec
0
μA
-2.5
-5.0
-40
0
+20
-70
-120
Recovery from fast inactivation
time constant (msec)
20
18
16
14
12
10
8
6
4
-120
-80
-40
0
40
test potential (mV)

FIG.7A

FIG.7B

slope = 118µS
tail current (µA)
test potential (mV)
half-point = 49 mv
slope factor = 28mV
Rectification factor (R)
test potential (mV)

FIG.7C

FIG.7D

FIG.8A
1600
800
0
nA
+50
-70
control
0
2500
5000
7500
msec
FIG.8B
800
nA
0
10 µM La3+
0
2500
5000
7500
msec
control
10 µM La3+
700
600
500
400
300
200
100
0
current (nA)
-60
-40
-20
0
20
40
test potential (mV)
FIG.8C
1600
1400
1200
1000
800
600
400
200
0
tail current (nA)
control
10 µM La3+
-60
-40
-20
0
20
40
test potential (mV)
FIG.8D

K2321
244 bp
235 bp

FIG. 9

K2322
244 bp
235 bp

FIG. 10

G S K K P Q K P I P
G G C T C C A A G A A G C C C C A G A A G C G C A T C C C
190
200
210
NORMAL
G S K K P I P
G G C T C C A A G A A G C C G A T C C C
220
230
$\Delta K_{1505}$-$Q_{1507}$

FIG. 11A

FIG. 11B

DI
DII
DIII
DIV
S1
S6
NH2
COOH

FIG.12

K1956
I
1
2
D7S505 1 2 (3 3)
D7S636 6 2 (5 6)
HERG5 1 1 (1 1)
HERG3 1 1 (3 2)
D7S483 1 1 (1 1)
II
1
2
3
4
5
2 3 2 3 2 3 (- 3) (- 3)
2 6 2 5 2 6 (- 5) (- 4)
1 1 1 1 1 1 (- 1) (- 1)
1 2 1 3 1 2 (- 3) (- 1)
1 1 1 1 1 1 (- 1) (- 2)
III
1
3 3
4 5
1 1
1 3
2 1

FIG. 13A

K2287
I
II
III
D7S505
D7S636
HERG5
HERG3
D7S483

FIG. 13B

K2595
I
II
III
IV
D7S505
D7S636
HERG5
HERG3
D7S483

FIG. 13C

K2596
I
1
2
D7S505
D7S636
HERG5
HERG3
D7S483
(- 4)
(- 2)
(- 1)
(- 2)
(- 2)
4 4
1 5
1 2
2 1
4 1
II
1
2
3
(- 3)
(- 3)
(- 2)
(- 1)
(- 4)
4 4
2 5
1 2
2 1
2 1
1 2
4 6
1 1
2 2
3 5
III
1
2
3 4
3 2
2 1
1 2
4 2
2 4
6 2
1 1
2 2
5 2

FIG. 13D

K2600
I
II
III
D7S505
D7S636
HERG5
HERG3
D7S483

FIG. 13E

22
21
15
14
13
12
11.2
11.21
11.22
11.23
21
22
31.1
31.2
31.3
32
33
34
35
36
7
D7S505
D7S636
HERG/LQT2
D7S483

FIG.14

S1
S2
S3
Intron I ~625 bp
S4
S5
Pore
S
Intron II ~325 bp
6
1
2
3
4
5
6
7
8
9
10
11
12
13
NBD
Intron III ~1100 bp
NBD
14
15
16
17
Scale 250 bp


FIG. 15

K2287
←170 bp
←143 bp
Normal
Deletion
G A T C
G A T C

FIG. 16A

FIG. 16B

TGG TTC CTC ATC GAC ATG GTG GCC GCC ATC CCC TTC GAC CTG CTC
W F L I D M V A A I P F D L L
S3
Normal GTC ATC TAC ACG GCT GTC TTC ACA CCC TAC TCG GCT GCC TTC CTG CTG AAG GAG
V I Y T A V F T P Y S A A F L L K E
Deletion GTC ATC TAC CGG CTG TCT TCA CAC CCT ACT CGG CTG CCT TCC TGC TGA
V I Y R L S S H P T R L P S C

FIG. 16C

FIG. 17C

K2595

FIG. 17A

Normal
Deletion
G A T C
G A T C

FIG. 17B

K1956
FIG. 18A
K2596
FIG. 18C
K2015
Sporadic LQT
N A
FIG. 18E
Normal
Missense
G A T C
G A T C
C/T
FIG. 18B
Normal
Missense
G A T C
G A T C
T/C
FIG. 18D
Normal
Affected
G A T C
G A T C
C/G
FIG. 18F

GTG → V
GCG
L I A H W L
S5
SEQ ID NO.:
67
K1956 L I V H W L 68
H-Erg L I A H W L 67
M-Eag L A A H W K 69
R-Eag L A A H W M 70
Eag L V A H W L 71
Elk L A A H W L 72
GAC → D
AAC
D I L I N F R
S2
SEQ ID NO.:
73
K2596 D I L I D F R 74
H-Erg D I L I N F R 73
M-Eag D I V L N F H 75
R-Eag D I V L N F H 75
Eag D I V L N F H 75
Elk D I L L N F R 76
C
5'-CAT CCT GG // gtatggg-3"

FIG. 18G

FIG. 18H

FIG. 18I

K2269
Normal
Missense
C / T
G A T C
G A T C

FIG. 19A

FIG. 19B

| | | | | AGC → S<br>GGC | | | | SEQ ID NO.: |
|---|---|---|---|---|---|---|---|---|
| | S | V | G | F | G | N | V | S | 77 |
| | ← Pore → | | | | | | | | |
| K2269 | S | V | G | F | S | N | V | S | 78 |
| H-Erg | S | V | G | F | G | N | V | S | 77 |
| M-Eag | S | V | G | F | G | N | I | A | 79 |
| R-Eag | S | V | G | F | G | N | I | A | 79 |
| Eag | S | V | G | F | G | N | V | A | 80 |
| Elk | S | V | G | F | G | N | V | S | 77 |
| Shaker | T | V | G | Y | G | D | M | T | 81 |

FIG. 19C

HEARTBRAIN
PLACENTA
LUNG
LIVER
SKELETAL MUSCLE
KIDNEY
PANCREAS
KB
9.5
7.5
4.4
2.4
1.35

FIG. 20

S1
S2
S3
S4
S5
Pore
S6
Extracellular
Intracellular
Frameshift
N470D
Δ I500-F508
A561V
G628S
Nucleotide binding
domain
Splice error
COO-
NH3+

FIG.21

SEQUENCE LISTING

(1) GENERAL INFORMATION:

(i) APPLICANT: University of Utah Research Foundation

(ii) TITLE OF INVENTION: Long QT Syndrome Genes and Methods for Diagnosis or Preventing the Occurrence of Long QT Syndrome and/or Torsade de *Pointes*

(iii) NUMBER OF SEQUENCES: 81

(iv) CORRESPONDENCE ADDRESS:

(A) ADDRESSEE: Venable, Baetjer, Howard & Civiletti, LLP
(B) STREET: 1201 New York Avenue, Suite 1000

(C) CITY: Washington
(D) STATE: DC
(E) COUNTRY: USA
(F) ZIP: 20005-3917

(v) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: Word for Windows 6.0

(vi) CURRENT APPLICATION DATA:

(A) APPLICATION NUMBER: WO
(B) FILING DATE: 08-MAR-1996
(C) CLASSIFICATION:

(vii) PRIOR APPLICATION DATA:

(A) APPLICATION NUMBER: US 08/426,118
(B) FILING DATE: 20 APR-1995

(vii) PRIOR APPLICATION DATA:

(A) APPLICATION NUMBER: US 08/401,512
(B) FILING DATE: 09-MAR-1995

(viii) ATTORNEY/AGENT INFORMATION:

(A) NAME: Ihnen, Jeffrey L.
(B) REGISTRATION NUMBER: 28,957
(C) REFERENCE/DOCKET NUMBER: 19780-113879-PCT

(ix) TELECOMMUNICATION INFORMATION:

(A) TELEPHONE: 202-962-4810
(B) TELEFAX: 202-962-8300

(2) INFORMATION FOR SEQ ID NO:1:

(i) SEQUENCE CHARACTERISTICS:

(A) LENGTH: 26 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(vi) ORIGINAL SOURCE:

(A) ORGANISM: Homo sapiens

(xi) SEQUENCE DESCRIPTION: SEQ ID NO:1:

ACTTTCATCG TACTGAATAA AGGCAA 26

(2) INFORMATION FOR SEQ ID NO:2:

(i) SEQUENCE CHARACTERISTICS:

(A) LENGTH: 23 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(vi) ORIGINAL SOURCE:

(A) ORGANISM: Homo sapiens

(xi) SEQUENCE DESCRIPTION: SEQ ID NO:2:

GAGTGAACCA GAATCTTCAC AGC 23

(2) INFORMATION FOR SEQ ID NO:3:

(i) SEQUENCE CHARACTERISTICS:

(A) LENGTH: 22 base pairs
(B) TYPE: nucleic acid
(C) STRANDEDNESS: single
(D) TOPOLOGY: linear.

(ii) MOLECULE TYPE: cDNA

(iii) HYPOTHETICAL: NO

(vi) ORIGINAL SOURCE:

(A) ORGANISM: Homo sapiens

(xi) SEQUENCE DESCRIPTION: SEQ ID NO:3:

GGACCGTGAG TCCATCGTGT GA 22

(2) INFORMATION FOR SEQ ID NO:4:

(i) SEQUENCE CHARACTERISTICS:

(A) LENGTH: 25 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(vi) ORIGINAL SOURCE:

(A) ORGANISM: Homo sapiens

(xi) SEQUENCE DESCRIPTION: SEQ ID NO:4:

AGCCCATTCA CAACATATAC AGTCT 25

(2) INFORMATION FOR SEQ ID NO:5:

(i) SEQUENCE CHARACTERISTICS:

(A) LENGTH: 24 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(vi) ORIGINAL SOURCE:

(A) ORGANISM: Homo sapiens

(xi) SEQUENCE DESCRIPTION: SEQ ID NO:5:

AGCAACTTCA TCCCAGCTGC TGAG 24

(2) INFORMATION FOR SEQ ID NO:6:

(i) SEQUENCE CHARACTERISTICS:

(A) LENGTH: 23 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(vi) ORIGINAL SOURCE:

(A) ORGANISM: Homo sapiens

(xi) SEQUENCE DESCRIPTION: SEQ ID NO:6:

CTCCCAGCAT CTCAGGTCAA GTG 23

(2) INFORMATION FOR SEQ ID NO:7:

(i) SEQUENCE CHARACTERISTICS:

(A) LENGTH: 24 base pairs

(B) TYPE: nucleic acid
(C) STRANDEDNESS: single
(D) TOPOLOGY: linear

(ii) MOLECULE TYPE: cDNA

(iii) HYPOTHETICAL: NO

(vi) ORIGINAL SOURCE:

(A) ORGANISM: Homo sapiens

(xi) SEQUENCE DESCRIPTION: SEQ ID NO:7:

TATGAAGAGC AGCCTCAGTG GGAA 24

(2) INFORMATION FOR SEQ ID NO:8:

(i) SEQUENCE CHARACTERISTICS:

(A) LENGTH: 25 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(vi) ORIGINAL SOURCE:

(A) ORGANISM: Homo sapiens

(xi) SEQUENCE DESCRIPTION: SEQ ID NO:8:

CTTTTTCTTC TGTTGGTTGA AGTTG 25

(2) INFORMATION FOR SEQ ID NO:9:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(vi) ORIGINAL SOURCE:

(A) ORGANISM: Homo sapiens

(xi) SEQUENCE DESCRIPTION: SEQ ID NO:9:

TTAGGGGGCC AGGACATCTT C 21

(2) INFORMATION FOR SEQ ID NO:10:

(i) SEQUENCE CHARACTERISTICS:

(A) LENGTH: 25 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(vi) ORIGINAL SOURCE:

(A) ORGANISM: Homo sapiens

(xi) SEQUENCE DESCRIPTION: SEQ ID NO:10:

CAGGGGCCGT GGGATGGGCT TCTGG 25

(2) INFORMATION FOR SEQ ID NO:11:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(vi) ORIGINAL SOURCE:

(A) ORGANISM: Homo sapiens

(xi) SEQUENCE DESCRIPTION: SEQ ID NO:11:

CACCATATTC AAGCAGATCA G 21

(2) INFORMATION FOR SEQ ID NO:12:

(i) SEQUENCE CHARACTERISTICS:

(A) LENGTH: 21 base pairs
(B) TYPE: nucleic acid
(C) STRANDNESS: single
(D) TOPOLOGY: linear

(ii) MOLECULE TYPE: cDNA
(iii) HYPOTHETICAL: NO
(vi) ORIGINAL SOURCE:

(A) ORGANISM: Homo sapiens

(xi) SEQUENCE DESCRIPTION: SEQ ID NO:12:

CTGCGCCACT ACTACTTCAC C 21

(2) INFORMATION FOR SEQ ID NO:13:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(vi) ORIGINAL SOURCE:

(A) ORGANISM: Homo sapiens

(xi) SEQUENCE DESCRIPTION: SEQ ID NO:13:

GCTGGGCCGC TCCCCTTGGA 20

(2) INFORMATION FOR SEQ ID NO:14:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(vi) ORIGINAL SOURCE:

(A) ORGANISM: Homo sapiens

(xi) SEQUENCE DESCRIPTION: SEQ ID NO:14:

GCATCTTCAT TAATTATTCA 20

(2) INFORMATION FOR SEQ ID NO:15:

(i) SEQUENCE CHARACTERISTICS:

(A) LENGTH: 11 base pairs
(B) TYPE: nucleic acid
(C) STRANDEDNESS: single
(D) TOPOLOGY: linear

(ii) MOLECULE TYPE: DNA (genomic)
(iii) HYPOTHETICAL: NO
(vi) ORIGINAL SOURCE:

(A) ORGANISM: Homo sapiens

(xi) SEQUENCE DESCRIPTION: SEQ ID NO:15:

AGGAGGTGGG G 11

(2) INFORMATION FOR SEQ ID NO:16:

(i) SEQUENCE CHARACTERISTICS:

(A) LENGTH: 12 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(vi) ORIGINAL SOURCE:

(A) ORGANISM: Homo sapiens

(xi) SEQUENCE DESCRIPTION: SEQ ID NO:16:

CCCCAGCTGA TC 12

(2) INFORMATION FOR SEQ ID NO:17:

(i) SEQUENCE CHARACTERISTICS:

(A) LENGTH: 11 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(vi) ORIGINAL SOURCE:

(A) ORGANISM: Homo sapiens

(xi) SEQUENCE DESCRIPTION: SEQ ID NO:17:

TGGCTGTGAG T 11

(2) INFORMATION FOR SEQ ID NO:18:

(i) SEQUENCE CHARACTERISTICS:

(A) LENGTH: 11 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(vi) ORIGINAL SOURCE:

(A) ORGANISM: Homo sapiens

(xi) SEQUENCE DESCRIPTION: SEQ ID NO:18:

CCCCAGCCCT C 11

(2) INFORMATION FOR SEQ ID NO:19:

(i) SEQUENCE CHARACTERISTICS:

(A) LENGTH: 11 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(vi) ORIGINAL SOURCE:

(A) ORGANISM: Homo sapiens

(xi) SEQUENCE DESCRIPTION: SEQ ID NO:19:

CCTGGGTATG G 11

(2) INFORMATION FOR SEQ ID NO:20:

(i) SEQUENCE CHARACTERISTICS:

(A) LENGTH: 11 base pairs
(B) TYPE: nucleic acid
(C) STRANDEDNESS: single
(D) TOPOLOGY: linear

(ii) MOLECULE TYPE: DNA (genomic)
(iii) HYPOTHETICAL: NO
(vi) ORIGINAL SOURCE:

(A) ORGANISM: Homo sapiens

(xi) SEQUENCE DESCRIPTION: SEQ ID NO:20:

CTCCAGGGAA G . 11

(2) INFORMATION FOR SEQ ID NO:21:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(xi) SEQUENCE DESCRIPTION: SEQ ID NO:21:

GACGTGCTGC CTGAGTACAA 20

(2) INFORMATION FOR SEQ ID NO:22:

(i) SEQUENCE CHARACTERISTICS:

(A) LENGTH: 22 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(xi) SEQUENCE DESCRIPTION: SEQ ID NO:22:

TTCCTGCTGA AGGAGACGGA AG 22

(2) INFORMATION FOR SEQ ID NG:23:

(i) SEQUENCE CHARACTERISTICS:

(A) LENGTH: 21 base pairs
(B) TYPE: nucleic acid
(C) STRANDEDNESS: single
(O) TOPOLOGY: linear

(ii) MOLECULE TYPE: DNA (genomic)
(iii) HYPOTHETICAL: NO
(iv) ANTI-SENSE: NO
(vi) ORIGINAL SOURCE:

(A) ORGANISM: Homo sapiens

(xi) SEQUENCE DESCRIPTION: SEQ ID NO:23:

ACCACCTACG TCAATGCCAA C 21

(2) INFORMATION FOR SEQ ID NO:24:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(xi) SEQUENCE DESCRIPTION: SEQ ID NO:24:

TGCCCCATCA ACGGAATGTG C 21

(2) INFORMATION FOR SEQ ID NO:25:

(i) SEQUENCE CHARACTERISTICS:

(A) LENGTH: 19 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(xi) SEQUENCE DESCRIPTION: SEQ ID NO:25:

GATCGCTACT CAGAGTACG 19

(2) INFORMATION FOR SEQ ID NO:26:

(i) SEQUENCE CHARACTERISTICS:

(A) LENGTH: 22 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(xi) SEQUENCE DESCRIPTION: SEQ ID NO:26:

GCCTGGGCGG CCCCTCCATC AA 22

(2) INFORMATION FOR SEQ ID NO:27:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: YES

(vi) ORIGINAL SOURCE:

(A) ORGANISM: Homo sapiens

(xi) SEQUENCE DESCRIPTION: SEQ ID NO:27:

CACCTCCTCG TTGGCATTGA C 21

(2) INFORMATION FOR SEQ ID NO:28:

(i) SEQUENCE CHARACTERISTICS:

(A) LENGTH: 25 base pairs
(B) TYPE: nucleic acid
(C) STRANDEDNESS: single
(D) TOPOLOGY: linear

(ii) MOLECULE TYPE: DNA (genomic)
(iii) HYPOTHETICAL: NO
(iv) ANTI-SENSE: YES
(vi) ORIGINAL SOURCE:

(A) ORGANISM: Homo sapiens

(xi) SEQUENCE DESCRIPTION: SEQ ID NO:28:

GTCGAAGGGG ATGGCGGCCA CCATG 25

(2) INFORMATION FOR SEQ ID NO:29:

(i) SEQUENCE CHARACTERISTICS:

(A) LENGTH: 23 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: YES

(vi) ORIGINAL SOURCE:

(A) ORGANISM: Homo sapiens

(xi) SEQUENCE DESCRIPTION: SEQ ID NO:29:

TACACCACCT GCCTCCTTGC TGA 23

(2) INFORMATION FOR SEQ ID NO:30:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: YES

(vi) ORIGINAL SOURCE:

(A) ORGANISM: Homo sapiens

(xi) SEQUENCE DESCRIPTION: SEQ ID NO:30:

GCCGCGCCGT ACTCTGAGTA G 21

(2) INFORMATION FOR SEQ ID NO:31:

(i) SEQUENCE CHARACTERISTICS:

(A) LENGTH: 23 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: YES

(vi) ORIGINAL SOURCE:

(A) ORGANISM: Homo sapiens

(xi) SEQUENCE DESCRIPTION: SEQ ID NO:31:

CAGCCAGCCG ATGCGTGAGT CCA 23

(2) INFORMATION FOR SEQ ID NO:32:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: YES

(vi) ORIGINAL SOURCE:

(A) ORGANISM: Homo sapiens

(xi) SEQUENCE DESCRIPTION: SEQ ID NO:32:

GCCCGCCCCT GGGCACACTC A 21

(2) INFORMATION FOR SEQ ID NO:33:

(i) SEQUENCE CHARACTERISTICS:

(A) LENGTH: 19 base pairs
(B) TYPE: nucleic acid
(C) STRANDEDNESS: single
(D) TOPOLOGY: linear

(ii) MOLECULE TYPE: DNA (genomic)
(iii) HYPOTHETICAL: NO
(iv) ANTI-SENSE: YES
(vi) ORIGINAL SOURCE:

(A) ORGANISM: Homo sapiens

(xi) SEQUENCE DESCRIPTION: SEQ ID NO:33:

CAGCATCTGT GTGTGGTAG 19

(2) INFORMATION FOR SEQ ID NO:34:

(i) SEQUENCE CHARACTERISTICS:

(A) LENGTH: 19 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: YES

(vi) ORIGINAL SOURCE:

(A) ORGANISM: Homo sapiens

(xi) SEQUENCE DESCRIPTION: SEQ ID NO:34:

GGCATTTCCA GTCCAGTGC 19

(2) INFORMATION FOR SEQ ID NO:35:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(xi) SEQUENCE DESCRIPTION: SEQ ID NO:35:

CCTGGCCATG AAGTTCAAGA 20

(2) INFORMATION FOR SEQ ID NO:36:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(xi) SEQUENCE DESCRIPTION: SEQ ID NO:36:

GCACTGCAAA CCCTTCCGAG 20

(2) INFORMATION FOR SEQ ID NO:37:

(i) SEQUENCE CHARACTERISTICS:

(A) LENGTH: 22 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: YES

(vi) ORIGINAL SOURCE:

(A) ORGANISM: Homo sapiens

(xi) SEQUENCE DESCRIPTION: SEQ ID NO:37:

GTCGGAGAAC TCAGGGTACA TG 22

(2) INFORMATION FOR SEQ ID NO:38:

(i) SEQUENCE CHARACTERISTICS:

(A) LENGTH: 23 base pairs
(B) TYPE: nucleic acid
(C) STRANDEDNESS: single
(D) TOPOLOGY: linear

(ii) MOLECULE TYPE: DNA (genomic)
(iii) HYPOTHETICAL: NO
(iv) ANTI-SENSE: NO
(vi) ORIGINAL SOURCE:

(A) ORGANISM: Homo sapiens

(xi) SEQUENCE DESCRIPTION: SEQ ID NO:38:

GCCTGTCTGA TCTCCCTGTG TGA 23

(2) INFORMATION FOR SEQ ID NO:39:

(i) SEQUENCE CHARACTERISTICS:

(A) LENGTH: 23 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: YES

(vi) ORIGINAL SOURCE:

(A) ORGANISM: Homo sapiens

(xi) SEQUENCE DESCRIPTION: SEQ ID NO:39:

ACCCAGCCCA GTGGGGAGCT GGT 23

(2) INFORMATION FOR SEQ ID NO:40:

(i) SEQUENCE CHARACTERISTICS:

(A) LENGTH: 22 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(xi) SEQUENCE DESCRIPTION: SEQ ID NO:40:

CCATGCTGGG GCCTCTAAAA CC 22

(2) INFORMATION FOR SEQ ID NO:41:

(i) SEQUENCE CHARACTERISTICS:

(A) LENGTH: 22 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: YES

(vi) ORIGINAL SOURCE:

(A) ORGANISM: Homo sapiens

(xi) SEQUENCE DESCRIPTION: SEQ ID NO:41:

GGCTCTGATG GCTGGCCATG TG 22

(2) INFORMATION FOR SEQ ID NO:42:

(i) SEQUENCE CHARACTERISTICS:

(A) LENGTH: 22 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(xi) SEQUENCE DESCRIPTION: SEQ ID NO:42:

CCCAGCGAGC ACTTTCCATT TG 22

(2) INFORMATION FOR SEQ ID NO:43:

(i) SEQUENCE CHARACTERISTICS:

(A) LENGTH: 24 base pairs
(B) TYPE: nucleic acid
(C) STRANDEDNESS: single
(D) TOPOLOGY: linear

(ii) MOLECULE TYPE: DNA (genomic)
(iii) HYPOTHETICAL: NO
(iv) ANTI-SENSE: YES
(vi) ORIGINAL SOURCE:

(A) ORGANISM: Homo sapiens

(xi) SEQUENCE DESCRIPTION: SEQ ID NO:43:

GCTTCTCCGT CCAGCTGACT TGTA 24

(2) INFORMATION FOR SEQ ID NO:44:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(xi) SEQUENCE DESCRIPTION: SEQ ID NO:44:

GAGCCCAGCC GTGGGCATCC T 21

(2) INFORMATION FOR SEQ ID NO:45:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: YES

(vi) ORIGINAL SOURCE:

(A) ORGANISM: Homo sapiens

(xi) SEQUENCE DESCRIPTION: SEQ ID NO:45:

GTCCCCACTC ACCATGGGCA G 21

(2) INFORMATION FOR SEQ ID NO:46:

(i) SEQUENCE CHARACTERISTICS:

(A) LENGTH: 53 amino acids
(B) TYPE: amino acid
(D) TOPOLOGY: linear

(ii) MOLECULE TYPE: peptide

(iii) HYPOTHETICAL: NO

(vi) ORIGINAL SOURCE:

(A) ORGANISM: Homo sapiens

(xi) SEQUENCE DESCRIPTION: SEQ ID NO:46:

Asp Asn Phe Asn Gln Gln Lys Lys Lys Leu Gly Gly Gln Asp Ile Phe
1 5 10 15

Met Thr Glu Glu Gln Lys Lys Tyr Tyr Asn Ala Met Lys Lys Leu Gly
20 25 30

Ser Lys Lys Pro Gln Lys Pro Ile Pro Arg Pro Leu Asn Lys Tyr Gln
35 40 45

Gly Phe Ile Phe Asp
50

(2) INFORMATION FOR SEQ ID NO:47:

(i) SEQUENCE CHARACTERISTICS:

(A) LENGTH: 53 amino acids
(B) TYPE: amino acid
(D) TOPOLOGY: linear

(ii) MOLECULE TYPE: peptide

(iii) HYPOTHETICAL: NO

(vi) ORIGINAL SOURCE:

(A) ORGANISM: Homo sapiens

(xi) SEQUENCE DESCRIPTION: SEQ ID NO:47:

Asp Asn Phe Asn Gln Gln Lys Lys Lys Leu Gly Gly Lys Asp Ile Phe
1 5 10 15

Met Thr Glu Glu Gln Lys Lys Tyr Tyr Asn Ala Met Lys Lys Leu Gly
20 25 30

Ser Lys Lys Pro Gln Lys Pro Ile Pro Arg Pro Gln Asn Lys Ile Gln
35 40 45

Gly Met Val Tyr Asp
50

(2) INFORMATION FOR SEQ ID NO:48:

(i) SEQUENCE CHARACTERISTICS:

(A) LENGTH: 53 amino acids

(B) TYPE: amino acid
(D) TOPOLOGY: linear

(ii) MOLECULE TYPE: peptide

(iii) HYPOTHETICAL: NO

(vi) ORIGINAL SOURCE:

(A) ORGANISM: Homo sapiens

(xi) SEQUENCE DESCRIPTION: SEQ ID NO:48:

```
Asp Asn Phe Asn Gln Gln Lys Lys Lys Phe Gly Gly Gln Asp Ile Phe
1               5                   10                  15

Met Thr Glu Glu Gln Lys Lys Tyr Tyr Asn Ala Met Lys Lys Leu Gly
            20                  25                  30

Ser Lys Lys Pro Gln Lys Pro Ile Pro Arg Pro Ala Asn Lys Phe Gln
        35                  40                  45

Gly Met Val Phe Asp
    50
```

(2) INFORMATION FOR SEQ ID NO:49:

(i) SEQUENCE CHARACTERISTICS:

(A) LENGTH: 53 amino acids
(B) TYPE: amino acid
(D) TOPOLOGY: linear

(ii) MOLECULE TYPE: peptide

(iii) HYPOTHETICAL: NO

(xi) SEQUENCE DESCRIPTION: SEQ ID NO:49:

Asp Asn Phe Asn Gln Gln Lys Lys Lys Phe Gly Gly Lys Asp Ile Phe
1 5 10 15

Met Thr Glu Glu Gln Lys Lys Tyr Tyr Asn Ala Met Lys Lys Leu Gly
20 25 30

Ser Lys Lys Pro Gln Lys Pro Ile Pro Arg Pro Gln Asn Lys Ile Gln
35 40 45

Gly Met Val Tyr Asp
50

(2) INFORMATION FOR SEQ ID NO:50:

(i) SEQUENCE CHARACTERISTICS:

(A) LENGTH: 53 amino acids
(B) TYPE: amino acid
(D) TOPOLOGY: linear

(ii) MOLECULE TYPE: peptide

(iii) HYPOTHETICAL: NO

(xi) SEQUENCE DESCRIPTION: SEQ ID NO:50:

Asp Asn Phe Asn Gln Gln Lys Lys Lys Phe Gly Gly Gln Asp Ile Phe
1 5 10 15

Met Thr Glu Glu Gln Lys Lys Tyr Tyr Asn Ala Met Lys Lys Leu Gly
20 25 30

Ser Lys Lys Pro Gln Lys Pro Ile Pro Arg Pro Ala Asn Lys Phe Gln
35 40 45

Gly Met Val Phe Asp
50

(2) INFORMATION FOR SEQ ID NO:51:

(i) SEQUENCE CHARACTERISTICS:

(A) LENGTH: 53 amino acids
(B) TYPE: amino acid
(D) TOPOLOGY: linear

(ii) MOLECULE TYPE: peptide

(iii) HYPOTHETICAL: NO

(xi) SEQUENCE DESCRIPTION: SEQ ID NO:51:

```
Asp Asn Phe Asn Gln Gln Lys Lys Lys Phe Gly Gly Gln Asp Ile Phe
1               5                   10                  15

Met Thr Glu Glu Gln Lys Lys Tyr Tyr Asn Ala Met Lys Lys Leu Gly
            20                  25                  30

Ser Lys Lys Pro Gln Lys Pro Ile Pro Arg Pro Gly Asn Lys Phe Gln
        35                  40                  45

Gly Met Val Phe Asp
    50
```

(2) INFORMATION FOR SEQ ID NO:52:

(i) SEQUENCE CHARACTERISTICS:

(A) LENGTH: 53 amino acids
(B) TYPE: amino acid
(D) TOPOLOGY: linear

(ii) MOLECULE TYPE: peptide

(iii) HYPOTHETICAL: NO

(vi) ORIGINAL SOURCE:

(A) ORGANISM: Fugu rubripes

(xi) SEQUENCE DESCRIPTION: SEQ ID NO:52:

```
Asp Asn Phe Asn Gln Gln Lys Lys Lys Phe Gly Gly Gln Asp Ile Phe
1               5                   10                  15

Met Thr Glu Glu Gln Lys Lys Tyr Tyr Asn Ala Met Lys Lys Leu Gly
            20                  25                  30

Ser Lys Lys Pro Gln Lys Pro Ile Pro Arg Pro Gln Asn Lys Ile Gln
        35                  40                  45

Gly Met Val Phe Asp
    50
```

(2) INFORMATION FOR SEQ ID NO:53:

(i) SEQUENCE CHARACTERISTICS:

(A) LENGTH: 54 amino acids
(B) TYPE: amino acid
(D) TOPOLOGY: linear

(ii) MOLECULE TYPE: peptide

(iii) HYPOTHETICAL: NO

(vi) ORIGINAL SOURCE:

(A) ORGANISM: Loligo opalescens

(xi) SEQUENCE DESCRIPTION: SEQ ID NO:53:

```
Asp Asn Phe Asn Gln Gln Lys Lys Gly Ala Gly Gly Ser Leu Glu Val
1               5                   10                  15

Phe Met Thr Asp Asp Gln Lys Lys Tyr Tyr Lys Ala Met Lys Asn Leu
            20                  25                  30

Gln Ser Lys Lys Pro Thr Lys Gly Ile Pro Met Pro Gly Phe Lys Ile
        35                  40                  45

Ala Glu Trp Met Phe His
    50
```

(2) INFORMATION FOR SEQ ID NO:54:

(i) SEQUENCE CHARACTERISTICS:

(A) LENGTH: 55 amino acids
(B) TYPE: amino acid
(D) TOPOLOGY: linear

(ii) MOLECULE TYPE: peptide

(iii) HYPOTHETICAL: NO

(vi) ORIGINAL SOURCE:

(A) ORGANISM: Loligo bleekiri

(xi) SEQUENCE DESCRIPTION: SEQ ID NO:54:

Asp Lys Phe Ser Phe Leu Lys Lys Lys Tyr Asp Gly Thr Tyr Leu Asp
1 5 10 15

Met Phe Leu Thr Pro Thr Gln Gln Asn Tyr Tyr Asn Thr Leu Lys Lys
20 25 30

Leu Gly Thr Lys Lys Pro Gln Lys Thr Val Lys Arg Pro Lys Asn Lys
35 40 45

Cys Gln Ala Val Val Tyr Asp
50 55

(2) INFORMATION FOR SEQ ID NO:55:

(i) SEQUENCE CHARACTERISTICS:

(A) LENGTH: 54 amino acids
(B) TYPE: amino acid
(D) TOPOLOGY: linear

(ii) MOLECULE TYPE: peptide

(iii) HYPOTHETICAL: NO

(vi) ORIGINAL SOURCE:

(A) ORGANISM: Drosophila melanogaster

(xi) SEQUENCE DESCRIPTION: SEQ ID NO:55:

Asp Asn Phe Asn Met Leu Arg Arg Ser Ile Glu Gly Val Leu Glu Met
1 5 10 15

Phe Leu Thr Glu Ser Gln Lys His Tyr Tyr Thr Ala Met Lys Lys Leu
20 25 30

Gly Arg Lys Lys Pro Gln Lys Val Ile Lys Arg Pro Ile Asn His Phe
35 40 45

Leu Ala Met Phe Tyr Asp
50

(2) INFORMATION FOR SEQ ID NO:56:

(i) SEQUENCE CHARACTERISTICS:

(A) LENGTH: 54 amino acids

(B) TYPE: amino acid
(D) TOPOLOGY: linear

(ii) MOLECULE TYPE: peptide

(iii) HYPOTHETICAL: NO

(vi) ORIGINAL SOURCE:

(A) ORGANISM: Heliothis virescens

(xi) SEQUENCE DESCRIPTION: SEQ ID NO:56:

```
Asp Asn Phe Asn Glu Gln Lys Lys Lys Ala Ala Gly Ser Leu Glu Met
1               5                   10                  15

Phe Met Thr Glu Asp Gln Lys Lys Tyr Tyr Asn Ala Met Lys Lys Met
            20                  25                  30

Gly Ser Lys Lys Pro Leu Lys Ala Ile Pro Arg Pro Lys Trp Arg Pro
        35                  40                  45

Gln Ala Ile Val Phe Glu
    50
```

(2) INFORMATION FOR SEQ ID NO:57:

(i) SEQUENCE CHARACTERISTICS:

(A) LENGTH: 10 amino acids
(B) TYPE: amino acid
(D) TOPOLOGY: linear

(ii) MOLECULE TYPE: peptide

(iii) HYPOTHETICAL: NO

(xi) SEQUENCE DESCRIPTION: SEQ ID NO:57:

```
Gly Ser Lys Lys Pro Gln Lys Pro Ile Pro
1               5                   10
```

(2) INFORMATION FOR SEQ ID NO:58:

(i) SEQUENCE CHARACTERISTICS:

(A) LENGTH: 29 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(vi) ORIGINAL SOURCE:

(A) ORGANISM: Homo sapiens

(xi) SEQUENCE DESCRIPTION: SEQ ID NO:58:

GGCTCCAAGA AGCCCCAGAA GCCCATCCC 29

(2) INFORMATION FOR SEQ ID NO:59:

(i) SEQUENCE CHARACTERISTICS:

(A) LENGTH: 7 amino acids
(B) TYPE: amino acid
(D) TOPOLOGY: linear

(ii) MOLECULE TYPE: peptide

(iii) HYPOTHETICAL: NO

(vi) ORIGINAL SOURCE:

(A) ORGANISM: Homo sapiens

(xi) SEQUENCE DESCRIPTION: SEQ ID NO:59:

Gly Ser Lys Lys Pro Ile Pro
1 5

(2) INFORMATION FOR SEQ ID NO:60:

(i) SEQUENCE CHARACTERISTICS:

(A) LENGTH: 20 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(vi) ORIGINAL SOURCE:

(A) ORGANISM: Homo sapiens

(xi) SEQUENCE DESCRIPTION: SEQ ID NO:60:

GGCTCCAAGA AGCCCATCCC 20

(2) INFORMATION FOR SEQ ID NO:61:

(i) SEQUENCE CHARACTERISTICS:

(A) LENGTH: 45 base pairs
(B) TYPE: nucleic acid
(C) STRANDEDNESS: double
(D) TOPOLOGY: linear

(ii) MOLECULE TYPE: DNA (genomic)
(iii) HYPOTHETICAL: NO
(vi) ORIGINAL SOURCE:

(A) ORGANISM: Homo sapiens

(xi) SEQUENCE DESCRIPTION: SEQ ID NO:61:

TGGTTCCTCA TCGACATGGT GGCCGCCATC CCCTTCGACC TGCTC 45

(2) INFORMATION FOR SEQ ID NO:62:

(i) SEQUENCE CHARACTERISTICS:

(A) LENGTH: 15 amino acids
(B) TYPE: amino acid
(D) TOPOLOGY: linear

(ii) MOLECULE TYPE: peptide

(iii) HYPOTHETICAL: NO

(vi) ORIGINAL SOURCE:

(A) ORGANISM: Homo sapiens

(xi) SEQUENCE DESCRIPTION: SEQ ID NO:62:

Trp Phe Leu Ile Asp Met Val Ala Ala Ile Pro Phe Asp Leu Leu
1 5 10 15

(2) INFORMATION FOR SEQ ID NO:63:

(i) SEQUENCE CHARACTERISTICS:

(A) LENGTH: 54 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(vi) ORIGINAL SOURCE:

(A) ORGANISM: Homo sapiens

(xi) SEQUENCE DESCRIPTION: SEQ ID NO:63:

GTCATCTACA CGGCTGTCTT CACACCCTAC TCGGCTGCCT TCCTGCTGAA GGAG 54

(2) INFORMATION FOR SEQ ID NO:64:

(i) SEQUENCE CHARACTERISTICS:

(A) LENGTH: 18 amino acids
(B) TYPE: amino acid
(D) TOPOLOGY: linear

(ii) MOLECULE TYPE: peptide
(iii) HYPOTHETICAL: NO
(vi) ORIGINAL SOURCE:

(A) ORGANISM: Homo sapiens

(xi) SEQUENCE DESCRIPTION: SEQ ID NO:64:

Val Ile Tyr .Thr Ala Val Phe Thr Pro Tyr Ser Ala Ala Phe Leu Leu
1 5 10 15

Lys Glu

(2) INFORMATION FOR SEQ ID NO:65:

(i) SEQUENCE CHARACTERISTICS:

(A) LENGTH: 48 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(vi) ORIGINAL SOURCE:

(A) ORGANISM: Homo sapiens

(xi) SEQUENCE DESCRIPTION: SEQ ID NO:65:

GTCATCTACC GGCTGTCTTC ACACCCTACT CGGCTGCCTT CCTGCTGA 48

(2) INFORMATION FOR SEQ ID NO:66:

(i) SEQUENCE CHARACTERISTICS:

(A) LENGTH: 15 amino acids
(B) TYPE: amino acid
(D) TOPOLOGY: linear

(ii) MOLECULE TYPE: peptide

(iii) HYPOTHETICAL: NO

(vi) ORIGINAL SOURCE:

(A) ORGANISM: Homo sapiens

(xi) SEQUENCE DESCRIPTION: SEQ ID NO:66:

```
Val Ile Tyr Arg Leu Ser Ser His Pro Thr Arg Leu Pro Ser Cys
1               5                   10                  15
```

(2) INFORMATION FOR SEQ ID NO:67:

(i) SEQUENCE CHARACTERISTICS:

(A) LENGTH: 6 amino acids
(B) TYPE: amino acid
(D) TOPOLOGY: linear

(ii) MOLECULE TYPE: peptide

(iii) HYPOTHETICAL: NO

(vi) ORIGINAL SOURCE:

(A) ORGANISM: Homo sapiens

(xi) SEQUENCE DESCRIPTION: SEQ ID NO:67:

```
Leu Ile Ala His Trp Leu
1               5
```

(2) INFORMATION FOR SEQ ID NO:68:

(i) SEQUENCE CHARACTERISTICS:

(A) LENGTH: 6 amino acids
(B) TYPE: amino acid
(D) TOPOLOGY: linear

(ii) MOLECULE TYPE: peptide

(iii) HYPOTHETICAL: NO

(xi) SEQUENCE DESCRIPTION: SEQ ID NO:6B:

Leu Ile Val His Trp Leu
1 5

(2) INFORMATION FOR SEQ ID NO:69:

(i) SEQUENCE CHARACTERISTICS:

(A) LENGTH: 6 amino acids
(B) TYPE: amino acid
(D) TOPOLOGY: linear

(ii) MOLECULE TYPE: peptide

(iii) HYPOTHETICAL: NO

(xi) SEQUENCE DESCRIPTION: SEQ ID NO:69:

Leu Ala Ala His Trp Lys
1 5

(2) INFORMATION FOR SEQ ID NO:70:

(i) SEQUENCE CHARACTERISTICS:

(A) LENGTH: 6 amino acids
(B) TYPE: amino acid
(D) TOPOLOGY: linear

(ii) MOLECULE TYPE: peptide

(iii) HYPOTHETICAL: NO

(xi) SEQUENCE DESCRIPTION: SEQ ID NO:70:

Leu Ala Ala His Trp Met
1 5

(2) INFORMATION FOR SEQ ID NO:71:

(i) SEQUENCE CHARACTERISTICS:

(A) LENGTH: 6 amino acids
(B) TYPE: amino acid
(D) TOPOLOGY: linear

(ii) MOLECULE TYPE: peptide

(iii) HYPOTHETICAL: NO

(xi) SEQUENCE DESCRIPTION: SEQ ID NO:71:

Leu Val Ala His Trp Leu
1 5

(2) INFORMATION FOR SEQ ID NO:72:

(i) SEQUENCE CHARACTERISTICS:

(A) LENGTH: 6 amino acids
(B) TYPE: amino acid
(D) TOPOLOGY: linear

(ii) MOLECULE TYPE: peptide

(iii) HYPOTHETICAL: NO

(xi) SEQUENCE DESCRIPTION: SEQ ID NO:72:

Leu Ala Ala His Trp Leu
1 5

(2) INFORMATION FOR SEQ ID NO:73:

(i) SEQUENCE CHARACTERISTICS:

(A) LENGTH: 7 amino acids
(B) TYPE: amino acid
(D) TOPOLOGY: linear

(ii) MOLECULE TYPE: peptide
(iii) HYPOTHETICAL: NO
(xi) SEQUENCE DESCRIPTION: SEQ ID NO:73:

Asp Ile Leu Ile Asn Phe Arg
1 5

(2) INFORMATION FOR SEQ ID NO:74:

(i) SEQUENCE CHARACTERISTICS:

(A) LENGTH: 7 amino acids
(B) TYPE: amino acid
(D) TOPOLOGY: linear

(ii) MOLECULE TYPE: peptide

(iii) HYPOTHETICAL: NO

(xi) SEQUENCE DESCRIPTION: SEQ ID NO:74:

Asp Ile Leu Ile Asp Phe Arg
1 5

(2) INFORMATION FOR SEQ ID NO:75:

(i) SEQUENCE CHARACTERISTICS:

(A) LENGTH: 7 amino acids
(B) TYPE: amino acid
(D) TOPOLOGY: linear

(ii) MOLECULE TYPE: peptide

(iii) HYPOTHETICAL: NO

(xi) SEQUENCE DESCRIPTION: SEQ ID NO:75:

Asp Ile Val Leu Asn Phe His
1 5

(2) INFORMATION FOR SEQ ID NO:76:

(i) SEQUENCE CHARACTERISTICS:

(A) LENGTH: 7 amino acids
(B) TYPE: amino acid
(D) TOPOLOGY: linear

(ii) MOLECULE TYPE: peptide
(iii) HYPOTHETICAL: NO
(xi) SEQUENCE DESCRIPTION: SEQ ID NO:76:

Asp Ile Leu Leu Asn Phe Arg
1 5

(2) INFORMATION FOR SEQ ID NO:77:

(i) SEQUENCE CHARACTERISTICS:

(A) LENGTH: 8 amino acids
(B) TYPE: amino acid
(D) TOPOLOGY: linear

(ii) MOLECULE TYPE: peptide

(iii) HYPOTHETICAL: NO

(vi) ORIGINAL SOURCE:

(A) ORGANISM: Homo sapiens

(xi) SEQUENCE DESCRIPTION: SEQ ID NO:77:

Ser Val Gly Phe Gly Asn Val Ser
1 5

(2) INFORMATION FOR SEQ ID NO:78:

(i) SEQUENCE CHARACTERISTICS:

(A) LENGTH: 8 amino acids
(B) TYPE: amino acid
(D) TOPOLOGY: linear

(ii) MOLECULE TYPE: peptide

(iii) HYPOTHETICAL: NO

(xi) SEQUENCE DESCRIPTION: SEQ ID NO:78:

```
Ser Val Gly Phe Ser Asn Val Ser
1               5
```

(2) INFORMATION FOR SEQ ID NO:79:

(i) SEQUENCE CHARACTERISTICS:

(A) LENGTH: 8 amino acids
(B) TYPE: amino acid
(D) TOPOLOGY: linear

(ii) MOLECULE TYPE: peptide
(iii) HYPOTHETICAL: NO
(xi) SEQUENCE DESCRIPTION: SEQ ID NO:79:

```
Ser Val Gly Phe Gly Asn Ile Ala
1               5
```

(2) INFORMATION FOR SEQ ID NO:80:

(i) SEQUENCE CHARACTERISTICS:

(A) LENGTH: 8 amino acids
(B) TYPE: amino acid
(D) TOPOLOGY: linear

(ii) MOLECULE TYPE: peptide

(iii) HYPOTHETICAL: NO

(xi) SEQUENCE DESCRIPTION: SEQ ID NO:80:

```
Ser Val Gly Phe Gly Asn Val Ala
1               5
```

(2) INFORMATION FOR SEQ ID NO:81:

(i) SEQUENCE CHARACTERISTICS:

(A) LENGTH: 8 amino acids
(B) TYPE: amino acid
(D) TOPOLOGY: linear

(ii) MOLECULE TYPE: peptide

(iii) HYPOTHETICAL: NO

(xi) SEQUENCE DESCRIPTION: SEQ ID NO:81:

```
Thr Val Gly Tyr Gly Asp Met Thr
1               5
```